# EUROPEAN PATENT SPECIFICATION

(11) **EP 2 909 183 B1**
(45) Date of publication and mention of the grant of the patent: **24.08.2016**
(21) Application number: 13777039.2
(22) Date of filing: 15.10.2013
(51) Int. Cl.: C07D 239/42, A61K 31/505, A61P 35/00

(54) **4-(ORTHO)-FLUOROPHENYL-5-FLUOROPYRIMIDIN-2-YL AMINES CONTAINING A SULFONE GROUP**
4-(ORTHO)-FLUORPHENYL-5-FLUORPYRIMIDIN-2-YL-AMINE MIT EINER SULFONGRUPPE
4-(ORTHO)-FLUOROPHÉNYL-5-FLUOROPYRIMIDIN-2-YL AMINES CONTENANT UN GROUPE SULFONE

(30) Priority: 18.10.2012 EP 12189137
(43) Date of publication of application: 26.08.2015
(73) Proprietor: Bayer Pharma Aktiengesellschaft, 13353 Berlin (DE)
(72) Inventor: LÜCKING, Ulrich, 10317 Berlin (DE); BOHLMANN, Rolf, 14055 Berlin (DE); KOSEMUND, Dirk, 12587 Berlin (DE); SCHOLZ, Arne, 10435 Berlin (DE); LIENAU, Philip, 10967 Berlin (DE); SIEMEISTER, Gerhard, 13503 Berlin (DE); BÖMER, Ulf, 16548 Glienicke (DE)
(74) Representative: BIP Patents
(86) International application number: PCT/EP2013/071461
(87) International publication number: WO 2014/060376

(56) References cited:
- WO-A1-2008/079918
- WO-A1-2008/129070
- WO-A1-2013/037896

## Description

The present invention relates to 4-(*ortho*)-fluorophenyl-5-fluoropyrimidin-2-yl amine derivatives of general formula (I) as described and defined herein, and methods for their preparation, and compounds of formula (I) for use in the treatment and/or prophylaxis of disorders, in particular of hyper-proliferative disorders and/or virally induced infectious diseases and/or of cardiovascular diseases. The invention further relates to intermediate compounds useful in the preparation of said compounds of general formula (I).

The family of cyclin-dependent kinase (CDK) proteins consists of members that are key regulators of the cell division cycle (cell cycle CDK's), that are involved in regulation of gene transcription (transcriptional CDK's), and of members with other functions. CDKs require for activation the association with a regulatory cyclin subunit. The cell cycle CDKs CDK1/cyclin B, CDK2/cyclin A, CDK2/cyclinE, CDK4/cyclinD, and CDK6/cyclinD get activated in a sequential order to drive a cell into and through the cell division cycle. The transcriptional CDKs CDK9/cyclin T and CDK7/cyclin H regulate the activity of RNApolymerase II via phosphorylation of the carboxy-terminal domain (CTD). Positive transcription factor b (P-TEFb) is a heterodimer of CDK9 and one of four cyclin partners, cyclin T1, cyclin K, cyclin T2a or T2b.

Whereas CDK9 (NCBI GenBank Gene ID 1025) is exclusively involved in transcriptional regulation, CDK7 in addition participates in cell cycle regulation as CDK-activating kinase (CAK).

Transcription of genes by RNA polymerase II is initiated by assembly of the pre-initiation complex at the promoter region and phosphorylation of Ser 5 and Ser 7 of the CTD by CDK7/cyclin H. For a major fraction of genes RNA polymerase II stops mRNA transcription after it moved 20-40 nucleotides along the DNA template. This promoter-proximal pausing of RNA polymerase II is mediated by negative elongation factors and is recognized as a major control mechanism to regulate expression of rapidly induced genes in response to a variety of stimuli (Cho et al., Cell Cycle 9, 1697, 2010). P-TEFb is crucially involved in overcoming promoter-proximal pausing of RNA polymerase II and transition into a productive elongation state by phosphorylation of Ser 2 of the CTD as well as by phosphorylation and inactivation of negative elongation factors.

Activity of P-TEFb itself is regulated by several mechanisms. About half of cellular P-TEFb exists in an inactive complex with 7SK small nuclear RNA (7SK snRNA), La-related protein 7 (LARP7/PIP7S) and hexamethylene bis-acetamide inducible proteins 1/2 (HEXIM1/2, He et al., Mol Cell 29, 588, 2008). The remaining half of P-TEFb exists in an active complex containing the bromodomain protein Brd4 (Yang et al., Mol Cell 19, 535, 2005). Brd4 recruits P-TEFb through interaction with acetylated histones to chromatin areas primed for gene transcription. Through alternately interacting with its positive and negative regulators, P-TEFb is maintained in a functional equilibrium: P-TEFb bound to the 7SK snRNA complex represents a reservoir from which active P-TEFb can be released on demand of cellular transcription and cell proliferation (Zhou & Yik, Microbiol Mol Biol Rev 70, 646, 2006). Furthermore, the activity of P-TEFb is regulated by posttranslational modifications including phosphorylation/dephosphorylation, ubiquitination, and acteylation (reviewed in Cho et al., Cell Cycle 9, 1697, 2010).

Deregulated activity of CDK9 kinase activity of the P-TEFb heterodimer is associated with a variety of human pathological settings such as hyper-proliferative diseases (e.g. cancer), virally induced infectious diseases or cardiovascular diseases:
Cancer is regarded as a hyper-proliferative disorder mediated by a disbalance of proliferation and cell death (apoptosis). High levels of anti-apoptotic Bcl-2-family proteins are found in various human tumors and account for prolonged survival of tumor cells and therapy resistance. Inhibition of P-TEFb kinase activity was shown to reduce transcriptional activity of RNA polymerase II leading to a decline of short-lived anti-apoptotic proteins, especially Mcl-1 and XIAP, reinstalling the ability of tumor cells to undergo apoptosis. A number of other proteins associated with the transformed tumor phenotype (such as Myc, NF-kB responsive gene transcripts, mitotic kinases) are either short-lived proteins or are encoded by short-lived transcripts which are sensitive to reduced RNA polymerase II activity mediated by P-TEFb inhibition (reviewed in Wang & Fischer, Trends Pharmacol Sci 29, 302, 2008).

Many viruses rely on the transcriptional machinery of the host cell for the transcription of their own genome. In case of HIV-1, RNA polymerase II gets recruited to the promoter region within the viral LTR's. The viral transcription activator (Tat) protein binds to nascent viral transcripts and overcomes promoter-proximal RNA polymerase II pausing by recruitment of P-TEFb which in turn promotes transcriptional elongation. Furthermore, the Tat protein increases the fraction of active P-TEFb by replacement of the P-TEFb inhibitory proteins HEXIM1/2 within the 7SK snRNA complex. Recent data have shown that inhibition of the kinase activity of P-TEFb is sufficient to block HIV-1 repliction at kinase inhibitor concentrations that are not cytotoxic to the host cells (reviewed in Wang & Fischer, Trends Pharmacol Sci 29, 302, 2008). Similarly, recruitment of P-TEFb by viral proteins has been reported for other viruses such as B-cell cancer-associated Epstein-Barr virus, where the nuclear antigen EBNA2 protein interacts with P-TEFb (Bark-Jones et al., Oncogene, 25, 1775, 2006), and the human T-lymphotropic virus type 1 (HTLV-1), where the transcriptional activator Tax recruits P-TEFb (Zhou et al., J Virol. 80, 4781, 2006).

Cardiac hypertrophy, the heart's adaptive response to mechanical overload and pressure (hemodynamic stress e.g. hypertension, myocardial infarction), can lead, on a long term, to heart failure and death. Cardiac hypertrophy was shown to be associated with increased transcriptional activity and RNA polymerase II CTD phosphorylation in cardiac muscle cells. P-TEFb was found to be activated by dissociation from the inactive 7SK snRNA/HEXIM1/2 complex. These findings suggest pharmacological inhibition of P-TEFb kinase activity as a therapeutic approach to treat cardiac hypertrophy (reviewed in Dey et al., Cell Cycle 6, 1856, 2007).

In summary, multiple lines of evidence suggest that selective inhibition of the CDK9 kinase activity of the P-TEFb heterodimer (= CDK9 and one of four cyclin partners, cyclin T1, cyclin K, cyclin T2a or T2b) represents an innovative approach for the treatment of diseases such as cancer, viral diseases, and/or diseases of the heart. CDK9 belongs to a family of at least 13 closely related kinases of which the subgroup of the cell cycle CDK's fulfills multiple roles in regulation of cell proliferation. Thus, co-inhibition of cell cycle CDKs (e.g. CDK1/cyclin B, CDK2/cyclin A, CDK2/cyclinE, CDK4/cyclinD, CDK6/cyclinD) and of CDK9, is expected to impact normal proliferating tissues such as intestinal mucosa, lymphatic and hematopoietic organs, and reproductive organs. To maximize the therapeutic margin of CDK9 kinase inhibitors, molecules with high selectivity towards CDK9 are required.

CDK inhibitors in general as well as CDK9 inhibitors are described in a number of different publications:
WO2008129070 and WO2008129071 both describe 2,4 disubstituted aminopyrimidines as CDK inhibitors in general. It is also asserted that some of these compounds may act as selective CDK9 inhibitors (WO2008129070) and as CDK5 inhibitors (WO2008129071), respectively, but no specific CDK9 IC₅₀ (WO2008129070) or CDK5 IC₅₀ (WO2008129071) data is presented. These compounds do not contain a fluoro atom in 5-position of the pyrimidine core.
WO2008129080 discloses 4,6 disubstituted aminopyrimidines and demonstrates that these compounds show an inhibitory effect on the protein kinase activity of various protein kinases, such as CDK1, CDK2, CDK4, CDK5, CDK6 and CDK9, with a preference for CDK9 inhibition (example 80).
WO2005026129 discloses 4,6 disubstituted aminopyrimidines and demonstrates that these compounds show an inhibitory effect on the protein kinase activity of various protein kinases, in particular CDK2, CDK4, and CDK9.
WO2011116951 discloses substituted triazine derivatives as selective CDK9 inhibitors.
WO2012117048 discloses disubstituted triazine derivatives as selective CDK9 inhibitors.
WO2012117059 discloses disubstituted pyridine derivatives as selective CDK9 inhibitors.
EP1218360 B1, which corresponds to US2004116388A1, US7074789B2 and WO2001025220A1, describes triazine derivatives as kinase inhibitors, but does not disclose potent or selective CDK9 inhibitors.
WO2008079933 discloses aminopyridine and aminopyrimidine derivatives and their use as CDK1, CDK2, CDK3, CDK4, CDK5, CDK6, CDK7, CDK8 or CDK9 inhibitors.
WO2011012661 describes aminopyridine derivatives useful as CDK inhibitors.
WO2011026917 discloses carboxamides derived from substituted 4-phenylpyridine-2-amines as inhibitors of CDK9.
WO2012066065 discloses phenyl-heteroaryl amines as inhibitors of CDK9. A selectivity towards CDK9 over other CDK isoforms is preferred, however disclosure of CDK-inhibition data is confined to CDK 9. No bicyclic ring systems are disclosed attached to the C4 position of the pyrimidine core. Within the group attached to C4 of the pyrimidine core, alkoxy phenyls can be regarded as encompassed, but there is no suggestion for a specific substitution pattern characterised by a fluoro atom attached to C5 of the pyrimidine ring, and an aniline at C2 of the pyrimidine, featuring a substituted sulfonyl-methylene group in meta position. Compounds shown in the examples typically feature a substituted cycloalkyl group as R¹ but no phenyl.
WO2012066070 discloses 3-(aminoaryl)-pyridine compounds as inhibitors of CDK9. The biaryl core mandatorily consists of two heteroaromatic rings.
WO2012101062 discloses substituted bi-heteroaryl compounds featuring a 2-aminopyridine core as inhibitors of CDK9. The biaryl core mandatorily consists of two heteroaromatic rings.
WO2012101063 discloses carboxamides derived from substituted 4-(heteroaryl)-pyridine-2-amines as inhibitors of CDK9.
WO 2012101064 discloses N-acyl pyrimidine biaryl compounds as inhibitors of CDK9.
WO 2012101065 discloses pyrimidine biaryl compounds as inhibitors of CDK9. The biaryl core mandatorily consists of two heteroaromatic rings.
WO 2012101066 discloses pyrimidine biaryl compounds as inhibitors of CDK9. Substitution R1 of the amino group attached to the heteroaromatic core is confined to non-aromatic groups but does not cover substituted phenyls. Furthermore, the biaryl core mandatorily consists of two heteroaromatic rings.
WO 2013037896, published after the priority date of the present application, describes disubstituted 5-fluoropyrimidines as selective inhibitors of CDK9. The document discloses 4-(2,4-Difluorophenyl)-5-fluoro-N-{3-[(methylsulfonyl)methyl]-phenyl}pyrimidin-2-amine as Intermediate compound 16.2 but not as an active compound inhibiting CDK9.
WO 2013037894 discloses disubstituted 5-fluoropyromidine derivatives containing a sulfoximine group as selective inhibitors of CDK9.
Wang et al. (Chemistry & Biology 17, 1111-1121, 2010) describe 2-anilino-4-(thiazol-5-yl)pyrimidine transcriptional CDK inhibitors, which show anticancer activity in animal models.
WO2004009562 discloses substituted triazine kinase inhibitors. For selected compounds CDK1 and CDK4 test data, but no CDK9 data is presented.
WO2004072063 describes heteroaryl (pyrimidine, triazine) substituted pyrroles as inhibitors of protein kinases such as ERK2, GSK3, PKA or CDK2.
WO2010009155 discloses triazine and pyrimidine derivatives as inhibitors of histone deacetylase and/or cyclin dependent kinases (CDKs). For selected compounds CDK2 test data is described.
WO2003037346 (corresponding to US7618968B2, US7291616B2, US2008064700A1, US2003153570A1) relates to aryl triazines and uses thereof, including to inhibit lysophosphatidic acid acyltransferase beta (LPAAT-beta) activity and/or proliferation of cells such as tumor cells.
WO2008025556 describes carbamoyl sulfoximides having a pyrimidine core, which are useful as kinase inhibitors. No CDK9 data is presented. No molecules are exemplified, which possess a fluoropyrimidine core.
WO2002066481 describes pyrimidine derivatives as cyclin dependent kinase inhibitors. CDK9 is not mentioned and no CDK9 data is presented.
WO2008109943 concerns phenyl aminopyri(mi)dine compounds and their use as kinase inhibitors, in particular as JAK2 kinase inhibitors. The specific examples mainly focus on compounds having a pyrimidine core.
WO2009032861 describes substituted pyrimidinyl amines as JNK kinase inhibitors. The specific examples mainly focus on compounds having a pyrimidine core.
WO2011046970 concerns amino-pyrimidine compounds as inhibitors of TBKL and/or IKK epsilon. The specific examples mainly focus on compounds having a pyrimidine core.

Despite the fact that various inhibitors of CDKs are known, there remains a need for selective CDK9 inhibitors to be used for the treatment of diseases such as hyper-proliferative diseases, viral diseases, and/or diseases of the heart, which offer one or more advantages over the compounds known from prior art, such as :
- improved activity and / or efficacy
- beneficial kinase selectivity profile according to the respective therapeutic need
- improved side effect profile, such as fewer undesired side effects, lower intensity of side effects, or reduced (cyto)toxicity
- improved physicochemical properties, such as solubility in water and body fluids
- improved pharmacokinetic properties, allowing e.g. for dose reduction or an easier dosing scheme
- easier drug substance manufacturing e.g. by shorter synthetic routes or easier purification.

A particular object of the invention is to provide CDK9 kinase inhibitors which, compared to the compounds known from prior art, show an increased selectivity for CDK9/Cyclin T1 as compared to CDK2/Cyclin E.

Another object of the invention is to provide CDK9 kinase inhibitors which show an increased potency to inhibit CDK9 activity (demonstrated by a lower IC₅₀ value for CDK9/Cyclin T1) compared to the compounds known from prior art.

Another object of the invention is to provide CDK9 kinase inhibitors which show an increased potency to inhibit CDK9 activity at high ATP concentrations compared to the compounds known from prior art.

Another object of the invention is to provide CDK9 kinase inhibitors, which show an improved anti-proliferative activity in tumor cell lines such as HeLa compared to the compounds known from prior art.

Further, it is also an object of the present invention to provide CDK9 kinase inhibitors, which, compared to the compounds known from prior art, are highly selective for CDK9/Cyclin T1 as compared to CDK2/Cyclin E, and/or which show an increased potency to inhibit CDK9 activity and/or which show an improved anti-proliferative activity in tumor cell lines such as HeLa and/or which show an increased potency to inhibit CDK9 activity at high ATP concentrations compared to the compounds known from prior art.

The selectivity CDK9 / CDK2 is preferred to be greater than 5, more preferred greater than 10, even more preferred greater than 15, particularly preferred greater than 25 and most preferred greater than 100.

The present invention relates to compounds of general formula (I) 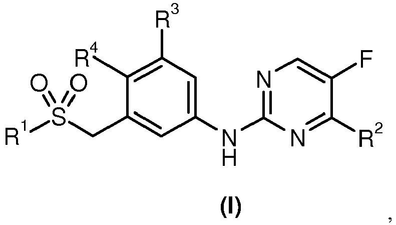 wherein
- R¹: represents a group selected from C₁-C₆-alkyl-, C₃-C₇-cycloalkyl-, heterocyclyl-, phenyl, heteroaryl, phenyl-C₁-C₃-alkyl- or heteroaryl-C₁-C₃-alkyl-,
wherein said group is optionally substituted with one or two or three substituents, identically or differently, selected from the group of hydroxy, cyano, halogen, halo-C₁-C₃-alkyl-, C₁-C₆-alkoxy-, C₁-C₃-fluoroalkoxy-, amino, alkylamino-, dialkylamino-, acetylamino-, N-methyl-N-acetylamino-, cyclic amines, -OP(O)(OH)₂, -C(O)OH, -C(O)NH₂;
- R²: represents a group

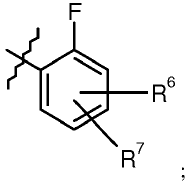

- R³, R⁴: represent, independently from each other, a group selected from a hydrogen atom, halogen atom, cyano, SF₅, C₁-C₃-alkyl-, C₁-C₃-alkoxy-, hydroxy, halo-C₁-C₃-alkyl-, C₁-C₃-fluoroalkoxy-,
- R⁶, R⁷: represent, independently from each other, a group selected from a hydrogen atom, fluoro atom, chloro atom, C₁-C₃-alkyl-, C₁-C₃-alkoxy-, halo-C₁-C₃-alkyl-, C₁-C₃-fluoroalkoxy-;
or their salts, solvates or salts of solvates,
with the proviso that the compound is not
4-(2,4-Difluorophenyl)-5-fluoro-N-{3-[(methylsulfonyl)methyl]phenyl}pyrimidin-2-amine.

Compounds according to the invention are the compounds of the formula (I) and the salts, solvates and solvates of the salts thereof, the compounds of the hereinafter recited formula which are encompassed by formula (I) and the salts, solvates and solvates of the salts thereof, and the compounds which are encompassed by formula (I) and are mentioned hereinafter as exemplary embodiments and the salts, solvates and solvates of the salts thereof, where the compounds which are encompassed by formula (I) and are mentioned hereinafter are not already salts, solvates and solvates of the salts.

The compounds according to the invention may, depending on their structure, exist in stereoisomeric forms (enantiomers, diastereomers). The invention therefore relates to the enantiomers or diastereomers and respective mixtures thereof. The stereoisomerically pure constituents can be isolated in a known manner from such mixtures of enantiomers and/or diastereomers.

If the compounds according to the invention can be in tautomeric forms, the present invention encompasses all tautomeric forms.

Further, the compounds of the present invention can exist in free form, e.g. as a free base, or as a free acid, or as a zwitterion, or can exist in the form of a salt. Said salt may be any salt, either an organic or inorganic addition salt, particularly any physiologically acceptable organic or inorganic addition salt, customarily used in pharmacy.

Salts which are preferred for the purposes of the present invention are physiologically acceptable salts of the compounds according to the invention. However, salts which are not suitable for pharmaceutical applications per se, but which, for example, can be used for the isolation or purification of the compounds according to the invention, are also comprised.
The term "physiologically acceptable salt" refers to a relatively non-toxic, inorganic or organic acid addition salt of a compound of the present invention, for example, see S. M. Berge, et al. "Pharmaceutical Salts," J. Pharm. Sci. 1977, 66, 1-19.

Physiologically acceptable salts of the compounds according to the invention encompass acid addition salts of mineral acids, carboxylic acids and sulfonic acids, for example salts of hydrochloric acid, hydrobromic acid, hydroiodic, sulfuric acid, bisulfuric acid, phosphoric acid, nitric acid or with an organic acid, such as formic, acetic, acetoacetic, pyruvic, trifluoroacetic, propionic, butyric, hexanoic, heptanoic, undecanoic, lauric, benzoic, salicylic, 2-(4-hydroxybenzoyl)-benzoic, camphoric, cinnamic, cyclopentanepropionic, digluconic, 3-hydroxy-2-naphthoic, nicotinic, pamoic, pectinic, persulfuric, 3-phenylpropionic, picric, pivalic, 2-hydroxyethanesulfonate, itaconic, sulfamic, trifluoromethanesulfonic, dodecylsulfuric, ethansulfonic, benzenesulfonic, para-toluenesulfonic, methansulfonic, 2-naphthalenesulfonic, naphthalinedisulfonic, camphorsulfonic acid, citric, tartaric, stearic, lactic, oxalic, masonic, succinic, malic, adipic, alginic, maleic, fumaric, D-gluconic, mandelic, ascorbic, glucoheptanoic, glycerophosphoric, aspartic, sulfosalicylic, hemisulfuric, or thiocyanic acid, for example.

Physiologically acceptable salts of the compounds according to the invention also comprise salts of conventional bases, such as, by way of example and by preference, alkali metal salts (for example sodium and potassium salts), alkaline earth metal salts (for example calcium and magnesium salts) and ammonium salts derived from ammonia or organic amines with 1 to 16 C atoms, such as, by way of example and by preference, ethylamine, diethylamine, triethylamine, ethyldiisopropylamine, monoethanolamine, diethanolamine, triethanolamine, dicyclohexylamine, dimethylaminoethanol, procaine, dibenzylamine, *N*-methylmorpholine, arginine, lysine, ethylenediamine, *N*-methylpiperidine, *N*-methylglucamine, dimethylglucamine, ethylglucamine, 1,6-hexadiamine, glucosamine, sarcosine, serinol, tris(hydroxymethyl)aminomethane, aminopropanediol, Sovak base, and 1-amino-2,3,4-butanetriol. Additionally, the compounds according to the invention may form salts with a quarternary ammonium ion obtainable e.g. by quarternisation of a basic nitrogen containing group with agents like lower alkylhalides such as methyl-, ethyl-, propyl-, and butylchlorides, -bromides and -iodides ; dialkylsulfates like dimethyl-, diethyl-, dibutyl- and diamylsulfates, long chain halides such as decyl-, lauryl-, myristyl- and stearylchlorides, -bromides and -iodides, aralkylhalides like benzyl- and phenethylbromides and others. Examples of suitable quarternary ammonium ions are tetramethylammonium, tetraethylammonium, tetra(*n*-propyl)ammonium, tetra (*n*-butyl)ammonium, or *N*-benzyl-*N,N,N*-trimethylammonium.
The present invention includes all possible salts of the compounds of the present invention as single salts, or as any mixture of said salts, in any ratio.

Solvates is the term used for the purposes of the invention for those forms of the compounds according to the invention which form a complex with solvent molecules by coordination in the solid or liquid state. Hydrates are a special form of solvates in which the coordination takes place with water. Hydrates are preferred as solvates within the scope of the present invention.

The invention also includes all suitable isotopic variations of a compound of the invention. An isotopic variation of a compound of the invention is defined as one in which at least one atom is replaced by an atom having the same atomic number but an atomic mass different from the atomic mass usually or predominantly found in nature. Examples of isotopes that can be incorporated into a compound of the invention include isotopes of hydrogen, carbon, nitrogen, oxygen, phosphorus, sulfur, fluorine, chlorine, bromine and iodine, such as ²H (deuterium), ³H (tritium), ¹³C, ¹⁴C, ¹⁵N, ¹⁷O, ¹⁸O, ³²P, ³³P, ³³S, ³⁴S, ³⁵S, ³⁶S, ¹⁸F , ³⁶Cl, ⁸²Br, ¹²³I, ¹²⁴I, ¹²⁹I and ¹³¹I, respectively. Certain isotopic variations of a compound of the invention, for example, those in which one or more radioactive isotopes such as ³H or ¹⁴C are incorporated, are useful in drug and/or substrate tissue distribution studies. Tritiated and carbon-14, i.e., ¹⁴C, isotopes are particularly preferred for their ease of preparation and detectability. Further, substitution with isotopes such as deuterium may afford certain therapeutic advantages resulting from greater metabolic stability, for example, increased in vivo half-life or reduced dosage requirements and hence may be preferred in some circumstances. Isotopic variations of a compound of the invention can generally be prepared by conventional procedures known by a person skilled in the art such as by the illustrative methods or by the preparations described in the examples hereafter using appropriate isotopic variations of suitable reagents.

Furthermore, the present invention includes all possible crystalline forms, or polymorphs, of the compounds of the present invention, either as single polymorphs, or as a mixture of more than one polymorphs, in any ratio.

Accordingly, the present invention includes all possible salts, polymorphs, hydrates, solvates, and diastereoisomeric forms of the the compounds of the present invention as single salt, polymorph, hydrate, solvate, or diastereoisomeric form, or as mixture of more than one salt, polymorph, hydrate, solvate, or diastereoisomeric form in any ratio.

For the purposes of the present invention, the substituents have the following meaning, unless otherwise specified:
The terms "halogen", "halogen atom" or "halo" represent fluorine, chlorine, bromine and iodine, particularly chlorine or fluorine, preferably fluorine.

The term "alkyl" represents a linear or branched alkyl radical having the number of carbon atoms specifically indicated, e.g. C₁-C₁₀ one, two, three, four, five, six, seven, eight, nine or ten carbon atoms, e.g. methyl, ethyl, *n*-propyl, isopropyl, *n*-butyl, isobutyl, *sec*-butyl, *tert*-butyl, pentyl, isopentyl, hexyl, heptyl, octyl, nonyl-, decyl-, 2-methylbutyl, 1-methylbutyl, 1-ethylpropyl, 1,2-dimethylpropyl, *neo-*pentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl. If the number of carbon atoms is not specifically indicated the term "alkyl" represents a linear or branched alkyl radical having, as a rule, 1 to 9, particularly 1 to 6, preferably 1 to 4 carbon atoms. Particularly, the alkyl group has 1, 2, 3, 4, 5 or 6 carbon atoms ("C₁-C₆-alkyl"), e.g. methyl, ethyl, *n*-propyl-, isopropyl, *n*-butyl, *tert*-butyl, pentyl, isopentyl, hexyl, 2-methylbutyl, 1-methylbutyl, 1-ethylpropyl, 1,2-dimethylpropyl, *neo*-pentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, or 1,2-dimethylbutyl. Preferably, the alkyl group has 1, 2 or 3 carbon atoms ("C₁-C₃-alkyl"), methyl, ethyl, *n-*propyl or isopropyl.

The term "C₃-C₇-cycloalkyl" is to be understood as preferably meaning a saturated, monovalent, monocyclic hydrocarbon ring which contains 3, 4, 5, 6 or 7 carbon atoms. Said C₃-C₇-cycloalkyl group is for example, a monocyclic hydrocarbon ring, *e.g.* a cyclopropyl, cyclobutyl, cyclopentyl, cyclohexyl or cycloheptyl group. Said cycloalkyl ring can optionally contain one or more double bonds *e.g.* cycloalkenyl, such as a cyclopropenyl, cyclobutenyl, cyclopentenyl, cyclohexenyl or cycloheptenyl group, wherein the bond between said ring with the rest of the molecule may be to any carbon atom of said ring, be it saturated or unsaturated. Particularly, said cycloalkyl group is a C₄-C₆-cycloalkyl, a C₅-C₆-cycloalkyl or a cyclohexyl group.

The term "C₃-C₅-cycloalkyl" is to be understood as preferably meaning a saturated, monovalent, monocyclic hydrocarbon ring which contains 3, 4 or 5 carbon atoms. In particular said C₃-C₅-cycloalkyl group is a monocyclic hydrocarbon ring such as a cyclopropyl, cyclobutyl or cyclopentyl group. Preferably said "C₃-C₅-cycloalkyl" group is a cyclopropyl group.

The term "C₃-C₆-cycloalkyl" is to be understood as preferably meaning a saturated, monovalent, monocyclic hydrocarbon ring which contains 3, 4, 5 or 6 carbon atoms. In particular said C₃-C₅-cycloalkyl group is a monocyclic hydrocarbon ring such as a cyclopropyl, cyclobutyl, cyclopentyl or cyclohexyl group.

The term "heterocyclyl" is to be understood as meaning a saturated or partially unsaturated, monovalent, mono- or bicyclic hydrocarbon ring which contains 3, 4, 5, 6, 7, 8 or 9 carbon atoms and further containing 1, 2 or 3 heteroatom-containing groups selected from oxygen, sulfur, nitrogen. Particularly, the term "heterocyclyl" is to be understood as meaning a "4- to 10-membered heterocyclic ring".

The term "a 4- to 10-membered heterocyclic ring" is to be understood as meaning a saturated or partially unsaturated, monovalent, mono- or bicyclic hydrocarbon ring which contains 3, 4, 5, 6, 7, 8 or 9 carbon atoms, and further containing 1, 2 or 3 heteroatom-containing groups selected from oxygen, sulfur, nitrogen. A C₃-C₉-heterocyclyl is to be understood as meaning a heterocyclyl which contains at least 3, 4, 5, 6, 7, 8 or 9 carbon atoms and additionally at least one heteroatom as ring atoms. Accordingly in case of one heteroatom the ring is 4- to 10-membered, in case of two heteroatoms the ring is 5- to 11-membered and in case of three heteroatoms the ring is 6- to 12-membered.

Said heterocyclic ring is for example, a monocyclic heterocyclic ring such as an oxetanyl, azetidinyl, tetrahydrofuranyl, pyrrolidinyl, 1,3-dioxolanyl, imidazolidinyl, pyrazolidinyl, oxazolidinyl, isoxazolidinyl, 1,4-dioxanyl, pyrrolinyl, tetrahydropyranyl, piperidinyl, morpholinyl, 1,3-dithianyl, thiomorpholinyl, piperazinyl, or chinuclidinyl group. Optionally, said heterocyclclic ring can contain one or more double bonds, *e.g.* 4*H*-pyranyl, 2*H*-pyranyl, 2,5-dihydro-1*H*-pyrrolyl, 1,3-dioxolyl, 4*H*-1,3,4-thiadiazinyl, 2,5-dihydrofuranyl, 2,3-dihydrofuranyl, 2,5-dihydrothienyl, 2,3-dihydrothienyl, 4,5-dihydrooxazolyl, 4,5-dihydroisoxazolyl, or 4*H*-1,4-thiazinyl group, or, it may be benzo fused.

Particularly a C₃-C₇-heterocyclyl is to be understood as meaning a heterocyclyl which contains at least 3, 4, 5, 6, or 7 carbon atoms and additionally at least one heteroatom as ring atoms. Accordingly in case of one heteroatom the ring is 4- to 8-membered, in case of two heteroatoms the ring is 5- to 9-membered and in case of three heteroatoms the ring is 6- to 10-membered.

Particularly a C₃-C₆-heterocyclyl is to be understood as meaning a heterocyclyl which contains at least 3, 4, 5 or 6 carbon atoms and additionally at least one heteroatom as ring atoms. Accordingly in case of one heteroatom the ring is 4- to 7-membered, in case of two heteroatoms the ring is 5- to 8-membered and in case of three heteroatoms the ring is 6- to 9-membered.

Particularly, the term "heterocyclyl" is to be understood as being a heterocyclic ring which contains 3, 4 or 5 carbon atoms, and 1, 2 or 3 of the above-mentioned heteroatom-containing groups (a "4- to 7-membered heterocyclic ring"), more particularly said ring can contain 4 or 5 carbon atoms, and 1, 2 or 3 of the above-mentioned heteroatom-containing groups (a "5- to 7-membered heterocyclic ring"), more particularly said heterocyclic ring is a "6-membered heterocyclic ring", which is to be understood as containing 4 carbon atoms and 2 of the above-mentioned heteroatom-containing groups or 5 carbon atoms and one of the above-mentioned heteroatom-containing groups, preferably 4 carbon atoms and 2 of the above-mentioned heteroatom-containing groups.

The term "C₁-C₆-alkoxy-" is to be understood as preferably meaning a linear or branched, saturated, monovalent, hydrocarbon group of formula -0-alkyl, in which the term "alkyl" is defined *supra*, *e.g.* a methoxy, ethoxy, *n*-propoxy, *iso*-propoxy, *n*-butoxy, *iso*-butoxy, *tert*-butoxy, *sec*-butoxy, pentyloxy, *iso-*pentyloxy, *n*-hexyloxy group, or an isomer thereof. Particularly, the "C₁-C₆-alkoxy-" group is a "C₁-C₄-alkoxy-", a "C₁-C₃-alkoxy-", a methoxy, ethoxy, or propoxy group, preferably a methoxy, ethoxy or propoxy group. Further preferred is a "C₁-C₂-alkoxy-" group, particularly a methoxy or ethoxy group.

The term "C₁-C₃-fluoroalkoxy-" is to be understood as preferably meaning a linear or branched, saturated, monovalent, C₁-C₃-alkoxy- group, as defined *supra,* in which one or more of the hydrogen atoms is replaced, identically or differently, by one or more fluoro atoms. Said C₁-C₃-fluoroalkoxy-group is, for example a 1,1-difluoromethoxy-, a 1,1,1-trifluoromethoxy-, a 2-fluoroethoxy-, a 3-fluoropropoxy-, a 2,2,2-trifluoroethoxy-, a 3,3,3-trifluoropropoxy- particularly a "C₁-C₂-fluoroalkoxy-" group.

The term "alkylamino-" is to be understood as preferably meaning an alkylamino group with one linear or branched alkyl group as defined supra. (C₁-C₃)-alkylamino- for example means a monoalkylamino group with 1, 2 oder 3 carbon atoms, (C₁-C₆)-alkylamino- with 1, 2, 3, 4, 5 or 6 carbon atoms. The term "alkylamino-" comprises for example methylamino-, ethylamino-, n-propylamino-, isopropylamino-, tert.-butylamino-, n-pentylamino- or n-hexylamino-.
The term "dialkylamino-" is to be understood as preferably meaning an alkylamino group having two linear or branched alkyl groups as defined supra, which are independent from each other. (C₁-C₃)-dialkylamino-for example represents a dialkylamino group with two alkyl groups each of them having 1 to 3 carbon atoms per alkyl group. The term "dialkylamino-" comprises for example: N,N-Dimethylamino-, N,N-Diethylamino-, N-Ethyl-N-methylamino-, N-Methyl-N-n-propylamino-, N-Isopropyl-N-n-propylamino-, N-t-Butyl-N-methylamino-, N-Ethyl-N-n-pentylamino- und N-n-Hexyl-N-methylamino-.

The term "cyclic amine" is to be understood as preferably meaning a cyclic amine group. Preferably, a cyclic amine means a saturated, monocyclic group with 4 to 10, preferably 4 to 7 ring atoms of which at least one ring atom is a nitrogen atom. Suitable cyclic amines are especially azetidine, pyrrolidine, piperidine, piperazine, 1-methylpiperazine, morpholine, thiomorpholine, which could be optionally substituted by one or two methyl groups.

The term "halo-C₁-C₃-alkyl-" is to be understood as preferably meaning a linear or branched, saturated, monovalent hydrocarbon group in which the term "C₁-C₃-alkyl" is defined supra, and in which one or more hydrogen atoms is replaced by a halogen atom, identically or differently, i.e. one halogen atom being independent from another. Particularly, said halogen atom is fluorine. Preferred halo-C₁-C₃-alkyl- group is a fluoro-C₁-C₃-alkyl-, or, used synonymously, a C₁-C₃-fluoroalkyl- group, such as for example -CF₃, -CHF₂,-CH₂F, -CF₂CF₃, or -CH₂CF₃, preferably it is -CF₃.

The term "phenyl-C₁-C₃-alkyl-" is to be understood as preferably meaning a phenyl group, in which one of the hydrogen atoms is replaced by a C₁-C₃-alkyl group, as defined supra, that links the phenyl-C₁-C₃-alkyl-group to the molecule. Particularly, the "phenyl-C₁-C₃-alkyl-" is a phenyl-C₁-C₂-alkyl-, preferably it is a benzyl- group.

The term "heteroaryl" is to be understood as preferably meaning a monovalent, aromatic ring system having 5, 6, 7, 8, 9, 10, 11, 12, 13 or 14 ring atoms (a "5- to 14-membered heteroaryl" group), particularly 5 (a "5-membered heteroaryl") or 6 (a "6-membered heteroaryl") or 9 (a"9-membered heteroaryl") or 10 ring atoms (a "10-membered heteroaryl"), and which contains at least one heteroatom which may be identical or different, said heteroatom being such as oxygen, nitrogen or sulfur, and can be monocyclic, bicyclic, or tricyclic, and in addition in each case can be benzo-condensed. Particularly, heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, tetrazolyl *etc.,* and benzo derivatives thereof, such as, for example, benzofuranyl, benzothienyl, benzoxazolyl, benzisoxazolyl, benzimidazolyl, benzotriazolyl, indazolyl, indolyl, isoindolyl, *etc.*; or pyridyl, pyridazinyl, pyrimidinyl, pyrazinyl, triazinyl, *etc.,* and benzo derivatives thereof, such as, for example, quinolinyl, quinazolinyl, isoquinolinyl, *etc.*; or azocinyl, indolizinyl, purinyl, *etc.,* and benzo derivatives thereof; or cinnolinyl, phthalazinyl, quinazolinyl, quinoxalinyl, naphthyridinyl, pteridinyl, carbazolyl, acridinyl, phenazinyl, phenothiazinyl, phenoxazinyl, xanthenyl, or oxepinyl, *etc.* Preferably, heteroaryl is selected from monocyclic heteroaryl, 5-membered heteroaryl or 6-membered heteroaryl.

The term "5-membered heteroaryl" is understood as preferably meaning a monovalent, aromatic ring system having 5 ring atoms and which contains at least one heteroatom which may be identical or different, said heteroatom being such as oxygen, nitrogen or sulfur. Particularly, "5-membered heteroaryl" is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, tetrazolyl.

The term "6-membered heteroaryl" is understood as preferably meaning a monovalent, aromatic ring system having 6 ring atoms and which contains at least one heteroatom which may be identical or different, said heteroatom being such as oxygen, nitrogen or sulfur. Particularly, "6-membered heteroaryl" is selected from pyridyl, pyridazinyl, pyrimidinyl, pyrazinyl, triazinyl.

The term "heteroaryl-C₁-C₃-alkyl-" is to be understood as preferably meaning a heteroaryl, a 5-membered heteroaryl or a 6-membered heteroaryl group, each as defined *supra*, in which one of the hydrogen atoms is replaced by a C₁-C₃-alkyl group, as defined *supra*, that links the heteroaryl-C₁-C₃-alkyl- group to the molecule. Particularly, the "heteroaryl-C₁-C₃-alkyl-" is a heteroaryl-C₁-C₂-alkyl-, a pyridinyl-C₁-C₃-alkyl-, a pyridinylmethyl-, a pyridinylethyl-, a pyridinylpropyl-, -a pyrimidinyl-C₁-C₃-alkyl-, a pyrimidinylmethyl-, a pyrimidinylethyl-, a pyrimidinylpropyl-, preferably a pyridinylmethyl- or a pyridinylethyl- or a pyrimidinylethyl- or a pyrimidinylpropyl- group.

The term "C₁-C₁₀", as used throughout this text, *e.g.* in the context of the definition of "C₁-C₁₀-alkyl" is to be understood as meaning an alkyl group having a finite number of carbon atoms of 1 to 10, *i.e. 1,* 2, 3, 4, 5, 6, 7, 8, 9 or 10 carbon atoms. It is to be understood further that said term "C₁-C₁₀" is to be interpreted as any sub-range comprised therein, *e.g.* C₁-C₁₀, C₁-C₉, C₁-C₈, C₁-C₇, C₁-C₆ C₁-C₅, C₁-C₄, C₁-C₃, C₁-C₂, C₂-C₁₀, C₂-C₉, C₂-C₈, C₂-C₇, C₂-C₆, C₂-C₅, C₂-C₄, C₂-C₃, C₃-C₁₀, C₃-C₉, C₃-C₈, C₃-C₇, C₃-C₆, C₃-C₅, C₃-C₄, C₄-C₁₀, C₄-C₉, C₄-C₈, C₄-C₇, C₄-C₆, C₄-C₅, C₅-C₁₀, C₅-C₉, C₅-C₈, C₅-C₇, C₅-C₆, C₆-C₁₀, C₆-C₉, C₆-C₈, C₆-C₇, C₇-C₁₀, C₇-C₉, C₇-C₈, C₈-C₁₀, C₈-C₉, C₉-C₁₀.

Similarly, as used herein, the term "C₁-C₆", as used throughout this text, *e.g.* in the context of the definition of "C₁-C₆-alkyl", "C₁-C₆-alkoxy" is to be understood as meaning an alkyl group having a finite number of carbon atoms of 1 to 6, *i.e.* 1, 2, 3, 4, 5 or 6 carbon atoms. It is to be understood further that said term "C₁-C₆" is to be interpreted as any sub-range comprised therein, *e.g.* C₁-C₆ C₁-C₅, C₁-C₄, C₁-C₃, C₁-C₂, C₂-C₆, C₂-C₅, C₂-C₄, C₂-C₃, C₃-C₆, C₃-C₅, C₃-C₄, C₄-C₆, C₄-C₅, C₅-C₆.

Similarly, as used herein, the term "C₁-C₃", as used throughout this text, *e.g.* in the context of the definition of "C₁-C₃-alkyl", "C₁-C₃-alkoxy" or "C₁-C₃-fluoroalkoxy" is to be understood as meaning an alkyl group having a finite number of carbon atoms of 1 to 3, *i.e.* 1, 2 or 3 carbon atoms. It is to be understood further that said term "C₁-C₃" is to be interpreted as any sub-range comprised therein, e.g. C₁-C₃, C₁-C₂, C₂-C₃.

Further, as used herein, the term "C₃-C₆", as used throughout this text, e.g. in the context of the definition of "C₃-C₆-cycloalkyl", is to be understood as meaning a cycloalkyl group having a finite number of carbon atoms of 3 to 6, i.e. 3, 4, 5 or 6 carbon atoms. It is to be understood further that said term "C₃-C₆" is to be interpreted as any sub-range comprised therein, e.g. C₃-C₆, C₃-C₅, C₃-C₄, C₄-C₆, C₄-C₅, C₅-C₆. Further, as used herein, the term "C₃-C₇", as used throughout this text, *e.g.* in the context of the definition of "C₃-C₇-cycloalkyl", is to be understood as meaning a cycloalkyl group having a finite number of carbon atoms of 3 to 7, *i.e.* 3, 4, 5, 6 or 7 carbon atoms, particularly 3, 4, 5 or 6 carbon atoms. It is to be understood further that said term "C₃-C₇" is to be interpreted as any sub-range comprised therein, *e.g.* C₃-C₇ , C₃-C₆, C₃-C₅, C₃-C₄, C₄-C₇, C₄-C₆, C₄-C₅, C₅-C₇, C₅-C₆, C₆-C₇.

A symbol 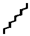 at a bond denotes the linkage site in the molecule.

As used herein, the term "one or more times", *e.g.* in the definition of the substituents of the compounds of the general formulae of the present invention, is understood as meaning one, two, three, four or five times, particularly one, two, three or four times, more particularly one, two or three times, even more particularly one or two times.

As used herein, the term "leaving group" refers to an atom or a group of atoms that is displaced in a chemical reaction as stable species taking with it the bonding electrons. Preferably, a leaving group is selected from the group comprising: halo, in particular chloro, bromo or iodo, methanesulfonyloxy, p-toluenesulfonyloxy, trifluoromethanesulfonyloxy, nonafluorobutanesulfonyloxy, (4-bromo-benzene)sulfonyloxy, (4-nitro-benzene)sulfonyloxy, (2-nitro-benzene)-sulfonyloxy, (4-isopropylbenzene)sulfonyloxy, (2,4,6-tri-isopropyl-benzene)-sulfonyloxy, (2,4,6-trimethyl-benzene)sulfonyloxy, (4-tertbutyl-benzene)sulfonyloxy, benzenesulfonyloxy, and (4-methoxy-benzene)sulfonyloxy.

Where the plural form of the word compounds, salts, hydrates, solvates and the like, is used herein, this is taken to mean also a single compound, salt, isomer, hydrate, solvate or the like.

In another embodiment the present invention concerns compounds of general formula (I),
- R¹: represents a group selected from C₁-C₆-alkyl-, C₃-C₇-cycloalkyl-, heterocyclyl-, phenyl, heteroaryl, phenyl-C₁-C₃-alkyl- or heteroaryl-C₁-C₃-alkyl-,
wherein said group is optionally substituted with one or two or three substituents, identically or differently, selected from the group of hydroxy, cyano, halogen, halo-C₁-C₃-alkyl-, C₁-C₆-alkoxy-, C₁-C₃-fluoroalkoxy-, amino, alkylamino-, dialkylamino-, acetylamino-, N-methyl-N-acetylamino-, cyclic amines, -OP(O)(OH)₂, -C(O)OH, -C(O)NH₂;
- R²: represents a group

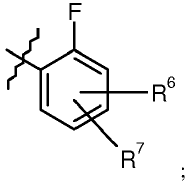

- R³: represents a group selected from a halogen atom, cyano, -SF₅, C₁-C₃-alkyl-, C₁-C₃-alkoxy-, hydroxy, halo-C₁-C₃-alkyl-, C₁-C₃-fluoroalkoxy-;
- R⁴: represents a group selected from a hydrogen atom, halogen atom, cyano, -SF₅, C₁-C₃-alkyl-, C₁-C₃-alkoxy-, hydroxy, halo-C₁-C₃-alkyl-, C₁-C₃-fluoroalkoxy-;
- R⁶, R⁷: represent, independently from each other, a group selected from a hydrogen atom, fluoro atom, chloro atom, C₁-C₃-alkyl-, C₁-C₃-alkoxy-, halo-C₁-C₃-alkyl-, C₁-C₃-fluoroalkoxy-;
or their salts, solvates or salts of solvates.

The present invention relates to compounds of general formula (I) 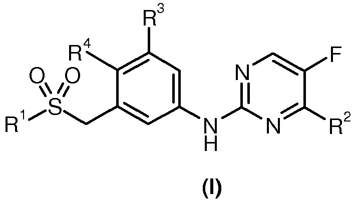 wherein
- R¹: represents a group selected from C₁-C₆-alkyl-, C₃-C₇-cycloalkyl-, heterocyclyl-, phenyl, heteroaryl, phenyl-C₁-C₃-alkyl- or heteroaryl-C₁-C₃-alkyl-,
wherein said group is optionally substituted with one or two or three substituents, identically or differently, selected from the group of hydroxy, cyano, halogen, halo-C₁-C₃-alkyl-, C₁-C₆-alkoxy-, C₁-C₃-fluoroalkoxy-, amino, alkylamino-, dialkylamino-, acetylamino-, N-methyl-N-acetylamino-, cyclic amines, -OP(O)(OH)₂, -C(O)OH, -C(O)NH₂;
- R²: represents a group

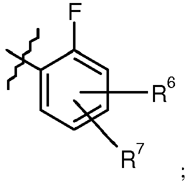

- R³: represents a trifluoromethyl group;
- R⁴: represents a group selected from a hydrogen atom, halogen atom, cyano, SF₅, C₁-C₃-alkyl-, C₁-C₃-alkoxy-, hydroxy, halo-C₁-C₃-alkyl-, C₁-C₃-fluoroalkoxy-,
- R⁶, R⁷: represent, independently from each other, a group selected from a hydrogen atom, fluoro atom, chloro atom, C₁-C₃-alkyl-, C₁-C₃-alkoxy-, halo-C₁-C₃-alkyl-, C₁-C₃-fluoroalkoxy-;
or their salts, solvates or salts of solvates,

In another embodiment the present invention concerns compounds of general formula (I), wherein
- R¹: represents a group selected from C₁-C₆-alkyl-, C₃-C₇-cycloalkyl, phenyl, or phenyl-C₁-C₃-alkyl-,
wherein said group is optionally substituted with one or two or three substituents, identically or differently, selected from the group of hydroxy, cyano, halogen, halo-C₁-C₃-alkyl-, C₁-C₃-alkoxy-, C₁-C₃-fluoroalkoxy-, amino, alkylamino-, dialkylamino-, acetylamino-, N-methyl-N-acetylamino-, cyclic amines, -OP(O)(OH)₂, -C(O)OH, -C(O)NH₂;
- R²: represents a group

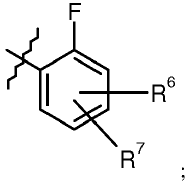

- R³: represents a group selected from a halogen atom, -SF₅, C₁-C₃-alkyl-, C₁-C₃-alkoxy-, halo-C₁-C₃-alkyl-, C₁-C₃-fluoroalkoxy-;
- R⁴: represents a group selected from a hydrogen atom, halogen atom, C₁-C₃-alkyl-, C₁-C₃-alkoxy-, halo-C₁-C₃-alkyl-, C₁-C₃-fluoroalkoxy-;
- R⁶, R⁷: represent, independently from each other, a group selected from a hydrogen atom, fluoro atom, chloro atom, C₁-C₃-alkyl-, C₁-C₃-alkoxy-, halo-C₁-C₃-alkyl-, C₁-C₃-fluoroalkoxy-;
or their salts, solvates or salts of solvates.

In another embodiment the present invention concerns compounds of general formula (I),
wherein
- R¹: represents a group selected from C₁-C₆-alkyl-, C₃-C₇-cycloalkyl, phenyl, or phenyl-C₁-C₃-alkyl-, wherein said group is optionally substituted with one or two or three substituents, identically or differently, selected from the group of hydroxy, cyano, halogen, halo-C₁-C₃-alkyl-, C₁-C₃-alkoxy-, C₁-C₃-fluoroalkoxy-, amino, alkylamino-, dialkylamino-, acetylamino-, N-methyl-N-acetylamino-, cyclic amines, -OP(O)(OH)₂, -C(O)OH, -C(O)NH₂;
- R²: represents a group

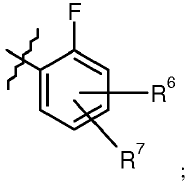

- R³, R⁴: represent, independently from each other, a group selected from a hydrogen atom, halogen atom, C₁-C₃-alkyl-, C₁-C₃-alkoxy-, halo-C₁-C₃-alkyl-, C₁-C₃-fluoroalkoxy-;
- R⁶, R⁷: represent, independently from each other, a group selected from a hydrogen atom, fluoro atom, chloro atom, C₁-C₃-alkyl-, C₁-C₃-alkoxy-, halo-C₁-C₃-alkyl-, C₁-C₃-fluoroalkoxy-;
or their salts, solvates or salts of solvates.

In another embodiment the present invention concerns compounds of general formula (I), wherein
- R¹: represents a group selected from C₁-C₆-alkyl-, C₃-C₇-cycloalkyl or phenyl-C₁-C₃-alkyl-, wherein said group is optionally substituted with one or two or three substituents, identically or differently, selected from the group of hydroxy or C₁-C₆-alkoxy,
- R²: represents a group

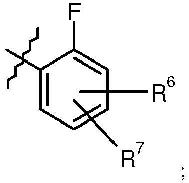

- R³, R⁴: represent, independently from each other, a group selected from a hydrogen or fluoro atom;
- R⁶, R⁷: represent, independently from each other, a group selected from a hydrogen atom, fluoro atom, chloro atom, C₁-C₃-alkyl-, C₁-C₃-alkoxy-, halo-C₁-C₃-alkyl-, C₁-C₃-fluoroalkoxy-;
or their salts, solvates or salts of solvates.

In another embodiment the present invention concerns compounds of general formula (I), wherein
- R¹: represents a group selected from C₁-C₆-alkyl- or C₃-C₅-cycloalkyl, wherein said group is optionally substituted with one substituent selected from the group of amino, C₁-C₃-alkoxy, hydroxy, -OP(O)(OH)₂;
- R²: represents a group

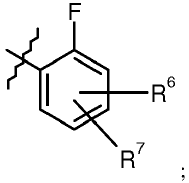

- R³: represents a group selected from a halogen atom, -SF₅ or halo-C₁-C₃-alkyl-;
- R⁴: represents a hydrogen atom or a fluoro atom;
- R⁶, R⁷: represent, independently from each other, a group selected from a hydrogen atom, fluoro atom,
or their salts, solvates or salts of solvates.

In another embodiment the present invention concerns compounds of general formula (I), wherein
- R¹: represents a group selected from C₁-C₆-alkyl-, wherein said group is optionally substituted with one substituent selected from the group of amino, hydroxy, -OP(O)(OH)₂;

- R²: represents a group

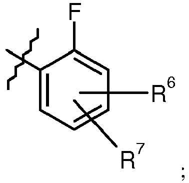

- R³: represents a group selected from a hydrogen atom, halogen atom, or halo-C₁-C₃-alkyl-;
- R⁴: represents a hydrogen atom or a fluoro atom;
- R⁶, R⁷: represent, independently from each other, a group selected from a hydrogen atom, fluoro atom,
or their salts, solvates or salts of solvates.

In another embodiment the present invention concerns compounds of general formula (I), wherein
- R¹: represents a group selected from C₁-C₆-alkyl-, C₃-C₅-cycloalkyl-, wherein said group is optionally substituted with one or two substituents, identically or differently, selected from the group of hydroxy, C₁-C₂-alkoxy-, halo-C₁-C₂-alkyl-, C₁-C₂-fluoroalkoxy-;
- R²: represents a group

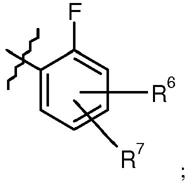

- R³: represents a hydrogen atom or a fluoro atom;
- R⁴: represents a hydrogen atom or a fluoro atom;
- R⁶, R⁷: represent, independently from each other, a group selected from a hydrogen atom, fluoro atom, chloro atom, C₁-C₃-alkyl-, C₁-C₃-alkoxy-, halo-C₁-C₃-alkyl-, C₁-C₃-fluoroalkoxy-;
or their salts, solvates or salts of solvates.

In another embodiment the present invention concerns compounds of general formula (I), wherein
- R¹: represents a group selected from C₁-C₄-alkyl-, C₃-C₆-cycloalkyl or phenyl-C₁-C₂-alkyl-, wherein said group is optionally substituted with one or two or three substituents, identically or differently, selected from the group of hydroxy or C₁-C₃-alkoxy,
- R²: represents a group

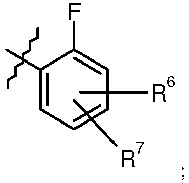

- R³, R⁴: represent, independently from each other a group selected from a hydrogen or fluoro atom,
- R⁶, R⁷: represent, independently from each other, a group selected from a hydrogen or fluoro atom or C₁-C₃-alkoxy-,
or their salts, solvates or salts of solvates.

In another embodiment the present invention concerns compounds of general formula (I), wherein
- R¹: represents a group selected from C₁-C₆-alkyl-, C₃-C₅-cycloalkyl-, wherein said group is optionally substituted with one or two substituents, identically or differently, selected from the group of hydroxy, C₁-C₆-alkoxy-;
- R²: represents a group

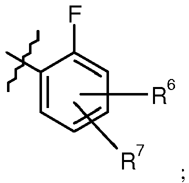

- R³: represents a hydrogen atom or fluoro atom;
- R⁴: represents a hydrogen atom or a fluoro atom;
- R⁶, R⁷: represent, independently from each other, a group selected from a hydrogen atom or a fluoro atom;
or their salts, solvates or salts of solvates.

In another embodiment the present invention concerns compounds of general formula (I), wherein
- R¹: represents a group selected from methyl, ethyl, isopropyl-, cyclopropyl, tert-butyl-, cyclohexyl,
wherein said group is optionally substituted with one substituent selected from the group of hydroxy, methoxy-, -OP(O)(OH)₂;
- R²: represents a group

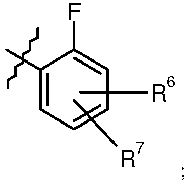

- R³: represents a hydrogen atom or fluoro atom;
- R⁴: represents a hydrogen atom;
- R⁶, R⁷: represent, independently from each other, a group selected from a hydrogen atom or fluoro atom;
or their salts, solvates or salts of solvates.

In another embodiment the present invention concerns compounds of general formula (I), wherein
- R¹: represents a group selected from C₁-C₆-alkyl-, C₃-C₅-cycloalkyl-,
wherein said group is optionally substituted with one or two substituents, identically or differently, selected from the group of hydroxy, C₁-C₆-alkoxy-;
- R²: represents a group

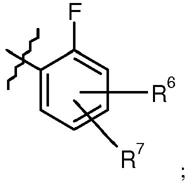

- R³: represents a hydrogen atom or fluoro atom;
- R⁴: represents a hydrogen atom;
- R⁶, R⁷: represent, independently from each other, a group selected from a hydrogen atom or fluoro atom;
or their salts, solvates or salts of solvates.

In another embodiment the present invention concerns compounds of general formula (I),
R¹ represents a group selected from C₁-C₃-alkyl-, C₃-C₅-cycloalkyl- or phenyl-C₁-C₂-alkyl-, wherein said group is optionally substituted with one or two or three substituents, identically or differently, selected from the group of hydroxy or methoxy,
R² represents a group 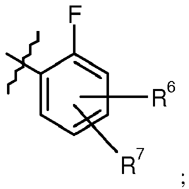 R³ represents a hydrogen or fluoro atom,
R⁴ represents a hydrogen atom,
R⁶, R⁷ represent, independently from each other, a group selected from a hydrogen or fluoro atom, or their salts, solvates or salts of solvates.

In a preferred embodiment the present invention concerns compounds of general formula (I), wherein
- R¹: represents a C₁-C₄-alkyl or cyclopropyl group, wherein said group is optionally substituted with one methoxy group;
- R²: represents a group

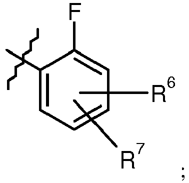

- R³: represents a group selected from a fluoro atom, -SF₅ or a fluoro-C₁-C₃-alkyl group;
- R⁴: represents a hydrogen atom;
- R⁶, R⁷: represent, independently from each other, a group selected from a hydrogen atom, fluoro atom;
or their salts, solvates or salts of solvates.

In a preferred embodiment the present invention concerns compounds of general formula (I), wherein
- R¹: represents a C₁-C₃-alkyl group;
- R²: represents a group

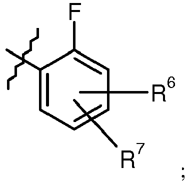

- R³: represents a group selected from a hydrogen atom or a C₁-C₃-fluoroalkyl group;
- R⁴: represents a hydrogen atom;
- R⁶, R⁷: represent, independently from each other, a group selected from a hydrogen atom, fluoro atom;
or their salts, solvates or salts of solvates.

In another preferred embodiment the present invention concerns compounds of general formula (I), wherein
- R¹: represents a methyl group;
- R²: represents a group

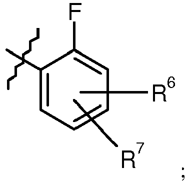

- R³: represents a hydrogen atom or fluoro atom;
- R⁴: represents a hydrogen atom;
- R⁶, R⁷: represent, independently from each other, a group selected from a hydrogen atom or fluoro atom;
or their salts, solvates or salts of solvates.

In a particularly preferred embodiment the present invention concerns compounds of general formula (I), wherein
- R¹: represents a group selected from methyl, ethyl, *n*-propyl, *iso*-propyl, *tert*-butyl, cyclopropyl or 2-methoxyethyl;
- R²: represents a group selected from 2,4-difluorophenyl, 2,3,4-trifluorophenyl or 2,4,5-trifluorophenyl;
- R³: represents a fluoro atom, -SF₅ or a trifluoromethyl group;
- R⁴: represents a hydrogen atom;
or their salts, solvates or salts of solvates.

In a particularly preferred embodiment the present invention concerns compounds of general formula (I), wherein
- R¹: represents a methyl or isopropyl group;
- R²: represents a 2,4-difluorophenyl group;
- R³: represents a hydrogen atom or a trifluoromethyl group;
- R⁴: represents a hydrogen atom;
or their salts, solvates or salts of solvates.

In another embodiment the invention relates to compounds of formula (I), in which R¹ represents a C₁-C₆-alkyl-, a C₃-C₇-cycloalkyl-, a heterocyclyl-, a phenyl, a heteroaryl, a phenyl-C₁-C₃-alkyl- or a heteroaryl-C₁-C₃-alkyl- group,
wherein said group is optionally substituted with one or two or three substituents, identically or differently, selected from the group of hydroxy, cyano, halogen, halo-C₁-C₃-alkyl-, C₁-C₆-alkoxy-, C₁-C₃-fluoroalkoxy-, amino, alkylamino-, dialkylamino-, acetylamino-, N-methyl-N-acetylamino-, cyclic amines, -OP(O)(OH)₂, -C(O)OH, -C(O)NH₂.

In another embodiment the invention relates to compounds of formula (I), in which R¹ represents a C₁-C₃-alkyl-, a C₃-C₅-cycloalkyl-, a 4- to 7-membered heterocyclic ring, a phenyl, a heteroaryl, a phenyl-C₁-C₂-alkyl- or a heteroaryl-C₁-C₂-alkyl- group,
wherein said group is optionally substituted with one or two or three substituents, identically or differently, selected from the group of hydroxy, cyano, halogen, halo-C₁-C₂-alkyl-, C₁-C₃-alkoxy-, C₁-C₂-fluoroalkoxy-, amino, alkylamino-, dialkylamino-, acetylamino-, N-methyl-N-acetylamino-, cyclic amines, -OP(O)(OH)₂, -C(O)OH, -C(O)NH₂.

In another embodiment the invention relates to compounds of formula (I), in which R¹ represents a phenyl or a heteroaryl group,
wherein said group is optionally substituted with one or two or three substituents, identically or differently, selected from the group of hydroxy, cyano, halogen, halo-C₁-C₂-alkyl-, C₁-C₃-alkoxy-, C₁-C₂-fluoroalkoxy-, amino, alkylamino-, dialkylamino-, acetylamino-, N-methyl-N-acetylamino-, cyclic amines, -OP(O)(OH)₂, -C(O)OH, -C(O)NH₂.

In another embodiment the invention relates to compounds of formula (I), in which R¹ represents a group selected from C₁-C₆-alkyl-, C₃-C₇-cycloalkyl, phenyl, or phenyl-C₁-C₃-alkyl-,
wherein said group is optionally substituted with one or two or three substituents, identically or differently, selected from the group of hydroxy, cyano, halogen, halo-C₁-C₃-alkyl-, C₁-C₃-alkoxy-, C₁-C₃-fluoroalkoxy-, amino, alkylamino-, dialkylamino-, acetylamino-, N-methyl-N-acetylamino-, cyclic amines., -OP(O)(OH)₂, -C(O)OH, -C(O)NH₂.

In another embodiment the invention relates to compounds of formula (I), in which R¹ represents a group selected from methyl, ethyl, isopropyl, cyclopropyl, *tert*-butyl, cyclopentyl, cyclohexyl or phenyl;
wherein said group is optionally substituted with one substituent selected from the group of hydroxyl, methoxy, -OP(O)(OH)₂.

In another embodiment the invention relates to compounds of formula (I), in which R¹ represents a group selected from C₁-C₆-alkyl-, C₃-C₇-cycloalkyl or phenyl-C₁-C₃-alkyl-,
wherein said group is optionally substituted with one or two or three substituents, identically or differently, selected from the group of hydroxy or C₁-C₆-alkoxy.

In another embodiment the invention relates to compounds of formula (I), in which R¹ represents a group selected from C₁-C₄-alkyl-, C₃-C₆-cycloalkyl or phenyl-C₁-C₂-alkyl-,
wherein said group is optionally substituted with one or two or three substituents, identically or differently, selected from the group of hydroxy or C₁-C₃-alkoxy.

In another embodiment the invention relates to compounds of formula (I), in which R¹ represents a group selected from C₁-C₆-alkyl- or C₃-C₅cycloalkyl, wherein said group is optionally substituted with one substituent selected from the group of amino, C₁-C₃-alkoxy, hydroxy, -OP(O)(OH)₂.

In another embodiment the invention relates to compounds of formula (I), in which R¹ represents a group selected from C₁-C₆-alkyl-, wherein said group is optionally substituted with one substituent selected from the group of amino, hydroxy, -OP(O)(OH)2.

In a preferred embodiment the invention relates to compounds of formula (I), in which R¹ represents a C₁-C₄-alkyl or cyclopropyl group, wherein said group is optionally substituted with one methoxy group.

In a preferred embodiment the invention relates to compounds of formula (I), in which R¹ represents a C₁-C₃-alkyl group.

In a particularly preferred embodiment the invention relates to compounds of formula (I), in which R¹ represents a group selected from methyl, ethyl, *n*-propyl, *iso*-propyl, *tert*-butyl, cyclopropyl or 2-methoxyethyl.

In a particularly preferred embodiment the invention relates to compounds of formula (I), in which R¹ represents a methyl or isopropyl group.

In a particularly preferred embodiment the invention relates to compounds of formula (I), in which R¹ represents a methyl group.

In a particularly preferred embodiment the invention relates to compounds of formula (I), in which R¹ represents an isopropyl group.

In another embodiment the invention relates to compounds of formula (I), in which R² represents a group selected from

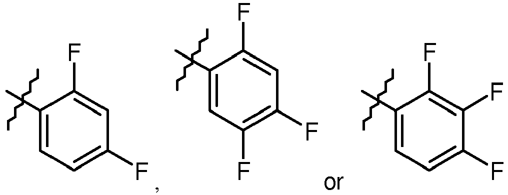

In another embodiment the invention relates to compounds of formula (I), in which R² represents a group selected from

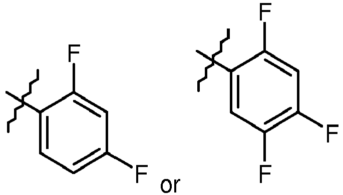

In another embodiment the invention relates to compounds of formula (I), in which R² represents

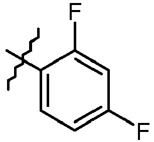

In another embodiment the invention relates to compounds of formula (I), in which R³ represents a group selected from a halogen atom, cyano, -SF₅, C₁-C₃-alkyl-, C₁-C₃-alkoxy-, hydroxy, halo-C₁-C₃-alkyl-, C₁-C₃-fluoroalkoxy-, and R⁴ represents a group selected from a hydrogen atom, halogen atom, cyano, - SF₅, C₁-C₃-alkyl-, C₁-C₃-alkoxy-, hydroxy, halo-C₁-C₃-alkyl-, C₁-C₃-fluoroalkoxy-.

In another embodiment the invention relates to compounds of formula (I), in which R³, R⁴ represent, independently from each other, a group selected from a hydrogen atom, halogen atom, cyano, SF₅, C₁-C₃-alkyl-, C₁-C₃-alkoxy-, hydroxy, halo-C₁-C₃-alkyl-, C₁-C₃-fluoroalkoxy-.

In another embodiment the invention relates to compounds of formula (I), in which R³ represents a group selected from a halogen atom, -SF₅, C₁-C₃-alkyl-, C₁-C₃-alkoxy-, halo-C₁-C₃-alkyl-, C₁-C₃-fluoroalkoxy-, and R⁴ represents a group selected from a hydrogen atom, halogen atom, C₁-C₃-alkyl-, C₁-C₃-alkoxy-, halo-C₁-C₃-alkyl-, C₁-C₃-fluoroalkoxy-.

In another embodiment the invention relates to compounds of formula (I), in which R³ and R⁴, represent, independently from each other, a group selected from a hydrogen atom, halogen atom, C₁-C₃-alkyl-, C₁-C₃-alkoxy-, halo-C₁-C₃-alkyl-, C₁-C₃-fluoroalkoxy-.

In another embodiment the invention relates to compounds of formula (I), in which R³ represents a group selected from a hydrogen atom, halogen atom, C₁-C₃-alkyl-, C₁-C₃-alkoxy-, halo-C₁-C₃-alkyl-, C₁-C₃-fluoroalkoxy-.

In another embodiment the invention relates to compounds of formula (I), in which R³ represents a group selected from a hydrogen atom, fluoro atom, chloro atom, C₁-C₂-alkyl-, C₁-C₂-alkoxy-, halo-C₁-C₂-alkyl-, C₁-C₂-fluoroalkoxy-.

In another embodiment the invention relates to compounds of formula (I), in which R³ represents a group selected from a hydrogen, a fluoro or a chloro atom.

In another embodiment the invention relates to compounds of formula (I), in which R³ and R⁴ represent independently from each other a group selected from a hydrogen or fluoro atom.

In another embodiment the invention relates to compounds of formula (I), in which R³ represents a group selected from a hydrogen atom, halogen atom, or halo-C₁-C₃-alkyl-.

In another embodiment the invention relates to compounds of formula (I), in which R³ represents a group selected from a halogen atom, -SF₅ or halo-C₁-C₃-alkyl-, and R⁴ represents a hydrogen atom or a fluoro atom.

In a preferred embodiment the invention relates to compounds of formula (I), in which R³ represents a group selected from a fluoro atom, -SF₅ or a fluoro-C₁-C₃-alkyl group, and R⁴ represents a hydrogen atom.

In a preferred embodiment the invention relates to compounds of formula (I), in which R³ represents a group selected from a hydrogen atom or a C₁-C₃-fluoroalkyl group, and in which R⁴ represents a hydrogen atom.

In a particularly preferred embodiment the invention relates to compounds of formula (I), in which R³ represents a fluoro atom, -SF₅ or a trifluoromethyl group, and R⁴ represents a hydrogen atom.

In a most preferred embodiment the invention relates to compounds of formula (I), in which R³ represents -SF₅, and R⁴ represents a hydrogen atom.

In a particularly preferred embodiment the invention relates to compounds of formula (I), in which R³ represents a hydrogen atom or a trifluoromethyl group, and in which R⁴ represents a hydrogen atom.

In another embodiment the invention relates to compounds of formula (I), in which R³ represents a hydrogen or fluoro atom and R⁴ represents a hydrogen atom.

In another embodiment the invention relates to compounds of formula (I), in which R³ represents a group selected from a halogen atom, cyano, -SF₅, C₁-C₃-alkyl-, C₁-C₃-alkoxy-, hydroxy, halo-C₁-C₃-alkyl-, C₁-C₃-fluoroalkoxy-.

In another embodiment the invention relates to compounds of formula (I), in which R³ represents a group selected from a halogen atom, -SF₅, C₁-C₃-alkyl-, C₁-C₃-alkoxy-, halo-C₁-C₃-alkyl-, C₁-C₃-fluoroalkoxy-.

In another embodiment the invention relates to compounds of formula (I), in which R³ represents a group selected from a halogen atom, -SF₅ or halo-C₁-C₃-alkyl-.

In another embodiment the invention relates to compounds of formula (I), in which R³ represents a halogen atom.

In a preferred embodiment the invention relates to compounds of formula (I), in which R³ represents a group selected from a fluoro atom, -SF₅ or a fluoro-C₁-C₃-alkyl group.

In another preferred embodiment the invention relates to compounds of formula (I), in which R³ represents a fluoro-C₁-C₃-alkyl group.

In a particularly preferred embodiment the invention relates to compounds of formula (I), in which R³ represents a fluoro atom, -SF₅ or a trifluoromethyl group.

In another particularly preferred embodiment the invention relates to compounds of formula (I), in which R³ represents a fluoro atom.

In a most preferred embodiment the invention relates to compounds of formula (I), in which R³ represents -SF₅.

In a preferred embodiment the invention relates to compounds of formula (I), in which R³ represents a group selected from a hydrogen atom or a C₁-C₃-fluoroalkyl group.

In a particularly preferred embodiment the invention relates to compounds of formula (I), in which R³ represents a hydrogen atom or a trifluoromethyl group.

In another particularly preferred embodiment the invention relates to compounds of formula (I), in which R³ represents a hydrogen atom.

In another particularly preferred embodiment the invention relates to compounds of formula (I), in which R³ represents a trifluoromethyl group;

In another embodiment the invention relates to compounds of formula (I), in which R⁴ represents a group selected from a hydrogen atom, halogen atom, cyano, -SF₅, C₁-C₃-alkyl-, C₁-C₃-alkoxy-, hydroxy, halo-C₁-C₃-alkyl-, C₁-C₃-fluoroalkoxy-.

In another embodiment the invention relates to compounds of formula (I), in which R⁴ represents a group selected from a hydrogen atom, halogen atom, C₁-C₃-alkyl-, C₁-C₃-alkoxy-, halo-C₁-C₃-alkyl-, C₁-C₃-fluoroalkoxy-.

In another embodiment the invention relates to compounds of formula (I), in which R⁴ represents a group selected from a hydrogen atom, fluoro atom, chloro atom, C₁-C₃-alkyl-, C₁-C₃-alkoxy-, halo-C₁-C₃-alkyl-, C₁-C₃-fluoroalkoxy-.

In another embodiment the invention relates to compounds of formula (I), in which R⁴ represents a group selected from a hydrogen atom, fluoro atom, chloro atom, C₁-C₂-alkyl-, C₁-C₂-alkoxy-, halo-C₁-C₂-alkyl-, C₁-C₂-fluoroalkoxy-.

In another embodiment the invention relates to compounds of formula (I), in which R⁴ represents a group selected from a hydrogen, a fluoro or a chloro atom.

In another embodiment the invention relates to compounds of formula (I), in which R⁴ represents a hydrogen atom or a fluoro atom.

In a preferred embodiment the invention relates to compounds of formula (I), in which R⁴ represents a fluoro atom.

In another preferred embodiment the invention relates to compounds of formula (I), in which R⁴ represents a hydrogen atom.

In another embodiment the invention relates to compounds of formula (I), in which R⁶, R⁷ represent, independently from each other, a group selected from a hydrogen atom, fluoro atom, chloro atom, C₁-C₃-alkyl-, C₁-C₃-alkoxy-, halo-C₁-C₃-alkyl-, C₁-C₃-fluoroalkoxy-;

In another embodiment the invention relates to compounds of formula (I), in which R⁶ and R⁷ represent, independently from each other, a group selected from a hydrogen atom, fluoro atom.

In another embodiment the invention relates to compounds of formula (I), in which R⁶ and R⁷ represent, independently from each other, a group selected from a hydrogen or fluoro atom or C₁-C₃-alkoxy-.

In another embodiment the invention relates to compounds of formula (I), in which R⁶ represents a fluoro atom and R⁷ represents a hydrogen atom.

In a particularly preferred embodiment the invention relates to compounds of formula (I), in which R⁶ is in *para* position to the 5-fluoro pyrimidine and represents a fluoro atom and R⁷ represents a hydrogen atom, or a fluoro atom in *meta* position to the 5-fluoro pyrimidine.

In a particularly preferred embodiment the invention relates to compounds of formula (I), in which R⁶ is in *para* position to the 5-fluoro pyrimidine and represents a fluoro atom and R⁷ represents a hydrogen atom.

In another embodiment the invention relates to compounds of formula (I), in which R⁶ represents a group selected from a hydrogen atom, fluoro atom, chloro atom, C₁-C₃-alkyl-, C₁-C₃-alkoxy-, halo-C₁-C₃-alkyl-, C₁-C₃-fluoroalkoxy-.

In another embodiment the invention relates to compounds of formula (I), in which R⁶ represents a group selected from a hydrogen atom, fluoro atom, chloro atom, C₁-C₂-alkyl-, C₁-C₂-alkoxy-, halo-C₁-C₂-alkyl-, C₁-C₂-fluoroalkoxy-.

In another embodiment the invention relates to compounds of formula (I), in which R⁶ represents a group selected from a hydrogen atom, fluoro atom, chloro atom.

In a preferred embodiment the invention relates to compounds of formula (I), in which R⁶ represents a hydrogen atom.

In another preferred embodiment the invention relates to compounds of formula (I), in which R⁶ represents a fluoro atom.

In a particularly preferred embodiment the invention relates to compounds of formula (I), in which R⁶ is in *para* position to the 5-fluoro pyrimidine and represents a fluoro atom.

In another embodiment the invention relates to compounds of formula (I), in which R⁷ represents a group selected from a hydrogen atom, fluoro atom, chloro atom, C₁-C₃-alkyl-, C₁-C₃-alkoxy-, halo-C₁-C₃-alkyl-, C₁-C₃-fluoroalkoxy-.

In another embodiment the invention relates to compounds of formula (I), in which R⁷ represents a group selected from a hydrogen atom, fluoro atom, chloro atom, C₁-C₂-alkyl-, C₁-C₂-alkoxy-, halo-C₁-C₂-alkyl-, C₁-C₂-fluoroalkoxy-.

In another embodiment the invention relates to compounds of formula (I), in which R⁷ represents a group selected from a hydrogen atom, fluoro atom, chloro atom.

In a particularly preferred embodiment the invention relates to compounds of formula (I), in which R⁷ represents a hydrogen atom, or a fluoro atom in *meta* position to the 5-fluoro pyrimidine.

In another particularly preferred embodiment the invention relates to compounds of formula (I), in which R⁷ represents a fluoro atom in *meta* position to the 5-fluoro pyrimidine.

In another particularly preferred embodiment the invention relates to compounds of formula (I), in which R⁷ represents a hydrogen atom.

It is to be understood that the present invention relates to any sub-combination within any embodiment of the present invention of compounds of formula (I), *supra.*

More particularly still, the present invention covers compounds of formula (I) which are disclosed in the Example section of this text, *infra.*

Very specially preferred are combinations of two or more of the abovementioned preferred embodiments.

In particular, preferred subjects of the present invention are the compounds selected from:
- 4-(2,4-Difluorophenyl)-5-fluoro-N-{3-[(methylsulfonyl)methyl]-5-(trifluoromethyl)phenyl}pyrimidin-2-amine
- 4-(2,4-Difluorophenyl)-5-fluoro-N-{3-[(propan-2-ylsulfonyl)methyl]phenyl}pyrimidin-2-amine
- N-{3-[(tert-Butylsulfonyl)methyl]phenyl}-4-(2,4-difluorophenyl)-5-fluoropyrimidin-2-amine
- 4-(2,4-Difluorophenyl)-5-fluoro-N-(3-{[(2-methoxyethyl)sulfonyl]methyl}phenyl)pyrimidin-2-amine
- 4-(2,4-Difluorophenyl)-5-fluoro-N-{3-[(methylsulfonyl)methyl]phenyl}pyrimidin-2-amine
- 4-(2,4-Difluorophenyl)-5-fluoro-N-{3-[(methylsulfonyl)methyl]-5-(pentafluoro-λ⁶-sulfanyl)phenyl}pyrimidin-2-amine
- 4-(2,4-Difluorophenyl)-N-{3-[(ethylsulfonyl)methyl]phenyl}-5-fluoropyrimidin-2-amine
- 4-(2,4-Difluorophenyl)-5-fluoro-N-{3-[(propylsulfonyl)methyl]phenyl}pyrimidin-2-amine
- N-{3-[(Cyclopropylsulfonyl)methyl]phenyl}-4-(2,4-difluorophenyl)-5-fluoropyrimidin-2-amine
- 4-(2,4-Difluorophenyl)-5-fluoro-N-{3-fluoro-5-[(methylsulfonyl)methyl]phenyl}pyrimidin-2-amine
- 5-Fluoro-N-{3-fluoro-5-[(methylsulfonyl)methyl]phenyl}-4-(2,4,5-trifluorophenyl)pyrimidin-2-amine
- 5-Fluoro-N-{3-fluoro-5-[(methylsulfonyl)methyl]phenyl}-4-(2,3,4-trifluorophenyl)pyrimidin-2-amine
- 4-(2,4-Difluorophenyl)-5-fluoro-N-{3-fluoro-5-[(propylsulfonyl)methyl]phenyl}pyrimidin-2-amine
- 5-Fluoro-N-{3-fluoro-5-[(propylsulfonyl)methyl]phenyl}-4-(2,4,5-trifluorophenyl)pyrimidin-2-amine or their salts, solvates or salts of solvates.

The abovementioned definitions of radicals which have been detailed in general terms or in preferred ranges also apply to the end products of the formula (I) and, analogously, to the starting materials or intermediates required in each case for the preparation.

In another embodiment, the present invention concerns compounds of general formula (5) 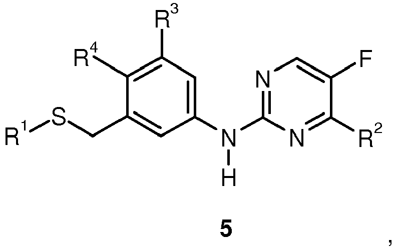 wherein R¹, R², R³ and R⁴ are as defined for the compound of general formula **(I)** according to the invention.

The invention furthermore relates to a method for the preparation of the compounds of formula **(I)** according to the invention, in which method a compound of formula **(5)** 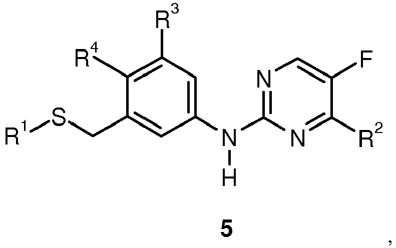 in which R¹, R², R³ and R⁴ are as defined for the compound of general formula **(I),** is oxidized with an alkali salt of permanganic acid in an aliphatic ketone of the formula C₁-C₂-alkyl-C(=O)-C₁-C₂-alkyl as a solvent, thus providing a compound of general formula **(I)** according to the present invention, and in which method the resulting compound of formula **(I)** is optionally, if appropriate, reacted with the corresponding (i) solvents and/or (ii) bases or acids to the solvates, salts and/or solvates of the salts of the compounds of formula **(I).**

The invention furthermore relates to a method for the preparation of the compounds of formula **(5),** in which R¹, R², R³ and R⁴ are as defined for the compound of general formula **(I)** according to the invention, in which method a compound of formula **(3)** 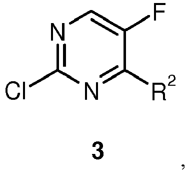 in which R² is as defined for the compound of general formula **(I),** is reacted with a compound of formula **(4)** 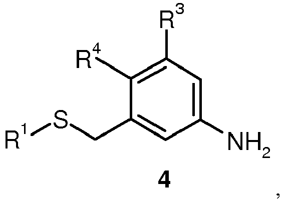 in which R¹, R², R³ and R⁴ are as defined for the compound of general formula **(I),** in the presence of a a strong inorganic or organic acid and in an aliphatic alcohol, an ether, or *N,N*-dimethylformamide as a solvent, or in a mixture of such solvents, thus providing a compound of general formula **(5)** 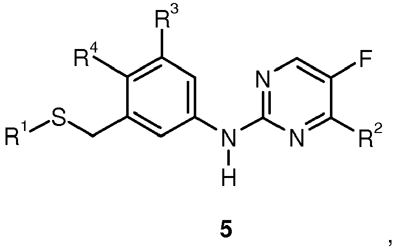 in which R¹, R², R³ and R⁴ are as defined for the compound of general formula **(I).**

The invention furthermore relates to a method for the preparation of the compounds of formula **(3)** in which R² is as defined for the compound of general formula **(I),** in which method 2-4-dichloro-5-fluoro-pyrimidine **(1),** 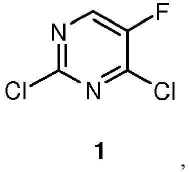 is reacted with a compound of formula **(2)** 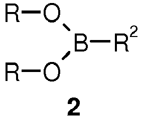 in which R² is as defined for the compound of general formula **(I)** according to the invention, and R represent, independently from each other, a hydrogen atom, or a C₁-C₁₀-alkyl- group or, alternatively, both R together form a R-R group, which is -C(CH₃)₂-C(CH₃)₂-, thus providing a compound of general formula **(3).**

The invention furthermore relates to a method for the preparation of the compounds of formula **(I)** according to the invention, in which method a compound of formula **(3)** 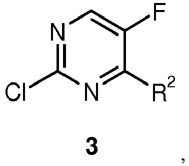 in which R² is as defined for the compound of general formula **(I),**
is reacted with a compound of formula **(6)** 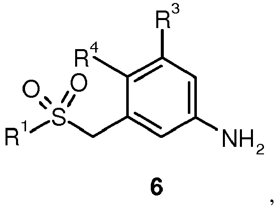 in which R¹, R³ and R⁴ are as defined for the compound of general formula **(I),** in the presence of a strong inorganic or organic acid and in an aliphatic alcohol, an ether, or *N,N*-dimethylformamide as a solvent, or in a mixture of such solvents, thus providing a compound of general formula **(I)** according to the present invention,
and in which method the resulting compound of formula **(I)** is optionally, if appropriate, reacted with the corresponding (i) solvents and/or (ii) bases or acids to the solvates, salts and/or solvates of the salts of the compounds of formula **(I).**

The compounds according to the invention show a valuable pharmacological and pharmacokinetic spectrum of action which could not have been predicted.

They are therefore suitable for use as medicaments for the treatment and/or prophylaxis of disorders in humans and animals.

Within the scope of the present invention, the term "treatment" includes prophylaxis.

The pharmaceutical activity of the compounds according to the invention can be explained by their action as inhibitors of CDK9. Thus, the compounds according to the general formula (I) as well as pharmaceutically acceptable salts thereof are used as inhibitors for CDK9.

Furthermore, the compounds according to the invention show a particularly high potency (demonstrated by a low IC₅₀ value in the CDK9/CycT1 assay) for inhibiting CDK9 activity.

In context of the present invention, the IC₅₀ value with respect to CDK9 can be determined by the methods described in the method section below. Preferably, it is determined according to Method 1a. ("CDK9/CycT1 kinase assay") described in the Materials and Method section below.

Surprisingly it turned out that the compounds according to the general formula **(I)** as well as pharmaceutically acceptable salts thereof selectively inhibit CDK9 in comparison to other cyclin-dependent protein kinases, preferably in comparison to CDK2. Thus, the compounds according to the general formula **(I)** as well as pharmaceutically acceptable salts thereof are preferably used as selective inhibitors for CDK9.

Compounds of the present invention according to general formula **(I)** show a significantly stronger CDK9 than CDK2 inhibition.

In context of the present invention, the IC₅₀ value with respect to CDK2 can be determined by the methods described in the method section below. Preferably, it is determined according to Method 2. ("CDK2/CycE kinase assay") described in the Materials and Method section below.

Further, as compared to the CDK9 inhibitors described in the prior art, preferred compounds of the present invention according to general formula (I) show a surprisingly high potency for inhibiting CDK9 activity at high ATP concentrations, which is demonstrated by their low IC₅₀ value in the CDK9/CycT1 high ATP kinase assay. Thus, these compounds have a lower probability to be competed out of the ATP-binding pocket of CDK9/CycT1 kinase due to the high intracellular ATP concentration (R. Copeland et al., Nature Reviews Drug Discovery 2006, 5, 730-739). According to this property the compounds of the present invention are particularly able to inhibit CDK9/CycT1 within cells for a longer period of time as compared to classical ATP competitive kinase inhibitors. This increases the anti-tumor cell efficacy at pharmacokinetic clearance-mediated declining serum concentrations of the inhibitor after dosing of a patient or an animal.

In context of the present invention, the IC₅₀ value with respect to CDK9 at high ATP concentrations can be determined by the methods described in the method section below. Preferably, it is determined according to Method 1b ("CDK9/CycT1 high ATP kinase assay") as described in the Materials and Method section below.

Further, preferred compounds of the present invention according to formula **(I)** show an improved anti-proliferative activity in tumor cell lines such as HeLa compared to the CDK9 inhibitors described in the prior art. In context of the present invention, the anti-proliferative activity in tumor cell lines such as HeLa is preferably determined according to Method 3. ("Proliferation Assay") as described in the Materials and Method section below.

A further subject matter of the present disclosure is the use of the compounds of general formula **(I)**
according to the invention for the treatment and/or prophylaxis of disorders, preferably of disorders relating to or mediated by CDK9 activity, in particular of hyper-proliferative disorders, virally induced infectious diseases and/or of cardiovascular diseases, more preferably of hyper-proliferative disorders.

The compounds of the present invention may be used to inhibit the activity or expression of CDK9. Therefore, the compounds of formula **(I)** are expected to be valuable as therapeutic agents. Accordingly, in another embodiment, the present disclosure provides a method of treating disorders relating to or
mediated by CDK9 activity in a patient in need of such treatment, comprising administering to the patient an effective amount of a compound of formula **(I)** as defined above. In certain embodiments, the disorders relating to CDK9 activity are hyper-proliferative disorders, virally induced infectious diseases and/or of cardiovascular diseases, more preferably hyper-proliferative disorders, particularly cancer.

The term "treating" or "treatment" as stated throughout this document is used conventionally, *e.g.,* the management or care of a subject for the purpose of combating, alleviating, reducing, relieving, improving the condition of a disease or disorder, such as a carcinoma.

The term "subject" or "patient" includes organisms which are capable of suffering from a cell proliferative disorder or a disorder associated with reduced or insufficient programmed cell death (apoptosis) or who could otherwise benefit from the administration of a compound of the invention, such as human and non-human animals. Preferred humans include human patients suffering from or prone to suffering from a cell proliferative disorder or associated state, as described herein. The term "non-human animals" includes vertebrates, *e.g.,* mammals, such as non-human primates, sheep, cow, dog, cat and rodents, *e.*g., mice, and non-mammals, such as chickens, amphibians, reptiles, etc.

The term "disorders relating to or mediated by CDK9" shall include diseases associated with or implicating CDK9 activity, for example the hyperactivity of CDK9, and conditions that accompany with these diseases. Examples of "disorders relating to or mediated by CDK9" include disorders resulting from increased CDK9 activity due to mutations in genes regulating CDK9 activity such as LARP7, HEXIM1/2 or 7sk snRNA, or disorders resulting from increased CDK9 activity due to activation of the CDK9/cyclinT/RNApolymerase II complex by viral proteins such as HIV-TAT or HTLV-TAX or disorders resulting from increased CDK9 activity due to activation of mitogenic signaling pathways.

The term "hyperactivity of CDK9" refers to increased enzymatic activity of CDK9 as compared to normal non-diseased cells, or it refers to increased CDK9 activity leading to unwanted cell proliferation, or to reduced or insufficient programmed cell death (apoptosis), or mutations leading to constitutive activation of CDK9.

The term "hyper-proliferative disorder" includes disorders involving the undesired or uncontrolled proliferation of a cell and it includes disorders involving reduced or insufficient programmed cell death (apoptosis). The compounds of the present invention can be utilized to prevent, inhibit, block, reduce, decrease, control, etc., cell proliferation and/or cell division, and/or produce apoptosis. This method comprises administering to a subject in need thereof, including a mammal, including a human, an amount of a compound of this invention, or a pharmaceutically acceptable salt, hydrate or solvate thereof which is effective to treat or prevent the disorder.

Hyper-proliferative disorders in the context of this invention include, but are not limited to, *e.g.,* psoriasis, keloids and other hyperplasias affecting the skin, endometriosis, skeletal disorders, angiogenic or blood vessel proliferative disorders, pulmonary hypertension, fibrotic disorders, mesangial cell proliferative disorders, colonic polyps, polycystic kidney disease, benign prostate hyperplasia (BPH), and solid tumors, such as cancers of the breast, respiratory tract, brain, reproductive organs, digestive tract, urinary tract, eye, liver, skin, head and neck, thyroid, parathyroid, and their distant metastases. Those disorders also include lymphomas, sarcomas and leukemias.
Examples of breast cancer include, but are not limited to invasive ductal carcinoma, invasive lobular carcinoma, ductal carcinoma *in situ*, and lobular carcinoma *in situ*, and canine or feline mammary carcinoma.
Examples of cancers of the respiratory tract include, but are not limited to small-cell and non-small-cell lung carcinoma, as well as bronchial adenoma, pleuropulmonary blastoma, and mesothelioma. Examples of brain cancers include, but are not limited to brain stem and hypophtalmic glioma, cerebellar and cerebral astrocytoma, glioblastoma, medulloblastoma, ependymoma, as well as neuroectodermal and pineal tumor.

Tumors of the male reproductive organs include, but are not limited to prostate and testicular cancer. Tumors of the female reproductive organs include, but are not limited to endometrial, cervical, ovarian, vaginal and vulvar cancer, as well as sarcoma of the uterus.
Tumors of the digestive tract include, but are not limited to anal, colon, colorectal, esophageal, gallbladder, gastric, pancreatic, rectal, small-intestine, and salivary gland cancers. Anal gland adenocarcinomas, mast cell tumors.
Tumors of the urinary tract include, but are not limited to bladder, penile, kidney, renal pelvis, ureter, urethral, and hereditary and sporadic papillary renal cancers.
Eye cancers include, but are not limited to intraocular melanoma and retinoblastoma.
Examples of liver cancers include, but are not limited to hepatocellular carcinoma (liver cell carcinomas with or without fibrolamellar variant), cholangiocarcinoma (intrahepatic bile duct carcinoma), and mixed hepatocellular cholangiocarcinoma.
Skin cancers include, but are not limited to squamous cell carcinoma, Kaposi's sarcoma, malignant melanoma, Merkel cell skin cancer, and non-melanoma skin cancer. mast cell tumors.
Head-and-neck cancers include, but are not limited to laryngeal, hypopharyngeal, nasopharyngeal, oropharyngeal cancer, lip and oral cavity cancer, and squamous cell cancer. Oral melanoma. Lymphomas include, but are not limited to AIDS-related lymphoma, non-Hodgkin's lymphoma, cutaneous T-cell lymphoma, Burkitt lymphoma, Hodgkin's disease, and lymphoma of the central nervous system.
Sarcomas include, but are not limited to sarcoma of the soft tissue, osteosarcoma, malignant fibrous histiocytoma, lymphosarcoma, and rhabdomyosarcoma. Malignant histiocytosis, fibrosarcoma, hemangiosarcoma, hemangiopericytoma, leiomyosarcoma.
Leukemias include, but are not limited to acute myeloid leukemia, acute lymphoblastic leukemia, chronic lymphocytic leukemia, chronic myelogenous leukemia, and hairy cell leukemia.
Fibrotic proliferative disorders, *i. e.* the abnormal formation of extracellular matrices, that may be treated with the compounds and methods of the present invention include lung fibrosis, atherosclerosis, restenosis, hepatic cirrhosis, and mesangial cell proliferative disorders, including renal diseases such as glomerulonephritis, diabetic nephropathy, malignant nephrosclerosis, thrombotic microangiopathy syndromes, transplant rejection, and glomerulopathies.
Other conditions in humans or other mammals that may be treated by administering a compound of the present invention include tumor growth, retinopathy, including diabetic retinopathy, ischemic retinalvein occlusion, retinopathy of prematurity and age-related macular degeneration, rheumatoid arthritis, psoriasis, and bullous disorders associated with subepidermal blister formation, including bullous pemphigoid, erythema multiforme and dermatitis herpetiformis.

The compounds of the present invention may also be used to prevent and treat diseases of the airways and the lung, diseases of the gastrointestinal tract as well as diseases of the bladder and bile duct.

The disorders mentioned above have been well characterized in humans, but also exist with a similar etiology in other animals, including mammals, and can be treated by administering pharmaceutical compositions of the present invention.

In a further aspect of the present disclosure, the compounds according to the invention are used in a method for preventing and/or treating infectious diseases, in particular virally induced infectious diseases. The virally induced infectious diseases, including opportunistic diseases, are caused by retroviruses, hepadnaviruses, herpesviruses, flaviviridae, and/or adenoviruses. In a further preferred embodiment of this method, the retroviruses are selected from lentiviruses or oncoretroviruses, wherein the lentivirus is selected from the group comprising: HIV-1, HIV-2, FIV, BIV, SIVs, SHIV, CAEV, VMV or EIAV, preferably HIV-1 or HIV-2 and wherein the oncoretrovirus is selected from the group of: HTLV-I, HTLV-II or BLV. In a further preferred embodiment of this method, the hepadnavirus is selected from HBV, GSHV or WHV, preferably HBV, the herpesivirus is selected from the group comprising: HSV I, HSV II, EBV, VZV, HCMV or HHV 8, preferably HCMV and the flaviviridae is selected from HCV, West nile or Yellow Fever.
The compounds according to general formula (I) are also useful for prophylaxis and/or treatment of cardiovascular diseases such as cardiac hypertrophy, adult congenital heart disease, aneurysm, stable angina, unstable angina, angina pectoris, angioneurotic edema, aortic valve stenosis, aortic aneurysm, arrhythmia, arrhythmogenic right ventricular dysplasia, arteriosclerosis, arteriovenous malformations, atrial fibrillation, Behcet syndrome, bradycardia, cardiac tamponade, cardiomegaly, congestive cardiomyopathy, hypertrophic cardiomyopathy, restrictive cardiomyopathy, cardiovascular disease prevention, carotid stenosis, cerebral hemorrhage, Churg-Strauss syndrome, diabetes, Ebstein's Anomaly, Eisenmenger complex, cholesterol embolism, bacterial endocarditis, fibromuscular dysplasia, congenital heart defects, heart diseases, congestive heart failure, heart valve diseases, heart attack, epidural hematoma, hematoma, subdural, Hippel-Lindau disease, hyperemia, hypertension, pulmonary hypertension, hypertrophic growth, left ventricular hypertrophy, right ventricular hypertrophy, hypoplastic left heart syndrome, hypotension, intermittent claudication, ischemic heart disease, Klippel-Trenaunay-Weber syndrome, lateral medullary syndrome, long QT syndrome mitral valve prolapse, moyamoya disease, mucocutaneous lymph node syndrome, myocardial infarction, myocardial ischemia, myocarditis, pericarditis, peripheral vascular diseases, phlebitis, polyarteritis nodosa, pulmonary atresia, Raynaud disease, restenosis, Sneddon syndrome, stenosis, superior vena cava syndrome, syndrome X, tachycardia, Takayasu's arteritis, hereditary hemorrhagic telangiectasia, telangiectasis, temporal arteritis, tetralogy of fallot, thromboangiitis obliterans, thrombosis, thromboembolism, tricuspid atresia, varicose veins, vascular diseases, vasculitis, vasospasm, ventricular fibrillation, Williams syndrome, peripheral vascular disease, varicose veins and leg ulcers, deep vein thrombosis, Wolff-Parkinson-White syndrome. Preferred are cardiac hypertrophy, adult congenital heart disease, aneurysms, angina, angina pectoris, arrhythmias, cardiovascular disease prevention, cardiomyopathies, congestive heart failure, myocardial infarction, pulmonary hypertension, hypertrophic growth, restenosis, stenosis, thrombosis and arteriosclerosis.

A further subject matter of the present disclosure is the use of the compounds of general formula (I), according to the invention, for the treatment and/or prophylaxis of disorders, in particular of the disorders mentioned above. One aspect of the present disclosure is the use of 4-(2,4-Difluorophenyl)-5-fluoro-N-{3-[(methylsulfonyl)methyl]phenyl}pyrimidin-2-amine for the treatment and/or prophylaxis of disorders.
A further subject matter of the present invention are the compounds according to the invention for use in a method for the treatment and/or prophylaxis of the disorders mentioned above. One aspect of the present invention is 4-(2,4-Difluorophenyl)-5-fluoro-N-{3-[(methylsulfonyl)methyl]phenyl}pyrimidin-2-amine for use in a method for the treatment and/or prophylaxis of the disorders mentioned above.
A preferred subject matter of the present invention are the compounds according to the invention for the use in a method for the treatment and/or prophylaxis of lung carcinomas, especially non-small cell lung carcinomas, prostate carcinomas, especially hormone-independent human prostate carcinomas, cervical carcinomas, including multidrug-resistant human cervical carcinomas, colorectal carcinomas, melanomas or ovarian carcinomas. One aspect of the present invention is 4-(2,4-Difluorophenyl)-5-fluoro-N-{3-[(methylsulfonyl)methyl]phenyl}pyrimidin-2-amine for the use in a method for the treatment and/or prophylaxis of lung carcinomas, especially non-small cell lung carcinomas, prostate carcinomas, especially hormone-independent human prostate carcinomas, cervical carcinomas, including multidrug-resistant human cervical carcinomas, colorectal carcinomas, melanomas or ovarian carcinomas.
A further subject matter of the present invention is the use of the compounds according to the invention in the manufacture of a medicament for the treatment and/or prophylaxis of disorders, in particular the disorders mentioned above. One aspect of the present invention is the use of 4-(2,4-Difluorophenyl)-5-fluoro-N-{3-[(methylsulfonyl)methyl]phenyl}pyrimidin-2-amine in the manufacture of a medicament for the treatment and/or prophylaxis of disorders.
A preferred subject matter of the present invention is the use of the compounds according to the invention in the manufacture of a medicament for the treatment and/or prophylaxis of lung carcinomas, especially non-small cell lung carcinomas, prostate carcinomas, especially hormone-independent human prostate carcinomas, cervical carcinomas, including multidrug-resistant human cervical carcinomas, colorectal carcinomas, melanomas or ovarian carcinomas. One aspect of the present invention is the use of 4-(2,4-Difluorophenyl)-5-fluoro-N-{3-[(methylsulfonyl)methyl]phenyl}pyrimidin-2-amine in the manufacture of a medicament for the treatment and/or prophylaxis of lung carcinomas, especially non-small cell lung carcinomas, prostate carcinomas, especially hormone-independent human prostate carcinomas, cervical carcinomas, including multidrug-resistant human cervical carcinomas, colorectal carcinomas, melanomas or ovarian carcinomas.

A further subject matter of the present disclosure is a method for the treatment and/or prophylaxis of disorders, in particular the disorders mentioned above, using an effective amount of the compounds according to the invention. One aspect of the present disclosure is a method for the treatment and/or prophylaxis of disorders, in particular the disorders mentioned above, using an effective amount of 4-(2,4-Difluorophenyl)-5-fluoro-N-{3-[(methylsulfonyl)methyl]phenyl}pyrimidin-2-amine.
A preferred subject matter of the present disclosure is a method for the treatment and/or prophylaxis of
lung carcinomas, especially non-small cell lung carcinomas, prostate carcinomas, especially hormone-independent human prostate carcinomas, cervical carcinomas, including multidrug-resistant human cervical carcinomas, colorectal carcinomas, melanomas or ovarian carcinomas using an effective amount of the compounds according to the invention. One aspect of the present disclosure is a method for the
treatment and/or prophylaxis of lung carcinomas, especially non-small cell lung carcinomas, prostate carcinomas, especially hormone-independent human prostate carcinomas, cervical carcinomas, including multidrug-resistant human cervical carcinomas, colorectal carcinomas, melanomas or ovarian carcinomas using an effective amount of 4-(2,4-Difluorophenyl)-5-fluoro-N-{3-[(methylsulfonyl)methyl]phenyl}pyrimidin-2-amine.
Another aspect of the present invention relates to pharmaceutical combinations comprising a compound of general formula (I) according to the invention in combination with at least one or more further active ingredients.
As used herein the term "pharmaceutical combination" refers to a combination of at least one compound of general formula (I) according to the invention as active ingredient together with at least one other active ingredient with or without further ingredients, carrier, diluents and/or solvents.
Another aspect of the present invention relates to pharmaceutical compositions comprising a compound of general formula (I) according to the invention in combination with an inert, nontoxic, pharmaceutically suitable adjuvant.
As used herein the term "pharmaceutical composition" refers to a galenic formulation of at least one pharmaceutically active agent together with at least one further ingredient, carrier, diluent and/or solvent.

Another aspect of the present invention relates to the pharmaceutical combinations and/or the pharmaceutical compositions according to the invention for use in the treatment and/or prophylaxis of
disorders, in particular of the disorders mentioned above.
Compounds of formula (I) may be administered as the sole pharmaceutical agent or in combination with one or more additional therapeutic agents where the combination causes no unacceptable adverse effects. This pharmaceutical combination includes administration of a single pharmaceutical dosage formulation which contains a compound of formula (I) and one or more additional therapeutic agents, as well as administration of the compound of formula (I) and each additional therapeutic agent in its own separate pharmaceutical dosage formulation. For example, a compound of formula (I) and a therapeutic agent may be administered to the patient together in a single oral dosage composition such as a tablet or capsule, or each agent may be administered in separate dosage formulations.
Where separate dosage formulations are used, the compound of formula (I) and one or more additional therapeutic agents may be administered at essentially the same time (e.g., concurrently) or at separately staggered times (e.g., sequentially).
In particular, the compounds of the present invention may be used in fixed or separate combination with other anti-tumor agents such as alkylating agents, anti-metabolites, plant-derived anti-tumor agents, hormonal therapy agents, topoisomerase inhibitors, camptothecin derivatives, kinase inhibitors, targeted drugs, antibodies, interferons and/or biological response modifiers, anti-angiogenic compounds, and other anti-tumor drugs. In this regard, the following is a non-limiting list of examples of secondary agents that may be used in combination with the compounds of the present invention:
- Alkylating agents include, but are not limited to, nitrogen mustard N-oxide, cyclophosphamide, ifosfamide, thiotepa, ranimustine, nimustine, temozolomide, altretamine, apaziquone, brostallicin, bendamustine, carmustine, estramustine, fotemustine, glufosfamide, mafosfamide, bendamustin, and mitolactol; platinum-coordinated alkylating compounds include, but are not limited to, cisplatin, carboplatin, eptaplatin, lobaplatin, nedaplatin, oxaliplatin, and satraplatin;
- Anti-metabolites include, but are not limited to, methotrexate, 6-mercaptopurine riboside, mercaptopurine, 5-fluorouracil alone or in combination with leucovorin, tegafur, doxifluridine, carmofur, cytarabine, cytarabine ocfosfate, enocitabine, gemcitabine, fludarabin, 5-azacitidine, capecitabine, cladribine, clofarabine, decitabine, eflornithine, ethynylcytidine, cytosine arabinoside, hydroxyurea, melphalan, nelarabine, nolatrexed, ocfosfite, disodium premetrexed, pentostatin, pelitrexol, raltitrexed, triapine, trimetrexate, vidarabine, vincristine, and vinorelbine;
- Hormonal therapy agents include, but are not limited to, exemestane, Lupron, anastrozole, doxercalciferol, fadrozole, formestane, 11-beta hydroxysteroid dehydrogenase 1 inhibitors, 17-alpha hydroxylase/17,20 lyase inhibitors such as abiraterone acetate, 5-alpha reductase inhibitors such as finasteride and epristeride, anti-estrogens such as tamoxifen citrate and fulvestrant, Trelstar,toremifene, raloxifene, lasofoxifene, letrozole, anti-androgens such as bicalutamide, flutamide, mifepristone, nilutamide, Casodex, and anti-progesterones and combinations thereof;
- Plant-derived anti-tumor substances include, *e.g*., those selected from mitotic inhibitors, for example epothilones such as sagopilone, ixabepilone and epothilone B, vinblastine, vinflunine, docetaxel, and paclitaxel;
- Cytotoxic topoisomerase inhibiting agents include, but are not limited to, aclarubicin, doxorubicin, amonafide, belotecan, camptothecin, 10-hydroxycamptothecin, 9-aminocamptothecin, diflomotecan, irinotecan, topotecan, edotecarin, epimbicin, etoposide, exatecan, gimatecan, lurtotecan, mitoxantrone, pirambicin, pixantrone, rubitecan, sobuzoxane, tafluposide, and combinations thereof;
- Immunologicals include interferons such as interferon alpha, interferon alpha-2a, interferon alpha-2b, interferon beta, interferon gamma-1a and interferon gamma-n1, and other immune enhancing agents such as L19-IL2 and other IL2 derivatives, filgrastim, lentinan, sizofilan, TheraCys, ubenimex, aldesleukin, alemtuzumab, BAM-002, dacarbazine, daclizumab, denileukin, gemtuzumab, ozogamicin, ibritumomab, imiquimod, lenograstim, lentinan, melanoma vaccine (Corixa), molgramostim, sargramostim, tasonermin, tecleukin, thymalasin, tositumomab, Vimlizin, epratuzumab, mitumomab, oregovomab, pemtumomab, and Provenge; Merial melanoma vaccine
- Biological response modifiers are agents that modify defense mechanisms of living organisms or biological responses such as survival, growth or differentiation of tissue cells to direct them to have anti-tumor activity; such agents include, *e.g*., krestin, lentinan, sizofiran, picibanil, ProMune, and ubenimex;
- Anti-angiogenic compounds include, but are not limited to, acitretin, aflibercept, angiostatin, aplidine, asentar, axitinib, recentin, bevacizumab, brivanib alaninat, cilengtide, combretastatin, DAST, endostatin, fenretinide, halofuginone, pazopanib, ranibizumab, rebimastat, removab, revlimid, sorafenib, vatalanib, squalamine, sunitinib, telatinib, thalidomide, ukrain, and vitaxin;
- Antibodies include, but are not limited to, trastuzumab, cetuximab, bevacizumab, rituximab, ticilimumab, ipilimumab, lumiliximab, catumaxomab, atacicept, oregovomab, and alemtuzumab;
- VEGF inhibitors such as, *e.g.*, sorafenib, DAST, bevacizumab, sunitinib, recentin, axitinib, aflibercept, telatinib, brivanib alaninate, vatalanib, pazopanib, and ranibizumab; Palladia
- EGFR (HER1) inhibitors such as, *e.g.*, cetuximab, panitumumab, vectibix, gefitinib, erlotinib, and Zactima;
- HER2 inhibitors such as, *e.g*., lapatinib, tratuzumab, and pertuzumab;
- mTOR inhibitors such as, *e.g*., temsirolimus, sirolimus/Rapamycin, and everolimus;
- c-Met inhibitors;
- PI3K and AKT inhibitors;
- CDK inhibitors such as roscovitine and flavopiridol;
- Spindle assembly checkpoints inhibitors and targeted anti-mitotic agents such as PLK inhibitors, Aurora inhibitors (*e.g*. Hesperadin), checkpoint kinase inhibitors, and KSP inhibitors;
- HDAC inhibitors such as, *e.g*., panobinostat, vorinostat, MS275, belinostat, and LBH589;
- HSP90 and HSP70 inhibitors;
- Proteasome inhibitors such as bortezomib and carfilzomib;
- Serine/threonine kinase inhibitors including MEK inhibitors (such as e.g. RDEA 119) and Raf inhibitors such as sorafenib;
- Farnesyl transferase inhibitors such as, *e.g.*, tipifarnib;
- Tyrosine kinase inhibitors including, *e.g.*, dasatinib, nilotibib, DAST, bosutinib, sorafenib, bevacizumab, sunitinib, AZD2171, axitinib, aflibercept, telatinib, imatinib mesylate, brivanib alaninate, pazopanib, ranibizumab, vatalanib, cetuximab, panitumumab, vectibix, gefitinib, erlotinib, lapatinib, tratuzumab, pertuzumab, and c-Kit inhibitors; Palladia, masitinib
- Vitamin D receptor agonists;
- Bcl-2 protein inhibitors such as obatoclax, oblimersen sodium, and gossypol;
- Cluster of differentiation 20 receptor antagonists such as, *e.g*., rituximab;
- Ribonucleotide reductase inhibitors such as, *e.g*., gemcitabine;
- Tumor necrosis apoptosis inducing ligand receptor 1 agonists such as, *e**.**g*., mapatumumab;
- 5-Hydroxytryptamine receptor antagonists such as, *e.g.*, rEV598, xaliprode, palonosetron hydrochloride, granisetron, Zindol, and AB-1001;
- Integrin inhibitors including alpha5-beta1 integrin inhibitors such as, *e.g.,* E7820, JSM 6425, volociximab, and endostatin;
- Androgen receptor antagonists including, *e.g.*, nandrolone decanoate, fluoxymesterone, Android, Prost-aid, andromustine, bicalutamide, flutamide, apo-cyproterone, apo-flutamide, chlormadinone acetate, Androcur, Tabi, cyproterone acetate, and nilutamide;
- Aromatase inhibitors such as, *e.g.,* anastrozole, letrozole, testolactone, exemestane, amino-glutethimide, and formestane;
- Matrix metalloproteinase inhibitors;
- Other anti-cancer agents including, *e.g.*, alitretinoin, ampligen, atrasentan bexarotene, bortezomib, bosentan, calcitriol, exisulind, fotemustine, ibandronic acid, miltefosine, mitoxantrone, I-asparaginase, procarbazine, dacarbazine, hydroxycarbamide, pegaspargase, pentostatin, tazaroten, velcade, gallium nitrate, canfosfamide, darinaparsin, and tretinoin.

The compounds of the present invention may also be employed in cancer treatment in conjunction with radiation therapy and/or surgical intervention.

Generally, the use of cytotoxic and/or cytostatic agents in combination with a compound or composition of the present invention will serve to:
(1) yield better efficacy in reducing the growth of a tumor or even eliminate the tumor as compared to administration of either agent alone,
(2) provide for the administration of lesser amounts of the administered chemotherapeutic agents,
(3) provide for a chemotherapeutic treatment that is well tolerated in the patient with fewer deleterious pharmacological complications than observed with single agent chemotherapies and certain other combined therapies,
(4) provide for treating a broader spectrum of different cancer types in mammals, especially humans,
(5) provide for a higher response rate among treated patients,
(6) provide for a longer survival time among treated patients compared to standard chemotherapy treatments,
(7) provide a longer time for tumor progression, and/or
(8) yield efficacy and tolerability results at least as good as those of the agents used alone, compared to known instances where other cancer agent combinations produce antagonistic effects.

Furthermore, the compounds of formula (I) may be utilized, as such or in compositions, in research and diagnostics, or as analytical reference standards, and the like, which are well known in the art.

The compounds according to the invention can act systemically and/or locally. For this purpose, they can be administered in a suitable way, such as, for example, by the oral, parenteral, pulmonal, nasal, sublingual, lingual, buccal, rectal, dermal, transdermal, conjunctival or otic route, or as an implant or stent.
For these administration routes, it is possible to administer the compounds according to the invention in suitable application forms.

Suitable for oral administration are administration forms which work as described in the prior art and deliver the compounds according to the invention rapidly and/or in modified form, which comprise the compounds according to the invention in crystalline and/or amorphous and/or dissolved form, such as, for example, tablets (coated or uncoated, for example tablets provided with enteric coatings or coatings whose dissolution is delayed or which are insoluble and which control the release of the compound according to the invention), tablets which rapidly decompose in the oral cavity, or films/wafers, films/lyophilizates, capsules (for example hard or soft gelatin capsules), sugar-coated tablets, granules, pellets, powders, emulsions, suspensions, aerosols or solutions.

Parenteral administration can take place with avoidance of an absorption step (for example intravenously, intraarterially, intracardially, intraspinally or intralumbally) or with inclusion of absorption (for example intramuscularly, subcutaneously, intracutaneously, percutaneously or intraperitoneally). Administration forms suitable for parenteral administration are, inter alia, preparations for injection and infusion in the form of solutions, suspensions, emulsions, lyophilizates or sterile powders.

Examples suitable for the other administration routes are pharmaceutical forms for inhalation (inter alia powder inhalers, nebulizers), nasal drops/solutions/sprays; tablets to be administered lingually, sublingually or buccally, films/wafers or capsules, suppositories, preparations for the eyes or ears, vaginal capsules, aqueous suspensions (lotions, shaking mixtures), lipophilic suspensions, ointments, creams, transdermal therapeutic systems (such as plasters, for example), milk, pastes, foams, dusting powders, implants or stents.

The compounds according to the invention can be converted into the stated administration forms. This can take place in a manner known per se by mixing with inert, nontoxic, pharmaceutically suitable adjuvants. These adjuvants include, inter alia, carriers (for example microcrystalline cellulose, lactose, mannitol), solvents (for example liquid polyethylene glycols), emulsifiers and dispersants or wetting agents (for example sodium dodecyl sulphate, polyoxysorbitan oleate), binders (for example polyvinylpyrrolidone), synthetic and natural polymers (for example albumin), stabilizers (for example antioxidants, such as, for example, ascorbic acid), colorants (for example inorganic pigments, such as, for example, iron oxides) and flavour- and/or odour-masking agents.

The present invention furthermore provides medicaments comprising at least one compound according to the invention, usually together with one or more inert, nontoxic, pharmaceutically suitable adjuvants, and their use for the purposes mentioned above.
When the compounds of the present invention are administered as pharmaceuticals, to humans or animals, they can be given per se or as a pharmaceutical composition containing, for example, 0.1% to 99,5% (more preferably 0.5% to 90%) of active ingredient in combination with one or more inert, nontoxic, pharmaceutically suitable adjuvants.

Regardless of the route of administration selected, the compounds of the invention of general formula (I) and/or the pharmaceutical composition of the present invention are formulated into pharmaceutically acceptable dosage forms by conventional methods known to those of skill in the art.
Actual dosage levels and time course of administration of the active ingredients in the pharmaceutical compositions of the invention may be varied so as to obtain an amount of the active ingredient which is effective to achieve the desired therapeutic response for a particular patient without being toxic to the patient.

### Materials and Methods:

The percentage data in the following tests and examples are percentages by weight unless otherwise indicated; parts are parts by weight. Solvent ratios, dilution ratios and concentration data of liquid/liquid solutions are in each case based on volume.

Examples were tested in selected biological assays one or more times. When tested more than once, data are reported as either average values or as median values, wherein
- the average value, also referred to as the arithmetic mean value, represents the sum of the values obtained divided by the number of times tested, and
- the median value represents the middle number of the group of values when ranked in ascending or descending order. If the number of values in the data set is odd, the median is the middle value. If the number of values in the data set is even, the median is the arithmetic mean of the two middle values.

Examples were synthesized one or more times. When synthesized more than once, data from biological assays represent average values or median values calculated utilizing data sets obtained from testing of one or more synthetic batch.

The *in vitro* pharmacological properties of the compounds can be determined according to the following assays and methods.

### 1a. CDK9/CycT1 kinase assay:

CDK9/CycT1 -inhibitory activity of compounds of the present invention was quantified employing the CDK9/CycT1 TR-FRET assay as described in the following paragraphs:
Recombinant full-length His-tagged human CDK9 and CycT1, expressed in insect cells and purified by Ni-NTA affinity chromatography, were purchased from Invitrogen (Cat. No PV4131). As substrate for the kinase reaction biotinylated peptide biotin-Ttds-YISPLKSPYKISEG (C-terminus in amid form) was used which can be purchased e.g. form the company JERINI Peptide Technologies (Berlin, Germany). For the assay 50 nl of a 100fold concentrated solution of the test compound in DMSO was pipetted into a black low volume 384well microtiter plate (Greiner Bio-One, Frickenhausen, Germany), 2 µl of a solution of CDK9/CycT1 in aqueous assay buffer [50 mM Tris/HCl pH 8.0, 10 mM MgCl₂, 1.0 mM dithiothreitol, 0.1 mM sodium ortho-vanadate, 0.01% (v/v) Nonidet-P40 (Sigma)] were added and the mixture was incubated for 15 min at 22°C to allow pre-binding of the test compounds to the enzyme before the start of the kinase reaction. Then the kinase reaction was started by the addition of 3 µl of a solution of adenosine-tri-phosphate (ATP, 16.7 µM => final conc. in the 5 µl assay volume is 10 µM) and substrate (1.67 µM => final conc. in the 5 µl assay volume is 1 µM) in assay buffer and the resulting mixture was incubated for a reaction time of 25 min at 22°C. The concentration of CDK9/CycT1 was adjusted depending of the activity of the enzyme lot and was chosen appropriate to have the assay in the linear range, typical concentrations were in the range of 1 µg/ml. The reaction was stopped by the addition of 5 µl of a solution of TR-FRET detection reagents (0.2 µM streptavidine-XL665 [Cisbio Bioassays, Codolet, France] and 1 nM anti-RB(pSer807/pSer811)-antibody from BD Pharmingen [# 558389] and 1.2 nM LANCE EU-W1024 labeled anti-mouse IgG antibody [Perkin-Elmer, product no. AD0077]) in an aqueous EDTA-solution (100 mM EDTA, 0.2 % (w/v) bovine serum albumin in 100 mM HEPES/NaOH pH 7.0).

The resulting mixture was incubated 1 h at 22°C to allow the formation of complex between the phosphorylated biotinylated peptide and the detection reagents. Subsequently the amount of phosphorylated substrate was evaluated by measurement of the resonance energy transfer from the Euchelate to the streptavidine-XL. Therefore, the fluorescence emissions at 620 nm and 665 nm after excitation at 350 nm was measured in a HTRF reader, e.g. a Rubystar (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition). Usually the test compounds were tested on the same microtiterplate in 11 different concentrations in the range of 20 µM to 0.1 nM (20 µM, 5.9 µM, 1.7 µM, 0.51 µM, 0.15 µM, 44 nM, 13 nM, 3.8 nM, 1.1 nM, 0.33 nM and 0.1 nM, the dilution series prepared separately before the assay on the level of the 100fold concentrated solutions in DMSO by serial 1:3.4 dilutions) in duplicate values for each concentration and IC₅₀ values were calculated by a 4 parameter fit using an inhouse software.

### 1b. CDK9/CycT1 high ATP kinase assay

CDK9/CycT1 -inhibitory activity of compounds of the present invention at a high ATP concentration after preincubation of enzyme and test compounds was quantified employing the CDK9/CycT1 TR-FRET assay as described in the following paragraphs.

Recombinant full-length His-tagged human CDK9 and CycT1, expressed in insect cells and purified by Ni-NTA affinity chromatography, were purchase from Invitrogen (Cat. No PV4131). As substrate for the kinase reaction biotinylated peptide biotin-Ttds-YISPLKSPYKISEG (C-terminus in amid form) was used which can be purchased e.g. form the company JERINI peptide technologies (Berlin, Germany). For the assay 50 nl of a 100fold concentrated solution of the test compound in DMSO was pipetted into a black low volume 384well microtiter plate (Greiner Bio-One, Frickenhausen, Germany), 2 µl of a solution of CDK9/CycT1 in aqueous assay buffer [50 mM Tris/HCl pH 8.0, 10 mM MgCl₂, 1.0 mM dithiothreitol, 0.1 mM sodium ortho-vanadate, 0.01% (v/v) Nonidet-P40 (Sigma)] were added and thetmixture was incubated for 15 min at 22°C to allow pre-binding of the test compounds to the enzyme before the start of the kinase reaction. Then the kinase reaction was started by the addition of 3 µl of a solution of adenosine-tri-phosphate (ATP, 3.3 mM => final conc. in the 5 µl assay volume is 2 mM) and substrate (1.67 µM => final conc. in the 5 µl assay volume is 1 µM) in assay buffer and the resulting mixture was incubated for a reaction time of 25 min at 22°C. The concentration of CDK9/CycT1 was adjusted depending of the activity of the enzyme lot and was chosen appropriate to have the assay in the linear range, typical concentrations were in the range of 0.5 µg/ml. The reaction was stopped by the addition of 5 µl of a solution of TR-FRET detection reagents (0.2 µM streptavidine-XL665 [Cisbio Bioassays, Codolet, France] and 1 nM anti-RB(pSer807/pSer811)-antibody from BD Pharmingen [# 558389] and 1.2 nM LANCE EU-W1024 labeled anti-mouse IgG antibody [Perkin-Elmer, product no. AD0077]) in an aqueous EDTA-solution (100 mM EDTA, 0.2 % (w/v) bovine serum albumin in 100 mM HEPES/NaOH pH 7.0).

The resulting mixture was incubated 1 h at 22°C to allow the formation of complex between the phosphorylated biotinylated peptide and the detection reagents. Subsequently the amount of phosphorylated substrate was evaluated by measurement of the resonance energy transfer from the Euchelate to the streptavidine-XL. Therefore, the fluorescence emissions at 620 nm and 665 nm after excitation at 350 nm was measured in a HTRF reader, e.g. a Rubystar (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition). Usually the test compounds were tested on the same microtiterplate in 11 different concentrations in the range of 20 µM to 0.1 nM (20 µM, 5.9 µM, 1.7 µM, 0.51 µM, 0.15 µM, 44 nM, 13 nM, 3.8 nM, 1.1 nM, 0.33 nM and 0.1 nM, the dilution series prepared separately before the assay on the level of the 100fold concentrated solutions in DMSO by serial 1:3.4 dilutions) in duplicate values for each concentration and IC₅₀ values were calculated by a 4 parameter fit using an inhouse software.

### 2. CDK2/CycE kinase assay:

CDK2/CycE -inhibitory activity of compounds of the present invention was quantified employing the CDK2/CycE TR-FRET assay as described in the following paragraphs:
Recombinant fusion proteins of GST and human CDK2 and of GST and human CycE, expressed in insect cells (Sf9) and purified by Glutathion-Sepharose affinity chromatography, were purchased from ProQinase GmbH (Freiburg, Germany). As substrate for the kinase reaction biotinylated peptide biotin-Ttds-YISPLKSPYKISEG (C-terminus in amid form) was used which can be purchased e.g. form the company JERINI Peptide Technologies (Berlin, Germany).

For the assay 50 nl of a 100fold concentrated solution of the test compound in DMSO was pipetted into a black low volume 384well microtiter plate (Greiner Bio-One, Frickenhausen, Germany), 2 µl of a solution of CDK2/CycE in aqueous assay buffer [50 mM Tris/HCl pH 8.0, 10 mM MgCl₂, 1.0 mM dithiothreitol, 0.1 mM sodium ortho-vanadate, 0.01% (v/v) Nonidet-P40 (Sigma)] were added and the mixture was incubated for 15 min at 22°C to allow pre-binding of the test compounds to the enzyme before the start of the kinase reaction. Then the kinase reaction was started by the addition of 3 µl of a solution of adenosine-tri-phosphate (ATP, 16.7 µM => final conc. in the 5 µl assay volume is 10 µM) and substrate (1.25 µM => final conc. in the 5 µl assay volume is 0.75 µM) in assay buffer and the resulting mixture was incubated for a reaction time of 25 min at 22°C. The concentration of CDK2/CycE was adjusted depending of the activity of the enzyme lot and was chosen appropriate to have the assay in the linear range, typical concentrations were in the range of 130 ng/ml. The reaction was stopped by the addition of 5 µl of a solution of TR-FRET detection reagents (0.2 µM streptavidine-XL665 [Cisbio Bioassays, Codolet, France] and 1 nM anti-RB(pSer807/pSer811)-antibody from BD Pharmingen [# 558389] and 1.2 nM LANCE EU-W1024 labeled anti-mouse IgG antibody [Perkin-Elmer, product no. AD0077]) in an aqueous EDTA-solution (100 mM EDTA, 0.2 % (w/v) bovine serum albumin in 100 mM HEPES/NaOH pH 7.0).

The resulting mixture was incubated 1 h at 22°C to allow the formation of complex between the phosphorylated biotinylated peptide and the detection reagents. Subsequently the amount of phosphorylated substrate was evaluated by measurement of the resonance energy transfer from the Euchelate to the streptavidine-XL. Therefore, the fluorescence emissions at 620 nm and 665 nm after excitation at 350 nm was measured in a TR-FRET reader, e.g. a Rubystar (BMG Labtechnologies, Offenburg, Germany) or a Viewlux (Perkin-Elmer). The ratio of the emissions at 665 nm and at 622 nm was taken as the measure for the amount of phosphorylated substrate. The data were normalised (enzyme reaction without inhibitor = 0 % inhibition, all other assay components but no enzyme = 100 % inhibition). Usually the test compounds were tested on the same microtiterplate in 11 different concentrations in the range of 20 µM to 0.1 nM (20 µM, 5.9 µM, 1.7 µM, 0.51 µM, 0.15 µM, 44 nM, 13 nM, 3.8 nM, 1.1 nM, 0.33 nM and 0.1 nM, the dilution series prepared separately before the assay on the level of the 100fold concentrated solutions in DMSO by serial 1:3.4 dilutions) in duplicate values for each concentration and IC₅₀ values were calculated by a 4 parameter fit using an inhouse software.

### 3. Proliferation Assay:

Cultivated tumour cells (HeLa, human cervical tumour cells, ATCC CCL-2; NCI-H460, human non-small cell lung carcinoma cells, ATCC HTB-177; A2780, human ovarian carcinoma cells, ECACC # 93112519; DU 145, hormone-independent human prostate carcinoma cells, ATCC HTB-81; HeLa-MaTu-ADR, multidrug-resistant human cervical carcinoma cells, EPO-GmbH Berlin; Caco-2, human colorectal carcinoma cells, ATCC HTB-37; B16F10, mouse melanoma cells, ATCC CRL-6475) were plated at a density of 5,000 cells/well (DU145, HeLa-MaTu-ADR), 3,000 cells/well (NCI-H460, HeLa), 2,500 cells/well (A2780), 1,500 cells/well (Caco-2), or 1,000 cells/well (B16F10) in a 96-well multititer plate in 200 µL of their respective growth medium supplemented 10% fetal calf serum. After 24 hours, the cells of one plate (zero-point plate) were stained with crystal violet (see below), while the medium of the other plates was replaced by fresh culture medium (200 µl), to which the test substances were added in various concentrations (0 µM, as well as in the range of 0.001-10 µM; the final concentration of the solvent dimethyl sulfoxide was 0.5%). The cells were incubated for 4 days in the presence of test substances. Cell proliferation was determined by staining the cells with crystal violet: the cells were fixed by adding 20 µl/measuring point of an 11% glutaric aldehyde solution for 15 minutes at room temperature. After three washing cycles of the fixed cells with water, the plates were dried at room temperature. The cells were stained by adding 100 µl/measuring point of a 0.1% crystal violet solution (pH 3.0). After three washing cycles of the stained cells with water, the plates were dried at room temperature. The dye was dissolved by adding 100 µl/measuring point of a 10% acetic acid solution. The extinction was determined by photometry at a wavelength of 595 nm. The change of cell number, in percent, was calculated by normalization of the measured values to the extinction values of the zero-point plate (=0%) and the extinction of the untreated (0 µm) cells (=100%). The IC₅₀ values (inhibitory concentration at 50% of maximal effect) were determined by means of a 4 parameter fit.

### 4. Carbonic anhydrase Assay

The principle of the assay is based on the hydrolysis of 4-nitrophenyl acetate by carbonic anhydrases (Pocker & Stone, Biochemistry, 1967, 6, 668), with subsequent photometric determination of the dye product 4-nitrophenolate at 400 nm by means of a 96-channel spectral photometer.

2 µL of the test compounds, dissolved in DMSO (100-fold final concentration), in a concentration range of 0.03-10 µmol/L (final), was pipetted as quadruplicates into the wells of a 96-hole microtiter plate. Wells that contained the solvent without test compounds were used as reference values (1. Wells without carbonic anhydrase for correction of the non-enzymatic hydrolysis of the substrate, and 2. Wells with carbonic anhydrase for determining the activity of the non-inhibited enzyme).

188 µL of assay buffer (10 mmol/L of Tris/HCl, pH 7.4, 80 mmol/L of NaCl), with or without 3 units/well of carbonic anhydrase-1 [= human carbonic anhydrase-1 (Sigma, #C4396)] in order to determine carbonic anhydrase-1 inhibition or 3 units/well of carbonic anhydrase-2 [= human carbonic anhydrase-2 (Sigma, #C6165)] for measuring carbonic anhydrase-2 inhibition, was pipetted into the wells of the microtiter plate. The enzymatic reaction was started by the addition of 10 microL of the substrate solution (1 mmol/L of 4-nitrophenyl acetate (Fluka #4602), dissolved in anhydrous acetonitrile (final substrate concentration: 50 µmol/L). The plate was incubated at room temperature for 15 minutes. Absorption was measured by photometry at a wavelength of 400 nm. The enzyme inhibition was calculated after the measured values were normalized to the absorption of the reactions in the wellswithout enzyme (=100% inhibition) and to the absorption of reactions in the wells with non-inhibited enzyme (=0% inhibition). IC₅₀ values were determined by means of a 4 parameter fit using the company's own software.

### Preparative Examples

### Syntheses of compounds

The syntheses of the compounds of general formula (I) according to the present invention are preferably carried out according to the general synthetic sequences, shown in scheme 1 below:

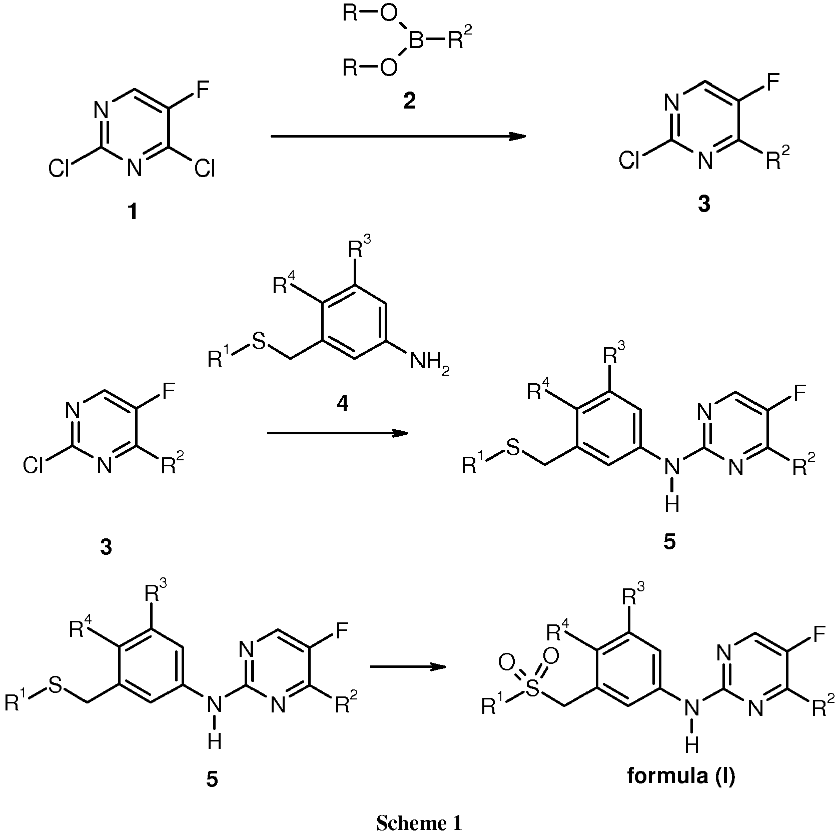

In the first step, 2,4-dichloro-5-fluoropyrimidine (CAS No.: 2927-71-1; 1) is reacted with a boronic acid derivative of formula (2), wherein R² is as defined for the compound of general formula (I), to give a compound of formula (3). The boronic acid derivative (2) may be a boronic acid (R = -H) or an ester of the boronic acid, e.g. its isopropyl ester (R = -CH(CH₃)₂), preferably an ester derived from pinacol in which the boronic acid intermediate forms a 2-aryl-4,4,5,5-tetramethyl-1,3,2-dioxaborolane (R-R = - C(CH₃)₂-C(CH₃)₂-).

Compounds of general formula **(2)** can be prepared analogously to known processes (review: D.G. Hall, Boronic Acids, 2005 WILEY-VCH Verlag GmbH & Co. KGaA, Weinheim, ISBN 3-527-30991-8 and references cited herein). Further, a wide variety of compounds of general formula **(2)** is commercially available.

The coupling reaction of 2-4-dichloro-5-fluoro-pyrimidine **(1)** with compounds of formula **(2)** is catalyzed by Pd catalysts, e.g. by Pd(0) catalysts or by Pd(II) catalysts. Examples for Pd(0) catalysts are tetrakis(triphenylphosphine)palladium(0) [Pd(PPh₃)₄] or tris(dibenzylideneacetone)di-palladium(0) [Pd₂(dba)₃], examples for Pd(II) catalysts dichlorobis(triphenylphosphine)-palladium(II) [Pd(PPh₃)₂Cl₂], palladium(II) acetate and triphenylphosphine or [1,1'-bis(diphenylphosphino)ferrocene]palladium dichloride (review: D.G. Hall, Boronic Acids, 2005 WILEY-VCH Verlag GmbH & Co. KGaA, Weinheim, ISBN 3-527-30991-8 and references cited therein).

This reaction is preferably carried out in aprotic or protic solvents, preferably in a mixture of aprotic and protic solvents, more preferably in solvents like, for example, 1,2-dimethoxyethane, dioxane, dimethylformamide, tetrahydrofuran, or isopropanol with water (review: D.G. Hall, Boronic Acids, 2005 WILEY-VCH Verlag GmbH & Co. KGaA, Weinheim, ISBN 3-527-30991-8 and references cited therein).
Preferably the reaction is carried out in the presence of a suitable base, such as for example aqueous potassium carbonate, aqueous sodium bicarbonate or potassium phosphate (review: D.G. Hall, Boronic Acids, 2005 WILEY-VCH Verlag GmbH & Co. KGaA, Weinheim, ISBN 3-527-30991-8 and references cited therein).
The reaction is performed at temperatures ranging from room temperature (=20°C) to the boiling point of the solvent. Further on, the reaction can be performed at temperatures above the boiling point using pressure tubes and a microwave oven. (review: D.G. Hall, Boronic Acids, 2005 WILEY-VCH Verlag GmbH & Co. KGaA, Weinheim, ISBN 3-527-30991-8 and references cited therein).The reaction is preferably completed after 1 to 36 hours of reaction time.

In the second step, a compound of formula **(3),** in which R² is as defined for the compound of general formula **(I),** is reacted with a suitable aniline of formula **(4),** in which R¹, R³ and R⁴ are as defined for the compound of general formula **(I),** to give a compound of formula **(5).**
This coupling reaction can be carried out in an aliphatic alcohol like 1-butanol or in an inert solvent like DMF, ethers such as THF, DME, dioxane, or in a mixture of such solvents, in the presence of a strong inorganic or organic acid like hydrogen chloride or 4-methylbenzenesulfonic acid. Preferably, the reaction is carried out at elevated temperatures, for example 140°C.

Alternatively, this coupling reaction can be carried out by a Palladium-catalyzed C-N cross-coupling reactions (for a review on C-N cross coupling reactions see for example: a) L. Jiang, S.L. Buchwald in 'Metal-Catalyzed Cross-Coupling Reactions', 2nd ed.: A. de Meijere, F. Diederich, Eds.: Wiley-VCH: Weinheim, Germany, 2004**).**
Anilines of formula **(4)** are commercially available in certain cases, or can be prepared by methods known to the person skilled in the art, e.g from the corresponding *meta*-hydroxymethyl aniline of formula **(7)** *visa* conversion of the hydroxy group contained therein into a suitable leaving group, such as chloro or bromo, followed by nucleophilic displacement with a thiol. If needed, the amino group present in said 4-hydroxymethylpyridine-2-amine can be protected by a suitable protecting group. Protecting groups for amino groups present in analogues and methods for their introduction and removal are well known to the person skilled in the art, see *e.g.* T.W. Greene and P.G.M. Wuts in: Protective Groups in Organic Synthesis, 3rd edition, Wiley (1999). Likewise, a nitro group can serve as inert precursor of an amino group, and can be converted into an amino group by reduction using methods known to the person skilled in the art, such as catalytic hydrogenolysis or reduction with titanium(III)chloride.

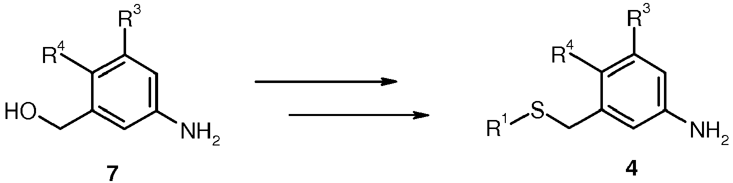

In the third step, a compound of formula **(5),** in which R¹, R², R³ and R⁴ are as defined for the compound of general formula **(I),** is oxidized to the corresponding sulfone of formula (I) with an alkali salt of permanganic acid in an aliphatic ketone of the formula C₁-C₂-alkyl-C(=O)-C₁-C₂-alkyl as a solvent. Preferred is the herein described use of potassium permanganate in acetone. The reactions are preferably run at 40-70 °C.

An alternative synthesis approach to the compounds of general formula (I) according to the present invention is shown in scheme 2 below.

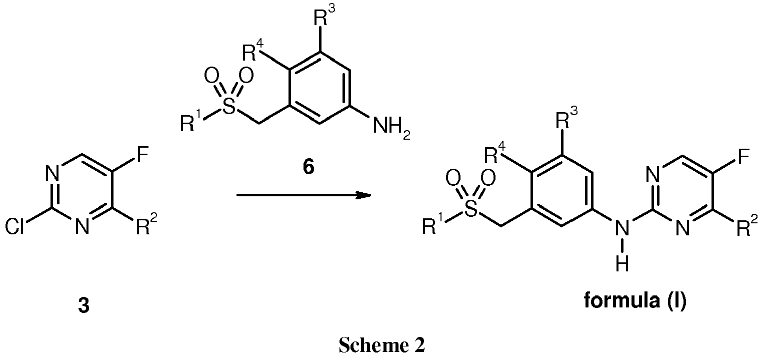

A compound of formula **(3),** in which R² is as defined for the compound of general formula **(I),** is reacted with a suitable aniline of formula **(6),** in which R¹, R³ and R⁴ are as defined for the compound of general formula **(I),** to give a compound of formula **(I).**
This coupling reaction can be carried out in an aliphatic alcohol like 1-butanol or in an inert solvent like DMF, ethers such as THF, DME, dioxane, or in a mixture of such solvents, in the presence of a strong organic or inorganic acid like hydrogen chloride or 4-methylbenzenesulfonic acid. Preferably, the reaction is carried out at elevated temperatures, for example 140°C.
Alternatively, this coupling reaction can be carried out by a Palladium-catalyzed C-N cross-coupling reactions (for a review on C-N cross coupling reactions see for example: a) L. Jiang, S.L. Buchwald in ʹMetal-Catalyzed Cross-Coupling Reactionsʹ, 2nd ed.: A. de Meijere, F. Diederich, Eds.: Wiley-VCH: Weinheim, Germany, 2004**).**
Anilines of formula **(6)** are commercially available in certain cases, or can be prepared by methods known to the person skilled in the art, e.g from the corresponding anilines of formula **(4)** by oxidation of the thioether group to the corresponding sulfone using reagents well known to the person skilled in the art, such as *m*-chloroperbenzoic acid (see e.g. Teall et al, Bioorg. Med. Chem. Lett. 2005, 15, 2685). If needed, the amino group present in said anilines of formula **(4)** can be protected by a suitable protecting group. Protecting groups for amino groups present in analogues and methods for their introduction and removal are well known to the person skilled in the art, see *e.g.* T.W. Greene and P.G.M. Wuts in: Protective Groups in Organic Synthesis, 3rd edition, Wiley (1999). Likewise, a nitro group can serve as inert precursor of an amino group, and can be converted into an amino group by reduction using methods known to the person skilled in the art, such as catalytic hydrogenation or reduction with titanium(III)chloride.

As shown in Scheme 3, anilines of formula **(6)** can furthermore be synthesised starting from nitrobenzene derivatives of the formula **(8),** in which LG represents a leaving group as defined herein, preferably chloro or bromo, which are reacted with sulfinate salts of the formula **(9),** in which R¹ is as defined for the compound of general formula **(I)** and in which M⁺ stands for a cation of an alkali metal, such as sodium, potassium or cesium (see *e.g.* a) Lewis et al, Tetrahedron 2011, 67, 7517; b) Lucking et al, WO 2012/143399; c) Lucking et al, WO 2013/37896), to give sulfones of the formula **(10),** which can be reduced to the corresponding anilines of the formula **(6)** by reduction methods known to the person skilled in the art, preferably using titanium(III)chloride. Nitrobenzene derivatives of formula (8) are commercially available in many cases.

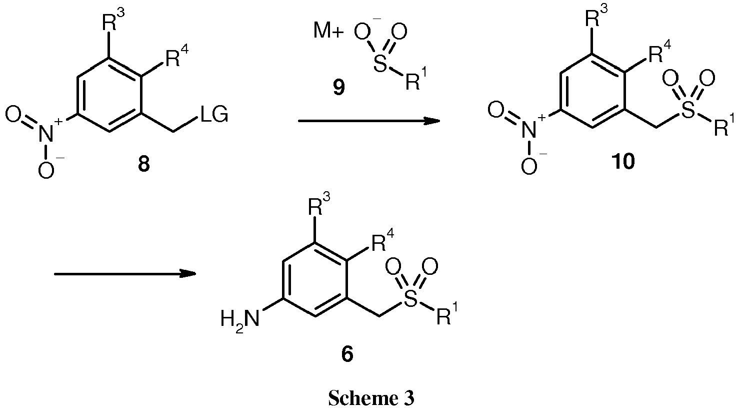

Scheme 4 outlines a further alternative approach to advanced intermediates of the formula (5). In the first step, a compound of formula **(3),** in which R² is as defined for the compound of general formula (I), is reacted with a suitable aniline of formula **(7),** in which R³ and R⁴ are as defined for the compound of general formula **(I),** to give a compound of formula **(11).** This coupling reaction can be carried out by a Palladium-catalyzed C-N cross-coupling reactions (for a review on C-N cross coupling reactions see for example: a) L. Jiang, S.L. Buchwald in ʹMetal-Catalyzed Cross-Coupling ʹReactionsʹ, 2nd ed.: A. de Meijere, F. Diederich, Eds.: Wiley-VCH: Weinheim, Germany, 2004**).**
Preferred is the herein described use of tris(dibenzylideneacetone)dipalladium(0), (9,9-dimethyl-9H-xanthene-4,5-diyl)bis(diphenylphosphane) and and cesium carbonate in dioxane. The reactions are preferably run under argon for 3-48 hours at 100°C in a microwave oven or in an oil bath.
Aniline derivatives of formula **(7)** are commercially available in many cases, or can be prepared by methods known to the person skilled in the art, *e.g.* by reduction of the corresponding carboxylic acids or esters thereof.

In the second step, a compound of formula **(11),** in which R², R³ and R⁴ are as defined for the compound of general formula **(I),** is converted to a compound of formula **(12),** in which R², R³ and R⁴ are as defined for the compound of general formula **(I)** and in which LG represents a leaving group, preferably chloro or bromo. Preferred is the herein described use of thionyl chloride in NMP or DMF and DCM for the formation the respective benzylic chlorides (LG = Cl). A possibility for the formation of the respective benzylic bromides (LG = Br) is the use of tetrabromomethane and triphenylphosphane in DCM (see for example: Polla et al, Bioorganic and Medicinal Chemistry, 2004, 12, 1151).

In the third step, a compound of formula **(12)** is converted to a thioether of formula **(5),** in which R¹, R², R³ and R⁴ are as defined for the compund of general formula **(I),** by reaction with suitable thiols of formula **(13),** in which R¹ is as defined for the compound of formula **(I),** under basic conditions (see for example: Sammond et al, Bioorg. Med. Chem. Lett. 2005, 15, 3519). Thiols of formula **(13)** are known to the person skilled in the art and are commercially available in considerable variety.

In the final step, the thioether of formula (5) is oxidized to the corresponding sulfone of formula **(I)** as described in scheme 1.

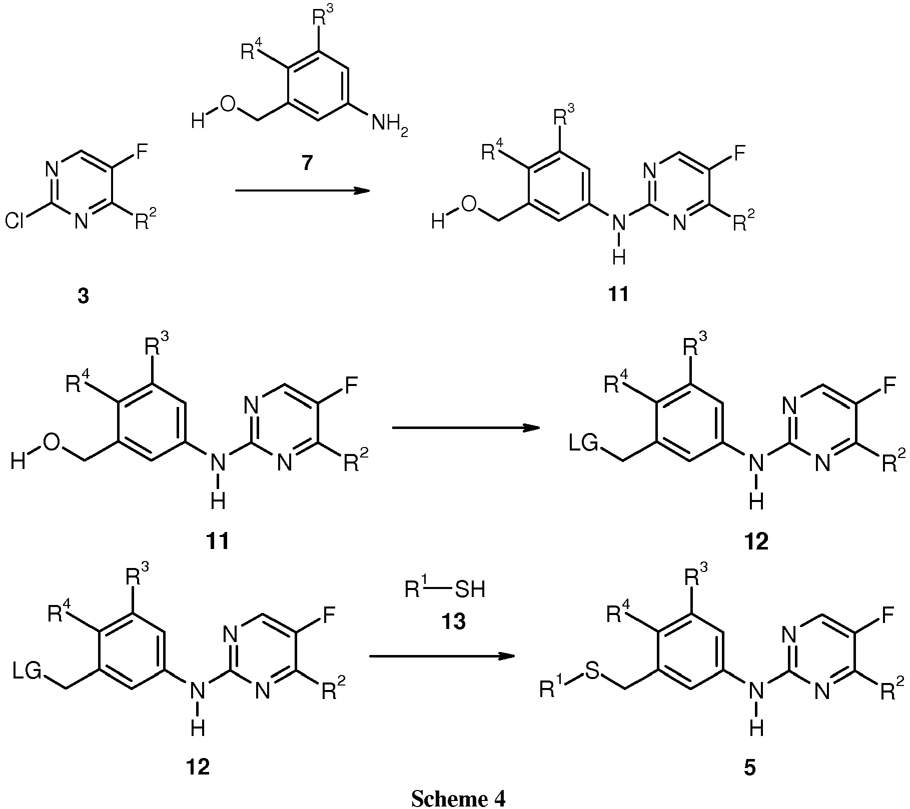

### Preparation of compounds:

**Abbreviations used in the description of the chemistry and in the Examples that follow are:**
approx. (approximately); br (broad); CDCl₃ (deuterated chloroform); cHex (cyclohexane); d (doublet); DCM (dichloromethane); DIPEA (di-iso-propylethylamine); DME (1,2-dimethoxyethane), DMF (dimethylformamide); DMSO (dimethyl sulfoxide); eq (equivalent); ES (electrospray); EtOAc (ethyl acetate); EtOH (ethanol); iPrOH (iso-propanol); HPLC (High Performance Liquid Chromatography); mCPBA (meta-chloroperoxybenzoic acid), MeCN (acetonitrile), MeOH (methanol); MS (mass spectrometry); NBS (N-bromosuccinimide), NMR (nuclear magnetic resonance); p (pentet); Pd(dppf)Cl₂ ([1,1'-bis(diphenylphosphino)ferrocene]dichloro palladium(II) complex with dichloromethane); iPrOH (iso-propanol); q (quartet); RT (room temperature); s (singlet); sat. aq. (saturated aqueous); SiO₂ (silica gel); sxt (sextet); TFA (trifluoroacetic acid); TFAA (trifluoroacetic anhydride), THF (tetrahydrofuran); tr (triplet).

The IUPAC names of the examples were generated using the program ʹACD/Name batch version 12.01ʹ from ACD LABS.

### Example 1:

### 4-(2,4-Difluorophenyl)-5-fluoro-N-{3-[(methylsulfonyl)methyl]-5-(trifluoromethyl)phenyl}pyrimidin-2-amine

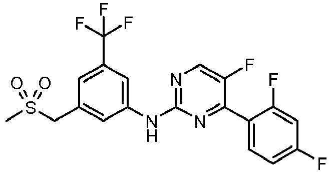

### Preparation of Intermediate 1.1:

### 2-Chloro-4-(2,4-difluorophenyl)-5-fluoropyrimidine

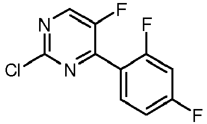

Under argon, a mixture of 2,4-dichloro-5-fluoropyrimidine (19.3 g; 115.5 mmol, Aldrich Chemical Company Inc.), (2,4-difluorophenyl)boronic acid (20.0 g; 127.0 mmol; Aldrich Chemical Company Inc.) and [1,1'-bis(diphenylphosphino)ferrocene]dichloropalladium(II) (9.4 g; 11.5 mmol; Aldrich Chemical Company Inc.) in a 2M solution of potassium carbonate (173 mL) and 1,2-dimethoxyethane (496 mL) was stirred for 90 minutes at 90 °C. After cooling, the batch was diluted with ethyl acetate and washed with diluted aqueous sodium chloride solution. The organic phase was filtered using a Whatman filter and concentrated. The residue was first purified by chromatography (hexane / ethyl acetate 20 % to 50 %) and then digested with hexane to give the desired product (15.0 g; 61.2 mmol).
¹H NMR (400MHz, CDCl₃, 300K) δ = 8.56 (m, 1H), 7.73 (m, 1H), 7.07 (m, 1H), 6.95 (m, 1H).

### Preparation of Intermediate 1.2:

### tert-Butyl {3-[(methylsulfanyl)methyl]-5-(trifluoromethyl)phenyl}carbamate

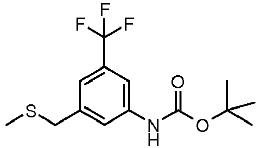

Sodium methanethiolate (2.32 g; 30.0 mmol) was added in two portions to a stirred solution of tert-butyl [3-(chloromethyl)-5-(trifluoromethyl)phenyl]carbamate (9.7 g; 30.0 mmol; Enamine) in ethanol (185 mL) at -40 °C. The cold bath was removed and the batch was stirred at room temperature for 2 hours. Additional sodium methanethiolate (0.46 g; 5.9 mmol) was added and the mixture was stirred for 2 hours at room temperature. The batch was diluted with saturated aqueous sodium chloride solution and extracted with ethyl acetate (2x). The combined organic layers were washed with water, dried (sodium sulfate), filtered and concentrated to give the desired product (10.0 g) that was used without further purification.
¹H NMR (400MHz, CDCl₃, 300K) δ = 7.57 (s, 1H), 7.51 (s, 1H), 7.23 (s, 1H), 6.59 (s, 1H), 3.67 (s, 2H), 2.00 (s, 3H), 1.53 (s, 9H).

### Preparation of Intermediate 1.3:

### 3-[(Methylsulfanyl)methyl]-5-(trifluoromethyl)aniline

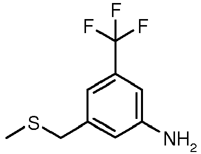

TFA (2.5 mL) was added to a stirred solution of tert-butyl {3-[(methylsulfanyl)methyl]-5-(trifluoromethyl)phenyl}carbamate (502 mg; 1.56 mmol) in DCM (5 mL) at 0°C. The ice bath was removed and the mixture was stirred for 45 min at RT. The batch was concentrated and saturated aqueous sodium bicarbonate solution was added. The batch was extracted with ethyl acetate (2x). The combined organic layers were dried (sodium sulfate), filtered and concentrated to give the crude product (336 mg), that was used without further purification.
¹H NMR (400MHz, CDCl₃, 300K) δ = 6.92 (s, 1H), 6.80 (s, 1H), 6.78 (s, 1H), 3.83 (br, 2H), 3.61 (s, 3H), 2.01 (s, 2H).

### Preparation of Intermediate 1.4:

### 4-(2,4-Difluorophenyl)-5-fluoro-N-{3-[(methylsulfanyl)methyl]-5-(trifluoromethyl)phenyl}pyrimidin-2-amine

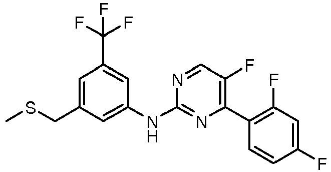

A 4N solution of hydrogen chloride in dioxane (0.63 mL; 2.52 mmol) was added to a mixture of 2-chloro-4-(2,4-difluorophenyl)-5-fluoropyrimidine (618 mg; 2.53 mmol) and 3-[(methylsulfanyl)methyl]-5-(trifluoromethyl)aniline (658; 2.53 mmol) in 1-butanol (7.5 mL) at room temperature. The batch was stirred at 140 °C for 3 days. After cooling, the batch was concentrated and the residue was purified by chromatography (hexane / ethyl acetate 7% to 60%) to give the desired product (711 mg; 1.54 mmol).
¹H NMR (400MHz, CDCl₃, 300K) δ = 8.41 (m, 1H), 7.98 (s, 1H), 7.73 (m, 1H), 7.71 (s, 1H), 7.29 (s, 1H), 7.22 (s, 1H), 7.06 (m, 1H), 6.98 (m, 1H), 3.70 (s, 2H), 2.02 (s, 3H).

### Preparation of end product:

Potassium permanganate (102 mg; 0.63 mmol) was added to a solution of 4-(2,4-difluorophenyl)-5-fluoro-N-{3-[(methylsulfanyl)methyl]-5-(trifluoromethyl)phenyl}pyrimidin-2-amine (93 mg; 0.22 mmol) in acetone (2.2 mL) at RT. The mixture was stirred at 40 °C for 35 minutes. The batch was concentrated and the residue was purified by chromatography (DCM to DCM / EtOH 95:5) to give the desired product (62 mg; 0.14 mmol).
¹H NMR (400MHz, d6-DMSO, 300K) δ = 10.34 (s, 1H), 8.75 (m, 1H), 8.27 (s, 1H), 8.01 (s, 1H), 7.84 (m, 1H), 7.48 (m, 1H), 7.32 (m, 2H), 4.58 (s, 2H), 2.96 (s, 3H).

### Example 2:

### 4-(2,4-Difluorophenyl)-5-fluoro-N-{3-[(propan-2-ylsulfonyl)methyl]phenyl}pyrimidin-2-amine

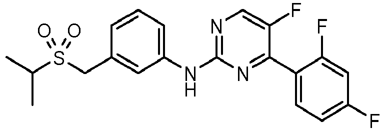

A 4N solution of hydrogen chloride in dioxane (0.15 mL; 0.61 mmol) was added to a mixture of 2-chloro-4-(2,4-difluorophenyl)-5-fluoropyrimidine (150 mg; 0.61 mmol) and 3-[(propan-2-ylsulfonyl)methyl]aniline (130 mg; 0.61 mmol; UkrOrgSynthesis Ltd.) in dioxane (0.1 mL). The batch was stirred at 140 °C for 5 hours. After cooling, the batch was slightly basified with sodium bicarbonate solution and extracted with DCM. The combined organic layers were filtered using a Whatman filter and concentrated. The residue was purified by preparative HPLC to give the desired product (51 mg; 0.12 mmol).

| | |
|---|---|
| *System:* | Waters Autopurificationsystem: Pump 2545, Sample Manager 2767, CFO, DAD 2996, ELSD 2424, SQD 3001 |
| *Column:* | XBrigde C18 5µm 100x30 mm |
| *Solvent:* | A = H₂O + 0.1% HCOOH |
| | B = MeCN |
| *Gradient:* | 0-1 min 1% B, 1-8 min 1-99% B, 8-10 min 99% B |
| *Flow:* | 50 mL/min |
| *Temperature:* | RT |
| *Solution:* | Max. 250 mg / max. 2.5 mL DMSO or DMF |
| *Injektion:* | 1 x 2.5 mL |
| *Detection:* | DAD scan range 210-400 nm |
| | MS ESI+, ESI-, scan range 160-1000 m/z |

¹H NMR (400MHz, CDCl₃, 300K) δ = 8.38 (m, 1H), 7.85 (s, 1H), 7.74 (m, 1H), 7.58 (m, 1H), 7.35 (m, 1H), 7.25 (s, 1H), 7.08 (m, 1H), 7.01 (m, 1H), 6.97 (m, 1H), 4.22 (s, 2H), 3.04 (m, 1H), 1.36 (d, 6H).

### Example 3:

### N-{3-[(tert-Butylsulfonyl)methyl]phenyl}-4-(2,4-difluorophenyl)-5-fluoropyrimidin-2-amine

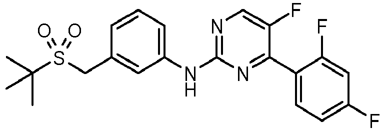

Example 3 was prepared under similar conditions as described in the preparation of Example 2 using 3-[(tert-butylsulfonyl)methyl]aniline (purchased from UkrOrgSynthesis Ltd.) and 2-chloro-4-(2,4-difluorophenyl)-5-fluoropyrimidine.
¹H NMR (400MHz, CDCl₃, 300K) δ = 8.37 (m, 1H), 7.81 (m, 1H), 7.76 (m, 1H), 7.81 (m, 1H), 7.34 (m, 1H), 7.25 (m, 1H), 7.07 (m, 2H), 6.96 (m, 1H), 4.20 (s, 2H), 1.44 (s, 9H).

### Example 4:

### 4-(2,4-Difluorophenyl)-5-fluoro-N-(3-{[(2-methoxyethyl)sulfonyl]methyl}phenyl)pyrimidin-2-amine

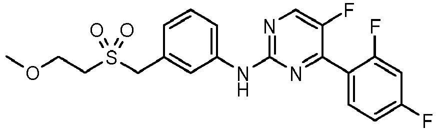

Example 4 was prepared under similar conditions as described in the preparation of Example 2 using 3-{[(2-methoxyethyl)sulfonyl]methyl}aniline (purchased from UkrOrgSynthesis Ltd.) and 2-chloro-4-(2,4-difluorophenyl)-5-fluoropyrimidine.
¹H NMR (400MHz, CDCl₃, 300K) δ = 8.37 (m, 1H), 7.75 (m, 1H), 7.73 (m, 1H), 7.65 (m, 1H), 7.35 (m, 1H), 7.24 (m, 1H), 7.11 (m, 1H), 7.08 (m, 1H), 6.97 (m, 1H), 4.32 (s, 2H), 3.81 (tr, 2H), 3.42 (s, 3H), 3.09 (tr, 2H).

### Reference- Example 5:

### 4-(2,4-Difluorophenyl)-5-fluoro-N-{3-[(methylsulfonyl)methyl]phenyl}pyrimidin-2-amine

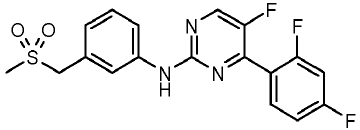

Reference- Example 5 was prepared under similar conditions as described in the preparation of Example 2 using 3-[(methylsulfonyl)methyl]aniline (purchased from UkrOrgSynthesis Ltd.) and 2-chloro-4-(2,4-difluorophenyl)-5-fluoropyrimidine.
¹H NMR (400MHz, CDCl₃, 300K) δ = 8.38 (m, 1H), 7.85 (m, 1H), 7.72 (m, 1H), 7.57 (m, 1H), 7.38 (m, 1H), 7.31 (s, 1H), 7.06 (m, 2H), 6.97 (m, 1H), 4.25 (s, 2H), 2.77 (s, 3H).

### Example 6:

### 4-(2,4-Difluorophenyl)-5-fluoro-N-{3-[(methylsulfonyl)methyl]-5-(pentafluoro-λ⁶-sulfanyl)phenyl}pyrimidin-2-amine

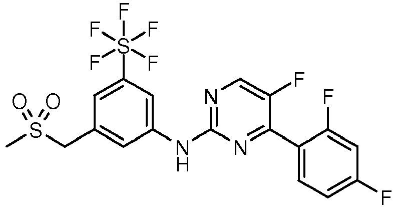

### Preparation of Intermediate 6.1:

### [3-{[4-(2,4-difluorophenyl)-5-fluoropyrimidin-2-yl]amino}-5-(pentafluoro-λ⁶-sulfanyl)phenyl]methanol

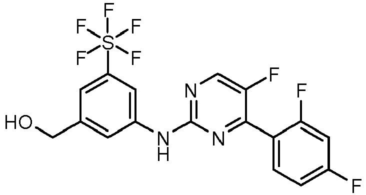

To a mixture of 2-chloro-4-(2,4-difluorophenyl)-5-fluoropyrimidin (10.0 g; 38.8 mmol) and [3-amino-5-(pentafluoro-λ⁶-sulfanyl)phenyl]methanol ([CAS Nr.1427316-37-7]; 10.0 g; 39.3 mmol) in 1-butanol (20 mL) trifluoroacetic acid (3.0 mL; 38.6 mmol) was added and the mixture was stirred for 17 hours at 140 °C in a sealed tube. The batch was cooled and concentrated to give the crude product (25.3 g) which was purified by column chromatography on silica gel (hexane / EtOAc, 10-80 %) to give the desired product (10.25 g).
¹H NMR (400MHz, DMSO-d6) δ [ppm] = 10.30 (s, 1 H), 8.76 (d, 1 H), 8.41 (s, 1 H), 7.83 - 7.89 (m, 1 H), 7.75 - 7.83 (m, 1 H), 7.45 - 7.56 (m, 1 H), 7.38 (s, 1 H), 7.26 - 7.35 (m, 1 H), 5.46 (br. s., 1 H), 4.54 (d, 2 H).

### Preparation of Intermediate 6.2:

### N-[3-(Chloromethyl)-5-(pentafluoro-λ⁶-sulfanyl)phenyl]-4-(2,4-difluorophenyl)-5-fluoropyrimidin-2-amine

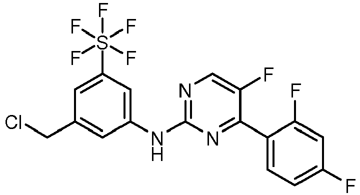

A suspension of [3-{[4-(2,4-difluorophenyl)-5-fluoropyrimidin-2-yl]amino}-5-(pentafluoro-λ⁶-sulfanyl)phenyl]methanol (11.42 g; 23.7 mmol) in DCM (60 mL) at 0 °C was treated with thionyl chloride (14 mL, 119 mmol). The mixture was stirred for 3 hours at 0 °C to 25 °C. The batch was concentrated to give the crude product (12.49 g) as the hydrochloride which was used without further purification.
¹H NMR (300MHz, DMSO-d6, 300K) δ [ppm] = 10.40 (s, 1 H), 8.78 (d, 1 H), 8.46 (s, 1 H), 8.04 (s, 1 H), 7.76 - 7.89 (m, 1 H), 7.53 - 7.58 (m, 1 H), 7.44 - 7.52 (m, 1 H), 7.27 - 7.38 (m, 1 H), 4.84 (m, 2 H).

### Preparation of Intermediate 6.3:

### 4-(2,4-Difluorophenyl)-5-fluoro-N-{3-[(methylsulfanyl)methyl]-5-(pentafluoro-λ⁶-sulfanyl)phenyl}pyrimidin-2-amine

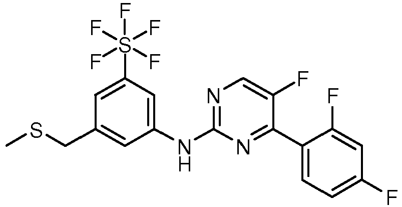

Sodium methanethiolate (3.23 g; 41.4 mmol) was added in three portions to a stirred solution of N-[3-(chloromethyl)-5-(pentafluoro-λ⁶-sulfanyl)phenyl]-4-(2,4-difluorophenyl)-5-fluoropyrimidin-2-amine hydrochloride (11.4 g; 21.6 mmol) in ethanol (100 mL) at -15°C. The cooling bath was removed and the batch was stirred at room temperature for 7 hours. The batch was diluted with saturated aqueous sodium chloride solution and extracted with ethyl acetate (2x). The combined organic layers were washed with water, dried (sodium sulfate), filtered and concentrated to give the desired product (11.38 g) that was used without further purification.
¹H NMR (400MHz, CDCl₃, 300K) δ [ppm] = 8.45 (s, 1 H), 8.29 (s, 1 H), 7.71 - 7.84 (m, 1 H), 7.64 (s, 1 H), 7.37 (br. s., 2 H), 7.09 (tr, 1 H), 6.91 - 7.05 (m, 1 H), 3.73 (s, 2 H), 2.05 (s, 3 H).

### Preparation of end product:

Example 6 was prepared under similar conditions as described in the preparation of Example 1 using 4-(2,4-difluorophenyl)-5-fluoro-N-{3-[(methylsulfanyl)methyl]-5-(pentafluoro-λ⁶-sulfanyl)phenyl}pyrimidin-2-amine (55 mg; 0.09 mmol) to give the desired product (21 mg; 0.03 mmol). ¹H NMR (DMSO-*d₆*) [ppm] = 10.41 (s, 1 H), 8.77 (d, 1 H), 8.52 (s, 1 H), 7.93 - 7.99 (m, 1 H), 7.79 - 7.89 (m, 1 H), 7.46 - 7.55 (m, 2 H), 7.28 - 7.36 (m, 1 H), 4.62 (s, 2 H), 2.97 (s, 3 H).

### Example 7:

### 4-(2,4-Difluorophenyl)-N-{3-[(ethylsulfonyl)methyl]phenyl}-5-fluoropyrimidin-2-amine

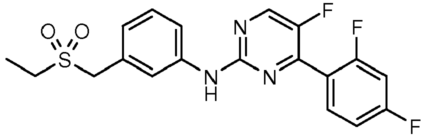

### Preparation of Intermediate 7.1:

### 1-[(Ethylsulfanyl)methyl]-3-nitrobenzene

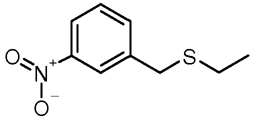

Sodium methanethiolate (5.39 g; 64 mmol) was added to a stirred solution of 1-(chloromethyl)-3-nitrobenzene (10.0 g; 58.3 mmol; Aldrich Chemical Company Inc.) in ethanol (120 mL) at -15°C. The cold bath was removed and the batch was stirred at room temperature for 3 hours. The batch was diluted with saturated aqueous sodium chloride solution and extracted with ethyl acetate (2x). The combined organic layers were washed with water, dried (sodium sulfate), filtered and concentrated to give the desired product (10.15 g) that was used without further purification.
¹H-NMR (400 MHz, CDCl₃): δ [ppm] = 8.20 (tr, 1H), 8.11 (dtr, 1H), 7.68 (d, 1H), 7.53-7.47 (m, 1H), 3.81 (s, 2H), 2.46 (q, 2H), 1.25 (t, 3H).

### Preparation of Intermediate 7.2:

### 1-[(Ethylsulfanyl)methyl]-3-nitrobenzene

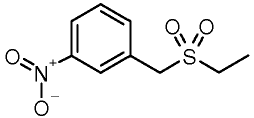

A solution of 1-[(ethylsulfanyl)methyl]-3-nitrobenzene (800 mg) in DCM (70 mL) was treated at 0 °C with portions of *meta*-chloroperbenzoic acid (1.98 g, 70%). The mixture was stirred at 0°C for further 30 minutes and then 18 hours at room temperature. The reaction mixture was diluted with DCM before sodium hydrogen sulfite and sodium bicarbonate solution was added and extracted with DCM (2x). The combinded organic layers were washed and concentrated. The residue was purified by column chromatography on silca gel (hexane / ethyl acetate) to give the title compound (824 mg; 3.59 mmol).
¹H-NMR (400 MHz, DMSO-d6): δ [ppm] = 8.31 (tr, 1H), 8.28-8.22 (m, 1H), 7.86 (d, 1H), 7.75-7.68 (m, 1H), 4.70 (s, 2H), 3.09 (q, 2H), 1.24 (tr, 3H).

### Preparation of Intermediate 7.3:

### 3-[(Ethylsulfonyl)methyl]aniline

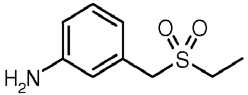

Titanium(III)chloride solution (approx. 15%) in approx. 10% hydrochloric acid (purchased from MERCK-SCHUCHARDT; 16 ml) was added to a stirred solution of 1-[(ethylsulfanyl)methyl]-3-nitrobenzene (810 mg) in THF (41 ml) at room temperature and the batch was stirred for 16 hours. The batch was cooled with an ice bath while 1N sodium hydroxide solution was added to raise the pH value of the reaction mixture to 8-9. It was stirred for 30 minutes at this temperature before the batch was extracted with ethyl acetate (2x). The combined organic layers were washed with brine, filtered using a Whatman filter and concentrated. The residue was purified by column chromatography on silica gel (hexane / ethyl acetate 1:1 to ethyl acetate) to give the desired product (560 mg; 2.67 mmol).
¹H-NMR (400 MHz, DMSO-d6): δ [ppm] = 7.00 (tr, 1H), 6.62-6.48 (m, 3H), 5.14 (s, 2H), 4.24 (s, 2H), 2.97 (q, 2H), 1.20 (tr, 3H).

### Preparation of end product:

A batch with 3-[(ethylsulfonyl)methyl]aniline (48 mg; 0.24 mmol), 2-chloro-4-(2,4-difluorophenyl)-5-fluoropyrimidine (80 mg; 0.31 mmol), chloro(2-dicyclohexylphosphino-2',4',6'-tri-iso-propyl-1,1'-biphenyl)[2-(2-aminoethyl)phenyl] palladium(II) methyl-tert-butylether adduct (14,8 mg; 0.018 mmol; ABCR GmbH & CO. KG), 2-dicyclohexylphosphino-2',4',6'-triisopropylbiphenyl (8.5 mg; 0.018 mmol; Aldrich Chemical Company Inc.) and potassium phosphate (253 mg; 1,19 mmol) in toluene (2.3 ml) and 1-methylpyrrolidin-2-one (0.4 ml) was degassed using argon. The batch was stirred under argon for 3 hours at 130°C. After cooling, the batch was diluted with saturated aqueous sodium chloride solution and extracted with ethyl acetate (2x). The combined organic layers were filtered using a Whatman filter and concentrated. The residue was purified by preparative HPLC to give the desired product (45 mg; 0.11 mmol).

| | |
|---|---|
| *System:* | Waters Autopurificationsystem: Pump 2545, Sample Manager 2767, CFO, DAD 2996, ELSD 2424, SQD 3001 |
| *Column:* | XBrigde C18 5µm 100x30 mm |
| *Solvent:* | A = H₂O + 0.1% HCOOH |
| | B = MeCN |
| *Gradient:* | 0-1 min 1% B, 1-8 min 1-99% B, 8-10 min 99% B |
| *Flow:* | 50 mL/min |
| *Temperature:* | RT |
| *Solution:* | Max. 250 mg / max. 2.5 mL DMSO or DMF |
| *Injektion:* | 1 x 2.5 mL |
| *Detection:* | DAD scan range 210-400 nm |
| | MS ESI+, ESI-, scan range 160-1000 m/z |

¹H-NMR (400 MHz, DMSO-d6): δ [ppm] = 9.96 (s, 1H), 8.68 (d, 1H), 7.85 (trd, 1H), 7.80 (s, 1H), 7.71 (dd, 1H), 7.49 (ddd, 1H), 7.36-7.26 (m, 2H), 7.00 (d, 1H), 4.40 (s, 2H), 3.02 (q, 2H), 1.20 (t, 3H).

### Example 8:

### 4-(2,4-difluorophenyl)-5-fluoro-N-{3-[(propylsulfonyl)methyl]phenyl}pyrimidin-2-amine

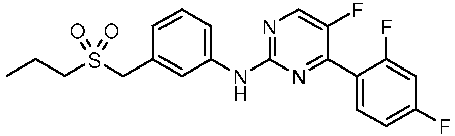

### Preparation of Intermediate 8.1:

### 1-Nitro-3-[(propylsulfanyl)methyl]benzene

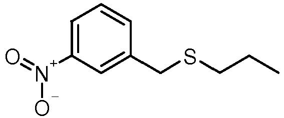

Propane-1-thiol (500 mg; 6.5 mmol) and 1-(chloromethyl)-3-nitrobenzene (1.03 g; 5.9 mmol; Aldrich Chemical Company Inc.) were dissolved in DMF (15 mL). Cesium carbonate was (3.85 g; 11.8 mmol) was added and the batch was stirred at ambient temperatur for 2 hours. DMF was removed under reduced pressure. The residue was partitioned between water and ethyl acetate. The organic layer was washed with water, dried (sodium sulfate), filtered and concentrated to give the crude product which was purified by column chromatography on silica gel (hexane / ethyl acetate) to give the desired product (1.19 g; 5.46 mmol).
1H-NMR (400 MHz, DMSO-d6): δ [ppm] = 8.19 (t, 1H), 8.10 (ddd, 1H), 7.79 (d, 1H), 7.66-7.59 (m, 1H), 3.88 (s, 2H), 2.39 (tr, 2H), 1.52 (sxt, 2H), 0.89 (t, 3H).

### Preparation of Intermediate 8.2:

### 1-Nitro-3-[(propylsulfonyl)methyl]benzene

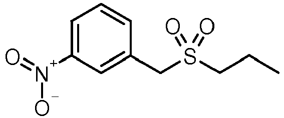

Intermediate 8.2 was prepared under similar conditions as described in the preparation of Intermediate 7.2 using 1-nitro-3-[(propylsulfanyl)methyl]benzene and *meta*-chloroperbenzoic acid. The batch was purified by column chromatography on silica gel (hexane / ethyl acetate).
1H-NMR (400 MHz, DMSO-d6): δ [ppm] = 8.19 (tr, 1H), 8.10 (ddd, 1H), 7.79 (d, 1H), 7.66-7.59 (m, 1H), 3.88 (s, 2H), 2.39 (tr, 2H), 1.52 (sxt, 2H), 0.89 (tr, 3H).

### Preparation of Intermediate 8.3

### 3-[(Propylsulfonyl)methyl]aniline

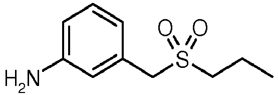

Intermediate 8.3 was prepared under similar conditions as described in the preparation of Intermediate 7.3 using 1-nitro-3-[(propylsulfonyl)methyl]benzene and titanium(III)chloride solution (approx. 15%) in approx. 10% hydrochloric acid (purchased from Merck Schuchardt OHG). The batch was purified by column chromatography on silica gel (hexane / ethyl acetate).
¹H NMR (400MHz, DMSO-d₆) δ = 7.00 (tr, 1H), 6.60-6.48 (m, 3H), 5.14 (s, 2H), 4.23 (s, 2H), 3.00-2.91 (m, 2H), 1.75-1.63 (m, 2H), 0.96 (tr, 3H)

### Preparation of end product:

A batch with 3-[(propylsulfonyl)methyl]aniline (55 mg; 0.24 mmol), 2-chloro-4-(2,4-difluorophenyl)-5-fluoropyrimidine (80 mg; 0.31 mmol), chloro(2-dicyclohexylphosphino-2',4',6'-tri-iso-propyl-1,1'-biphenyl)[2-(2-aminoethyl)phenyl] palladium(II) methyl-tert-butylether adduct (14,8 mg; 0.018 mmol; ABCR GmbH & CO. KG), 2-dicyclohexylphosphino-2',4',6'-triisopropylbiphenyl (8.5 mg; 0.018 mmol; Aldrich Chemical Company Inc.) and potassium phosphate (253 mg; 1,19 mmol) in toluene (2.3 ml) and 1-methylpyrrolidin-2-one (0.4 ml) was degassed using argon. The batch was stirred under argon for 3 hours at 130°C. After cooling, the batch was diluted with saturated aqueous sodium chloride solution and extracted with ethyl acetate (2x). The combined organic layers were filtered using a Whatman filter and concentrated. The residue was purified by preparative HPLC to give the desired product (67 mg; 0.15 mmol).

| | |
|---|---|
| *System:* | Waters Autopurificationsystem: Pump 2545, Sample Manager 2767, CFO, DAD 2996, ELSD 2424, SQD 3001 |
| *Column:* | XBrigde C18 5µm 100x30 mm |
| *Solvent:* | A = H₂O + 0.1% HCOOH |
| | B = MeCN |
| *Gradient:* | 0-1 min 1% B, 1-8 min 1-99% B, 8-10 min 99% B |
| *Flow:* | 50 mL/min |
| *Temperature:* | RT |
| *Solution:* | Max. 250 mg / max. 2.5 mL DMSO or DMF |
| *Injektion:* | 1 x 2.5 mL |
| *Detection:* | DAD scan range 210-400 nm |
| | MS ESI+, ESI-, scan range 160-1000 m/z |

¹H NMR (400MHz, DMSO-d₆) δ = 9.96 (s, 1H), 8.67 (d, 1H), 7.85 (trd, 1H), 7.80 (tr, 1H), 7.72 (dd, 1H), 7.48 (ddd, 1H), 7.35-7.27 (m, 2H), 7.00 (d, 1H), 4.39 (s, 2H), 3.03-2.96 (m, 2H), 1.75-1.64 (m, 2H), 0.94 (tr, 3H).

### Example 9:

### N-{3-[(Cyclopropylsulfonyl)methyl]phenyl}-4-(2,4-difluorophenyl)-5-fluoropyrimidin-2-amine

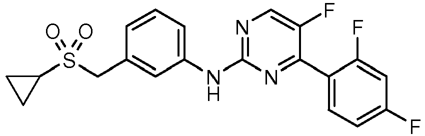

### Preparation of Intermediate 9.1:

### 1-[(Cyclopropylsulfonyl)methyl]-3-nitrobenzene

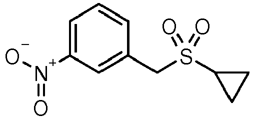

Sodium cyclopropanesulfinate (1.04 g; 8.1 mmol) was added to a stirred solution of 1-(bromomethyl)-3-nitrobenzene (1.17 g; 5.4 mmol) in acetonitrile (50 ml) at room temperature. The batch was stirred at 90°C for 4 hours. After cooling, the batch was diluted with water and extracted with DCM (2x). The combined organic layers were filtered using a Whatman filter and concentrated to give the desired product (1.26 g) that was used without further purification.
¹H NMR (400MHz, CDCl₃, 300K) δ = 8.28 (m, 2H), 7.81 (m, 1H), 7.61 (m, 1H), 4.37 (s, 2H), 2.29 (m, 1H), 1.21 (m, 2H), 1.03 (m, 2H).

### Preparation of Intermediate 9.2:

### 3-[(Cyclopropylsulfonyl)methyl]aniline

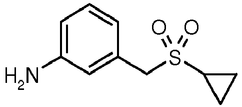

Intermediate 9.2 was prepared under similar conditions as described in the preparation of Intermediate 7.3 using 1-[(cyclopropylsulfonyl)methyl]-3-nitrobenzene and titanium(III)chloride solution (approx. 15%) in approx. 10% hydrochloric acid (purchased from Merck Schuchardt OHG). The batch was purified by chromatography (hexane / ethyl acetate).
¹H NMR (400MHz, CDCl₃) δ = 7.15 (m, 1H), 6.77 (m, 2H), 6.67 (m, 1H), 4.16 (s, 2H), 3.70 (br, 2H), 2.23 (m, 1H), 1.15 (m, 2H), 0.94 (m, 2H).

### Preparation of end product:

A batch with 3-[(cyclopropylsulfonyl)methyl]aniline (97 mg; 0.45 mmol), 2-chloro-4-(2,4-difluorophenyl)-5-fluoropyrimidine (150 mg; 0.58 mmol), chloro(2-dicyclohexylphosphino-2',4',6'-tri-iso-propyl-1,1'-biphenyl)[2-(2-aminoethyl)phenyl] palladium(II) methyl-*tert*-butylether adduct (27.8 mg; 0.034 mmol; ABCR GmbH & CO. KG), 2-dicyclohexylphosphino-2',4',6'-triisopropylbiphenyl (16 mg; 0.034 mmol; Aldrich Chemical Company Inc.) and potassium phosphate (475 mg; 2.24 mmol) in toluene (6.6 ml) and 1-methylpyrrolidin-2-one (1 ml) was degassed using argon. The batch was stirred under an atmosphere of argon for 3 hours at 130°C. After cooling, the batch was diluted with saturated aqueous sodium chloride solution and extracted with ethyl acetate (2x). The combined organic layers were filtered using a Whatman filter and concentrated. The residue was purified by preparative HPLC to give the desired product (50 mg; 0.12 mmol).

| | |
|---|---|
| *System:* | Waters Autopurificationsystem: Pump 2545, Sample Manager 2767, CFO, DAD 2996, ELSD 2424, SQD 3001 |
| *Column:* | XBrigde C18 5µm 100x30 mm |
| *Solvent:* | A = H₂O + 0.1% HCOOH |
| | B = MeCN |
| *Gradient:* | 0-1 min 1% B, 1-8 min 1-99% B, 8-10 min 99% B |
| *Flow:* | 50 mL/min |
| *Temperature:* | RT |
| *Solution:* | Max. 250 mg / max. 2.5 mL DMSO or DMF |
| *Injektion:* | 1 x 2.5 mL |
| *Detection:* | DAD scan range 210-400 nm |
| | MS ESI+, ESI-, scan range 160-1000 m/z |

¹H NMR (400MHz, DMSO-d₆) δ = 9.96 (s, 1H), 8.68 (d, 1H), 7.88-7.80 (m, 2H), 7.72 (dd, 1H), 7.49 (ddd, 1H), 7.35-7.26 (m, 2H), 7.02 (d, 1H), 4.44 (s, 2H), 2.57-2.52 (m, 1H), 0.97-0.85 (m, 4H).

### Example 10:

### 4-(2,4-Difluorophenyl)-5-fluoro-N-{3-fluoro-5-[(methylsulfonyl)methyl]phenyl}pyrimidin-2-amine

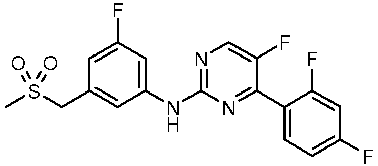

### Preparation of Intermediate 10.1:

### 1-Fluoro-3-[(methylsulfanyl)methyl]-5-nitrobenzene

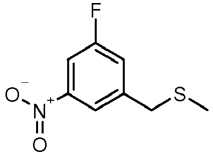

Sodium methanethiolate (1.22 g; 17.4 mmol) was added in three portions to a stirred solution of 1-(chloromethyl)-3-fluoro-5-nitrobenzene (3.00 g; 15.8 mmol, HE Chemical Co., Ltd.) in ethanol (33 ml) at 0°C. The ice bath was removed and the batch was stirred at room temperature for 18 hours. Further sodium methanethiolate (0.33 g; 4.7 mmol) was added and the batch was stirred for 5 additional hours at room temperature. The batch was diluted with brine and extracted with ethyl acetate (2x). The combined organic layers were washed with water, dried (sodium sulfate), filtered and concentrated to give the desired product (3.4 g) that was used without further purification.
¹H NMR (400MHz, CDCl₃, 300K) δ = 8.00 (m, 1H), 7.81 (m, 1H), 7.42 (m, 1H), 3.74 (s, 2H), 2.02 (s, 3H).

### Preparation of Intermediate 10.2:

### 1-Fluoro-3-[(methylsulfonyl)methyl]-5-nitrobenzene

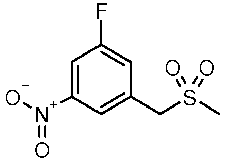

*meta*-Chloroperbenzoic acid (77%; 3.68 g; 16.4 mmol) was added to a stirred solution of 1-fluoro-3-[(methylsulfanyl)methyl]-5-nitrobenzene (1.50 g) in DCM (178 ml) at 0°C. The batch was stirred at 0°C for 30 minutes and then 2.5 hours at room temperature. The batch was diluted with water (450 ml) before sodium bicarbonate (1.50 g) was added. The batch was extracted with DCM (2x). The combinded organic layers were filtered using a Whatman filter and concentrated to give the crude product (3.33 g) that was used without further purification.

### Preparation of Intermediate 10.3:

### 3-Fluoro-5-[(methylsulfonyl)methyl]aniline

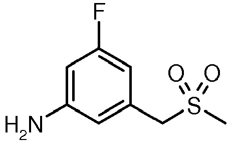

Titanium(III)chloride solution (approx. 15%) in approx. 10% hydrochloric acid (purchased from MERCK-SCHUCHARDT; 29 ml) was added to a stirred solution of crude 1-fluoro-3-[(methylsulfonyl)methyl]-5-nitrobenzene (1.00 g) in THF (45 ml) at room temperature and the batch was stirred for 16 hours. The batch was cooled with an ice bath while 1N sodium hydroxide solution was added to raise the pH value of the reaction mixture to 8-9. The mixture was stirred for 30 minutes at this temperature before the batch was extracted with ethyl acetate (2x). The combined organic layers were washed with brine, filtered using a Whatman filter and concentrated. The residue was purified by column chromatography on silica gel (hexane / ethyl acetate 1:1 to ethyl acetate) to give the desired product (262 mg; 1.29 mmol).
¹H NMR (400MHz, CDCl₃, 300K) δ = 6.48 (m, 2H), 6.39 (m, 1H), 4.11 (s, 2H), 3.88 (br, 2H), 2.79 (s, 3H).

### Preparation of end product:

The end product was prepared under similar conditions as described in the preparation of example 9 using 3-fluoro-5-[(methylsulfonyl)methyl]aniline (42.4 mg; 0.19 mmol) and 2-chloro-4-(2,4-difluorophenyl)-5-fluoropyrimidine (65 mg; 0.25 mmol). Preparative HPLC gave the desired product (44.8 mg; 0.11 mmol).

| | |
|---|---|
| *System:* | Waters Autopurificationsystem: Pump 2545, Sample Manager 2767, CFO, DAD 2996, ELSD 2424, SQD 3001 |
| *Column:* | XBrigde C18 5µm 100x30 mm |
| *Solvent:* | A = H₂O + 0.1% HCOOH |
| | B = MeCN |
| *Gradient:* | 0-1 min 1% B, 1-8 min 1-99% B, 8-10 min 99% B |
| *Flow:* | 50 mL/min |
| *Temperature:* | RT |
| *Solution:* | Max. 250 mg / max. 2.5 mL DMSO or DMF |
| *Injektion:* | 1 x 2.5 mL |
| *Detection:* | DAD scan range 210-400 nm |
| | MS ESI+, ESI-, scan range 160-1000 m/z |

¹H NMR (400MHz, DMSO-d₆) δ = 10.22 (s, 1H), 8.73 (d, 1H), 7.84 (trd, 1H), 7.76 (dtr, 1H), 7.54 (s, 1H), 7.50 (ddd, 1H), 7.33 (trd, 1H), 6.86-6.81 (m, 1H), 4.46 (s, 2H), 2.94 (s, 3H).

### Example 11:

### 5-Fluoro-N-{3-fluoro-5-[(methylsulfonyl)methyl]phenyl}-4-(2,4,5-trifluorophenyl)pyrimidin-2-amine

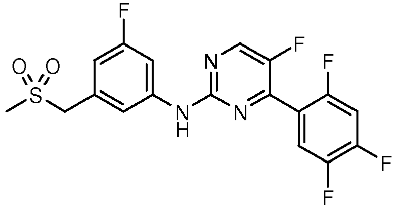

### Preparation of Intermediate 11.1:

### 2-Chloro-5-fluoro-4-(2,4,5-trifluorophenyl)pyrimidine

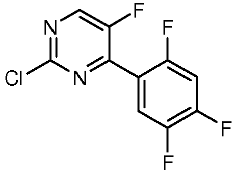

Under an atmosphere of argon, a mixture of 2,4-dichloro-5-fluoropyrimidine (1.31 g; 7.6 mmol, Aldrich Chemical Company Inc.), (2,4,5-trifluorophenyl)boronic acid (1.5 g; 8.35 mmol; Aldrich Chemical Company Inc.) and [1,1'-bis(diphenylphosphino)ferrocene]dichloropalladium(II) (620 mg; 0.76 mmol; Aldrich Chemical Company Inc.) in a 2M solution of potassium carbonate (11.39 mL) and 1,2-dimethoxyethane (39.5 mL) was stirred for 90 minutes at ambient temperature. The batch was diluted with ethyl acetate and washed with diluted aqueous sodium chloride solution. The organic layer was dried over sodium sulphate and concentrated. The residue was purified by column chromatography on silica gel (hexane / ethyl acetate) to give the desired product (1.4 g; 5.22 mmol).
¹H-NMR (400 MHz, DMSO-*d₆*): δ [ppm] = 9.08 (d, 1H), 7.91-7.81 (m, 2H).

### Preparation of end product:

The end product was prepared under similar conditions as described in the preparation of example 9 using 3-fluoro-5-[(methylsulfonyl)methyl]aniline (40.8 mg; 0.19 mmol) and 2-chloro-5-fluoro-4-(2,4,5-trifluorophenyl)pyrimidine (65 mg; 0.24 mmol). Preparative HPLC gave the desired product (44.6 mg; 0.1 mmol).

| | |
|---|---|
| *System:* | Waters Autopurificationsystem: Pump 2545, Sample Manager 2767, CFO, DAD 2996, ELSD 2424, SQD 3001 |
| *Column:* | XBrigde C18 5µm 100x30 mm |
| *Solvent:* | A = H₂O + 0.1% HCOOH |
| | B = MeCN |
| *Gradient:* | 0-1 min 1% B, 1-8 min 1-99% B, 8-10 min 99% B |
| *Flow:* | 50 mL/min |
| *Temperature:* | RT |
| *Solution:* | Max. 250 mg / max. 2.5 mL DMSO or DMF |
| *Injektion:* | 1 x 2.5 mL |
| *Detection:* | DAD scan range 210-400 nm |
| | MS ESI+, ESI-, scan range 160-1000 m/z |

¹H-NMR (400 MHz, DMSO-*d₆*): d [ppm] = 10.25 (s, 1H), 8.77 (d, 1H), 7.90 (ddd, 1H), 7.81 (trd, 1H), 7.73 (dtr, 1H), 7.56 (s, 1H), 6.85 (d, 1H), 4.47 (s, 2H), 2.94 (s, 3H).

### Example 12:

### 5-fluoro-N-{3-fluoro-5-[(methylsulfonyl)methyl]phenyl}-4-(2,3,4-trifluorophenyl)pyrimidin-2-amine

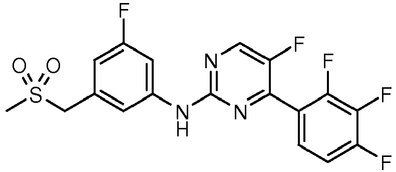

### Preparation of Intermediate 12.1:

### 2-Chloro-5-fluoro-4-(2,3,4-trifluorophenyl)pyrimidine

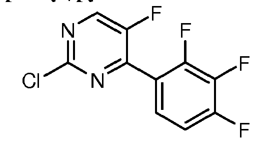

Under an atmosphere of argon, a mixture of 2,4-dichloro-5-fluoropyrimidine (1.31 g; 7.6 mmol, Aldrich Chemical Company Inc.), (2,3,4-trifluorophenyl)boronic acid (1.5 g; 8.35 mmol; Aldrich Chemical Company Inc.) and [1,1'-bis(diphenylphosphino)ferrocene]dichloropalladium(II) (620 mg; 0.76 mmol; Aldrich Chemical Company Inc.) in a 2M solution of potassium carbonate (11.39 mL) and 1,2-dimethoxyethane (39.5 mL) was stirred for 90 minutes at ambient temperature. The batch was diluted with ethyl acetate and washed with diluted aqueous sodium chloride solution. The organic layer was dried over sodium sulphate and concentrated. The residue was purified by column chromatography on silica gel (hexane / ethyl acetate) to give the desired product (1.73 g; 6.39 mmol).
¹H-NMR (400 MHz, DMSO-*d*₆): δ [ppm] = 9.08 (d, 1H), 7.68-7.53 (m, 2H).

### Preparation of end product:

The end product was prepared under similar conditions as described in the preparation of example 9 using 3-fluoro-5-[(methylsulfonyl)methyl]aniline (40.4 mg; 0.18 mmol) and 2-chloro-5-fluoro-4-(2,3,4-trifluorophenyl)pyrimidine (65 mg; 0.24 mmol). Preparative HPLC gave the desired product (47 mg; 0.11 mmol).

| | |
|---|---|
| *System:* | Waters Autopurificationsystem: Pump 2545, Sample Manager 2767, CFO, DAD 2996, ELSD 2424, SQD 3001 |
| *Column:* | XBrigde C18 5µm 100x30 mm |
| *Sol vent:* | A = H₂O + 0.1% HCOOH |
| | B = MeCN |
| *Gradient:* | 0-1 min 1% B, 1-8 min 1-99% B, 8-10 min 99% B |
| *Flow:* | 50 mL/min |
| *Temperature:* | RT |
| *Solution:* | Max. 250 mg / max. 2.5 mL DMSO or DMF |
| *Injektion:* | 1 x 2.5 mL |
| *Detection:* | DAD scan range 210-400 nm |
| | MS ESI+, ESI-, scan range 160-1000 m/z |

¹H-NMR (400 MHz, DMSO-*d*₆): δ [ppm] = 10.26 (s, 1H), 8.78 (d, 1H), 7.74 (dtr, 1H), 7.70-7.62 (m, 1H), 7.61-7.51 (m, 2H), 6.84 (d, 1H), 4.46 (s, 2H), 2.94 (s, 3H).

### Example 13:

### 4-(2,4-Difluorophenyl)-5-fluoro-N-{3-fluoro-5-[(propylsulfonyl)methyl]phenyl}pyrimidin-2-amine

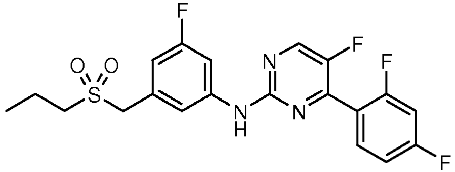

### Preparation of Intermediate 13.1:

### 1-Fluoro-3-nitro-5-[(propylsulfanyl)methyl]benzene

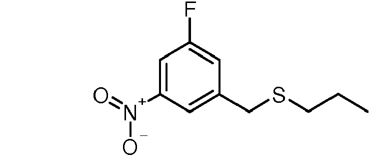

Intermediate 13.1 was prepared under similar conditions as described in the preparation of intermediate 8.1 using propane-1-thiol (ACROS) and 1-(chlormethyl)-3-fluor-5-nitrobenzol (HE Chemical Co., Ltd.). ¹H-NMR (400 MHz, DMSO-*d*₆): δ [ppm] = 8.09 (tr, 1H), 7.98 (dtr, 1H), 7.71 (dtr, 1H), 3.89 (s, 2H), 2.40 (tr, 2H), 1.51 (sxt, 2H), 0.89 (tr, 3H).

### Preparation of Intermediate 13.2:

### 1-Fluoro-3-nitro-5-[(propylsulfonyl)methyl]benzene

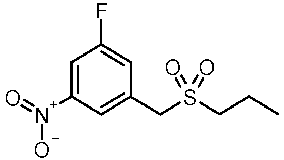

Intermediate 13.2 was prepared under similar conditions as described in the preparation of Intermediate 7.2. using 1-fluoro-3-nitro-5-[(propylsulfanyl)methyl]benzene and *meta*-chloroperbenzoic acid. ¹H-NMR (400 MHz, DMSO-*d*₆): δ [ppm] = 8.21-8.13 (m, 2H), 7.79-7.73 (m, 1H), 4.72 (s, 2H), 3.12-3.06 (m, 2H), 1.79-1.67 (m, 2H), 0.98 (tr, 3H).

### Preparation of Intermediate 13.3

### 3-Fluoro-5-[(propylsulfonyl)methyl]aniline

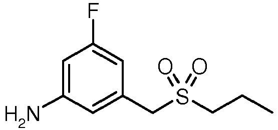

Intermediate 13.3. was prepared under similar conditions as described in the preparation of Intermediate 7.3. using 1-fluoro-3-nitro-5-[(propylsulfonyl)methyl]benzene and titanium(III)chloride solution (approx. 15%) in approx. 10% hydrochloric acid (purchased from Merck Schuchardt OHG).
¹H-NMR (400 MHz, DMSO-*d*₆): δ [ppm] = 6.40 (tr, 1H), 6.35-6.27 (m, 2H), 5.52 (s, 2H), 4.27 (s, 2H), 3.02-2.94 (m, 2H), 1.75-1.64 (m, 2H), 0.97 (tr, 3H).

### Preparation of end product:

The end product was prepared under similar conditions as described in the preparation of example 9 using 3-fluoro-5-[(propylsulfonyl)methyl]aniline (40 mg; 0.16 mmol) and 2-chloro-4-(2,4-difluorophenyl)-5-fluoropyrimidine (55 mg; 0.21 mmol). Preparative HPLC gave the desired product (41 mg; 0.09 mmol).

| | |
|---|---|
| *System:* | Waters Autopurificationsystem: Pump 2545, Sample Manager 2767, CFO, DAD 2996, ELSD 2424, SQD 3001 |
| *Column:* | XBrigde C18 5µm 100x30 mm |
| *Sol vent:* | A = H₂O + 0.1% HCOOH |
| | B = MeCN |
| *Gradient:* | 0-1 min 1% B, 1-8 min 1-99% B, 8-10 min 99% B |
| *Flow:* | 50 mL/min |
| *Temperature:* | RT |
| *Solution:* | Max. 250 mg / max. 2.5 mL DMSO or DMF |
| *Injektion:* | 1 x 2.5 mL |
| *Detection:* | DAD scan range 210-400 nm |
| | MS ESI+, ESI-, scan range 160-1000 m/z |

¹H-NMR (400 MHz, DMSO-*d*₆): δ [ppm] = 10.23 (s, 1H), 8.73 (d, 1H), 7.85 (trd, 1H), 7.76 (dtr, 1H), 7.55 (tr, 1H), 7.50 (ddd, 1H), 7.37-7.30 (m, 1H), 6.86-6.80 (m, 1H), 4.43 (s, 2H), 3.06-2.99 (m, 2H), 1.77-1.65 (m, 2H), 0.96 (tr, 3H).

### Example 14:

### 5-Fluoro-N-{3-fluoro-5-[(propylsulfonyl)methyl]phenyl}-4-(2,4,5-trifluorophenyl)pyrimidin-2-amine

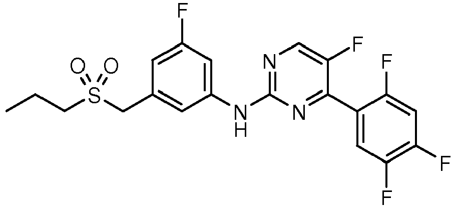

Example 14 was prepared under similar conditions as described in the preparation of example 9 using 3-fluoro-5-[(propylsulfonyl)methyl]aniline (40 mg; 0.16 mmol) and 2-chloro-5-fluoro-4-(2,4,5-trifluorophenyl)pyrimidine (57 mg; 0.21 mmol). Preparative HPLC gave the desired product (51 mg; 0.11 mmol).

| | |
|---|---|
| *System:* | Waters Autopurificationsystem: Pump 2545, Sample Manager 2767, CFO, DAD 2996, ELSD 2424, SQD 3001 |
| *Column:* | XBrigde C18 5µm 100x30 mm |
| *Sol vent:* | A = H₂O + 0.1% HCOOH |
| | B = MeCN |
| *Gradient:* | 0-1 min 1% B, 1-8 min 1-99% B, 8-10 min 99% B |
| *Flow:* | 50 mL/min |
| *Temperature:* | RT |
| *Solution:* | Max. 250 mg / max. 2.5 mL DMSO or DMF |
| *Injektion:* | 1 x 2.5 mL |
| *Detection:* | DAD scan range 210-400 nm |
| | MS ESI+, ESI-, scan range 160-1000 m/z |

¹H-NMR (400 MHz, DMSO-*d*₆): δ [ppm] = 10.25 (s, 1H), 8.76 (d, 1H), 7.91 (ddd, 1H), 7.81 (trd, 1H), 7.73 (dtr, 1H), 7.57 (s, 1H), 6.87-6.79 (m, 1H), 4.44 (s, 2H), 3.07-2.98 (m, 2H), 1.77-1.65 (m, 2H), 0.96 (tr, 3H).

The following Table 1 provides an overview on the compounds described in the example section:

**Table 1**

| **Example No**. | **Structure** | **Name of compound** |
|---|---|---|
| **1** | 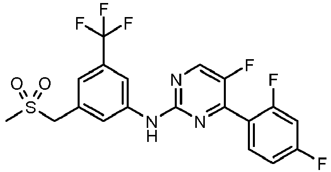 | 4-(2,4-Difluorophenyl)-5-fluoro-N-{3-[(methylsulfonyl)methyl]-5-(trifluoromethyl)phenyl}pyrimidin-2-amine |
| **2** | 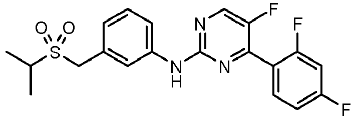 | 4-(2,4-Difluorophenyl)-5-fluoro-N-{3-[(propan-2-ylsulfonyl)methyl]phenyl}pyrimidin-2-amine |
| **3** | 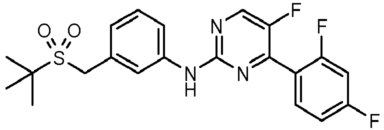 | N- {3-[(tert-Butylsulfonyl)methyl]phenyl}-4-(2,4-difluorophenyl)-5-fluoropyrimidin-2-amine |
| **4** | 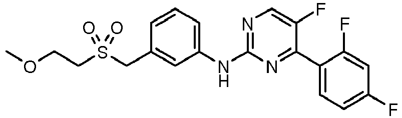 | 4-(2,4-Difluorophenyl)-5-fluoro-N-(3-{[(2-methoxyethyl)sulfonyl] methyl }phenyl)pyri midin-2-amine |
| **5** | 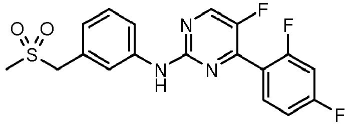 | 4-(2,4-Difluorophenyl)-5-fluoro-N-{3-[(methylsulfonyl)methyl]phenyl }pyrimidin-2-amine |
| **6** | 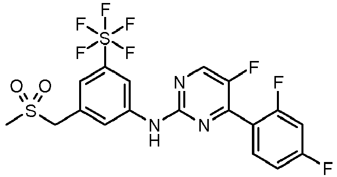 | 4-(2,4-Difluorophenyl)-5-fluoro-N-{3-[(methylsulfonyl)methyl]-5-(pentafluoro-λ⁶-sulfanyl)phenyl}pyrimidin-2-amine |
| **7** | 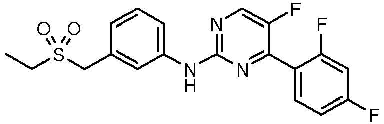 | 4-(2,4-Difluorophenyl)-N-{3-[(ethylsulfonyl)methyl]phenyl}-5-fluoropyrimidin-2-amine |
| **8** | 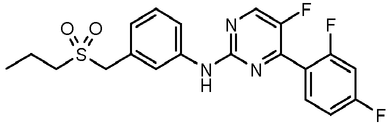 | 4-(2,4-Difluorophenyl)-5-fluoro-N-{3-[(propylsulfonyl)methyl]phenyl }pyrimidin-2-amine |
| **9** | 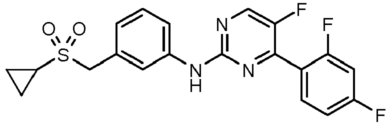 | N-{3-[(Cyclopropylsulfonyl)methyl]phenyl} -4-(2,4-difluorophenyl)-5-fluoropyrimidin-2-amine |
| **10** | 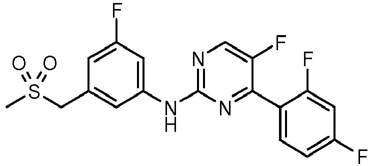 | 4-(2,4-Difluorophenyl)-5-fluoro-N-{3-fluoro-5-[(methylsulfonyl)methyl]phenyl}pyrimidin-2-amine |
| **11** | 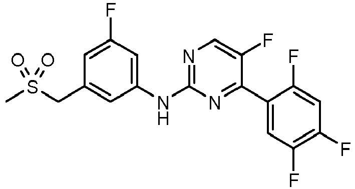 | 5-Fluoro-N-{3-fluoro-5-[(methylsulfonyl)methyl]phenyl }-4-(2,4,5-trifluorophenyl)pyrimidin-2-amine |
| **12** | 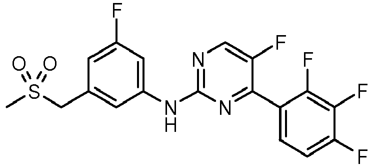 | 5-Fluoro-N-{3-fluoro-5-[(methylsulfonyl)methyl]phenyl -4-(2,3,4-trifluorophenyl)pyrimidin-2-amine |
| **13** | 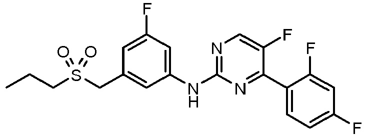 | 4-(2,4-Difluorophenyl)-5-fluoro-N-{3-fluoro-5-[(propylsulfonyl)methyl]phenyl}pyrimidin-2-amine |
| **14** | 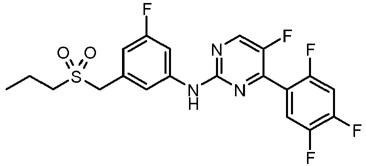 | 5-Fluoro-N-{3-fluoro-5-[(propylsulfonyl)methyl]phenyl }-4-(2,4,5-trifluorophenyl)pyrimidin-2-amine |

### Results:

**Table 2: Inhibition for CDK9 and CDK2 of compounds according to the present invention The IC₅₀ (inhibitory concentration at 50% of maximal effect) values are indicated in nM, "n.t." means that the compounds have not been tested in this assay.**

| | | | | | |
|---|---|---|---|---|---|
| ①: | Example Number | | | | |
| ②: | CDK9: CDK9/CycT1 kinase assay as described under Method 1a. of Materials and Methods | | | | |
| ③: | CDK2: CDK2/CycE kinase assay as described under Method 2. of Materials and Methods | | | | |
| ④: | high ATP CDK9: CDK9/CycT1 kinase assay as described under Method 1b. of Materials and Methods | | | | |
| ⑤: | Selectivity CDK9 / CDK2 as derived from Methods 1a. and 2. of Materials and Methods | | | | |

| ① | **Structure of compound** | ② | ③ | ④ | ⑤ |
|---|---|---|---|---|---|
| **1** | 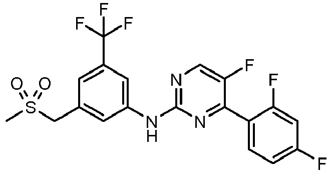 | 20 | 20000 | n.t. | 1000 |
| **2** | 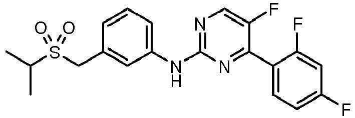 | 50 | 1200 | 2260 | 24 |
| **3** | 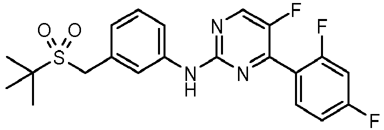 | 110 | 1000 | 14500 | 10 |
| **4** | 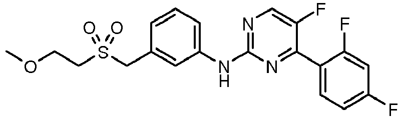 | 130 | 2600 | 2260 | 20 |
| **5** | 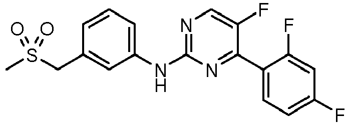 | 51 | 470 | 858 | 9.1 |
| **6** | 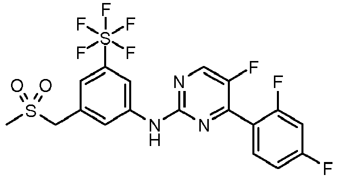 | 14 | 1760 | 99 | 122 |
| **7** | 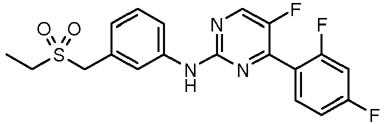 | 40 | 360 | n.t. | 8.9 |
| **8** | 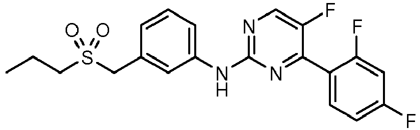 | 39 | 570 | 904 | 15 |
| **9** | 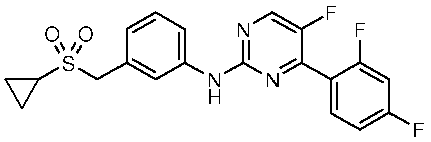 | 13 | 410 | 330 | 32 |
| **10** | 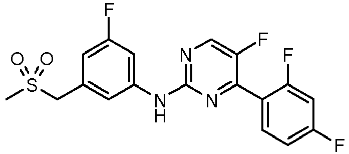 | 130 | 5800 | 2280 | 28 |
| **11** | 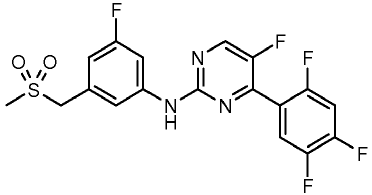 | 74 | 20000 | 20000 | 270 |
| **12** | 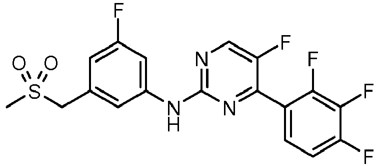 | 25 | 350 | 271 | 14 |
| **13** | 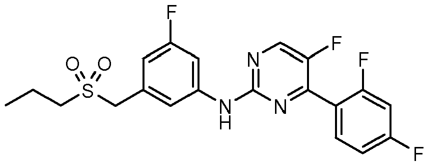 | 13 | 580 | 2280 | 44 |
| **14** | 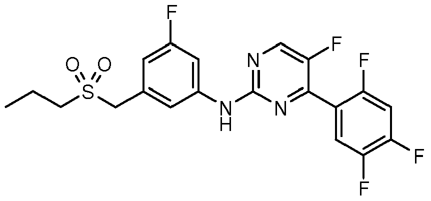 | n.t. | 20000 | 20000 | n.t. |

**Table 3: Inhibition of proliferation of HeLa, HeLa-MaTu-ADR, A2780, NCI-H460, DU145, Caco-2 and B16F10 cells by compounds according to the present invention, determined as described above (Method 3. of Materials and Methods section). All IC₅₀ (inhibitory concentration at 50% of maximal effect) values are indicated in nM, "n.t." means that the compounds have not been tested in this assay.**

| | | | | | | | | |
|---|---|---|---|---|---|---|---|---|
| ①: | Example Number | | | | | | | |
| ②: | Inhibition of HeLa cell proliferation | | | | | | | |
| ③: | Inhibition of HeLa-MaTu-ADR cell proliferation | | | | | | | |
| ④: | Inhibition of H460 cell proliferation | | | | | | | |
| ⑤: | Inhibition of DU 145 cell proliferation | | | | | | | |
| ⑥: | Inhibition of Caco-2 cell proliferation | | | | | | | |
| ⑦: | Inhibition of B16F10 cell proliferation | | | | | | | |
| ⑧: | Inhibition of A2780 cell proliferation | | | | | | | |

| ① | Structure of compounds | ② | ③ | ④ | ⑤ | ⑥ | ⑦ | ⑧ |
|---|---|---|---|---|---|---|---|---|
| **1** | 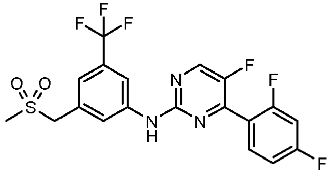 | 1050 | n.t. | n.t. | n.t. | n.t. | n.t. | n.t. |
| **2** | 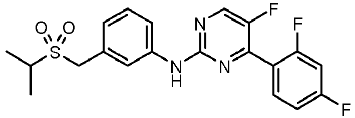 | 3000 | n.t. | n.t. | n.t. | n.t. | n.t. | n.t. |
| **3** | 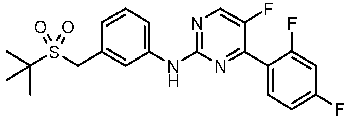 | 3000 | n.t. | n.t. | n.t. | n.t. | n.t. | n.t. |
| **6** | 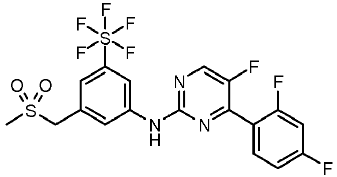 | 617 | 220 | 386 | 352 | 382 | 951 | n.t. |
| **7** | 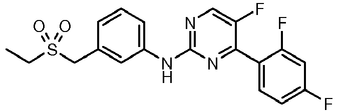 | 2240 | n.t. | n.t. | n.t. | n.t. | n.t. | n.t. |
| **8** | 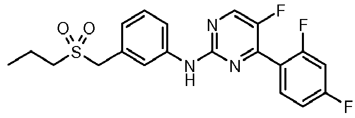 | 3000 | n.t. | n.t. | n.t. | n.t. | n.t. | n.t. |
| **9** | 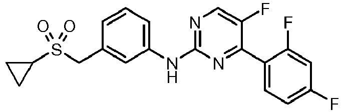 | 3000 | n.t. | n.t. | n.t. | n.t. | n.t. | n.t. |
| **10** | 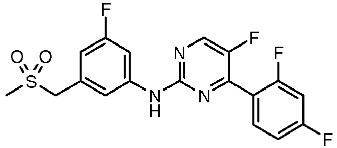 | 3000 | n.t. | n.t. | n.t. | n.t. | n.t. | n.t. |
| **11** | 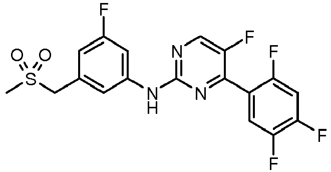 | 2960 | n.t. | n.t. | n.t. | n.t. | n.t. | n.t. |
| **12** | 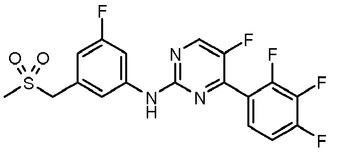 | 1000 | n.t. | n.t. | n.t. | n.t. | n.t. | n.t. |
| **13** | 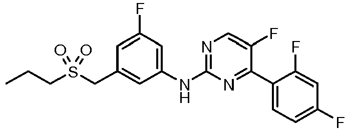 | 3000 | n.t. | n.t. | n.t. | n.t. | n.t. | n.t. |
| **14** | 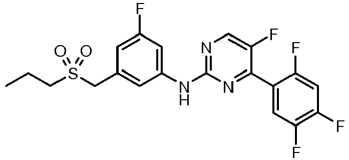 | 3000 | n.t. | n.t. | n.t. | n.t. | n.t. | n.t. |

## Claims

1. A compound of general formula (I) 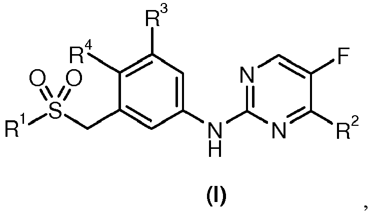 wherein
R¹ represents a group selected from C₁-C₆-alkyl-, C₃-C₇-cycloalkyl-, heterocyclyl-, phenyl, heteroaryl, phenyl-C₁-C₃-alkyl- or heteroaryl-C₁-C₃-alkyl-,
wherein said group is optionally substituted with one or two or three substituents, identically or differently, selected from the group of hydroxy, cyano, halogen, halo-C₁-C₃-alkyl-, C₁-C₆-alkoxy-, C₁-C₃-fluoroalkoxy-, amino, alkylamino-, dialkylamino-, acetylamino-, N-methyl-N-acetylamino-, cyclic amines, -OP(O)(OH)₂, -C(O)OH, -C(O)NH₂;
R² represents a group 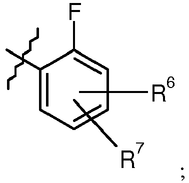
R³ , R⁴ represent, independently from each other, a group selected from a hydrogen atom, halogen atom, cyano, SF₅, C₁-C₃-alkyl-, C₁-C₃-alkoxy-, hydroxy, halo-C₁-C₃-alkyl-, C₁-C₃-fluoroalkoxy-;
R⁶, R⁷ represent, independently from each other, a group selected from a hydrogen atom, fluoro atom, chloro atom, C₁-C₃-alkyl-, C₁-C₃-alkoxy-, halo-C₁-C₃-alkyl-, C₁-C₃-fluoroalkoxy-;
or its salts, solvates or salts of solvates,
with the proviso that the compound is not 4-(2,4-Difluorophenyl)-5-fluoro-N-{3-[(methylsulfonyl)methyl]phenyl}pyrinudin-2-amine.

2. The compound of general formula (I) according to claim 1, wherein
R¹ represents a group selected from C₁-C₆-alkyl-, C₃-C₇-cycloalkyl-, heterocyclyl-, phenyl, heteroaryl, phenyl-C₁-C₃-alkyl- or heteroaryl-C₁-C₃-alkyl-,
wherein said group is optionally substituted with one or two or three substituents, identically or differently, selected from the group of hydroxy, cyano, halogen, halo-C₁-C₃-alkyl-, C₁-C₆-alkoxy-, C₁-C₃-fluoroalkoxy-, amino, alkylamino-, dialkylamino-, acetylamino-, N-methyl-N-acetylamino-, cyclic amines, -OP(O)(OH)₂,-C(O)OH, -C(O)NH₂;
R² represents a group 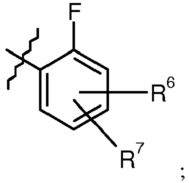
R³ represents a group selected from a halogen atom, cyano, -SF₅, C₁-C₃-alkyl-, C₁-C₃-alkoxy-, hydroxy, halo-C₁-C₃-alkyl-, C₁-C₃-fluoroalkoxy-;
R⁴ represents a group selected from a hydrogen atom, halogen atom, cyano, -SF₅, C₁-C₃-alkyl-, C₁-C₃-alkoxy-, hydroxy, halo-C₁-C₃-alkyl-, C₁-C₃-fluoroalkoxy-;
R⁶, R⁷ represent, independently from each other, a group selected from a hydrogen atom, fluoro atom, chloro atom, C₁-C₃-alkyl-, C₁-C₃-alkoxy-, halo-C₁-C₃-alkyl-, C₁-C₃-fluoroalkoxy-;
or its salts, solvates or salts of solvates.

3. The compound of general formula (I) according to claim 1, 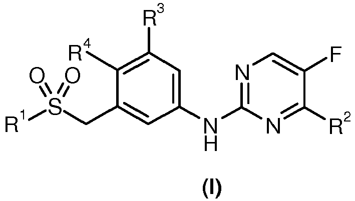 wherein
R¹ represents a group selected from C₁-C₆-alkyl-, C₃-C₇-cycloalkyl-, heterocyclyl-, phenyl, heteroaryl, phenyl-C₁-C₃-alkyl- or heteroaryl-C₁-C₃-alkyl-,
wherein said group is optionally substituted with one or two or three substituents, identically or differently, selected from the group of hydroxy, cyano, halogen, halo-C₁-C₃-alkyl-, C₁-C₆-alkoxy-, C₁-C₃-fluoroalkoxy-, amino, alkylamino-, dialkylamino-, acetylamino-, N-methyl-N-acetylamino-, cyclic amines, -OP(O)(OH)₂,-C(O)OH, -C(O)NH₂;
R² represents a group 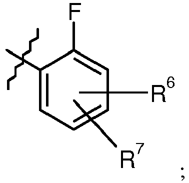
R³ represents a trifluoromethyl group;
R⁴ represents a group selected from a hydrogen atom, halogen atom, cyano, SF₅, C₁-C₃-alkyl-, C₁-C₃-alkoxy-, hydroxy, halo-C₁-C₃-alkyl-, C₁-C₃-fluoroalkoxy-,
R⁶, R⁷ represent, independently from each other, a group selected from a hydrogen atom, fluoro atom, chloro atom, C₁-C₃-alkyl-, C₁-C₃-alkoxy-, halo-C₁-C₃-alkyl-, C₁-C₃-fluoroalkoxy-;
or its salts, solvates or salts of solvates.

4. The compound of general formula (I) according to claim 1, wherein
R¹ represents a group selected from C₁-C₆-alkyl- or C₃-C₅-cycloalkyl,
wherein said group is optionally substituted with one substituent selected from the group of amino, C₁-C₃-alkoxy, hydroxy, -OP(O)(OH)₂;
R² represents a group 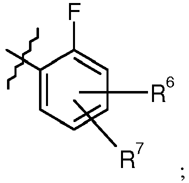 R³ represents a group selected from a halogen atom, -SF₅ or halo-C₁-C₃-alkyl-;
R⁴ represents a hydrogen atom or a fluoro atom;
R⁶, R⁷ represent, independently from each other, a group selected from a hydrogen atom, fluoro atom, or its salts, solvates or salts of solvates.

5. The compound of general formula (I) according to any one of claim 1, 2 or 4, wherein
R³ represents -SF₅, and
R⁴ represents a hydrogen atom;
or its salts, solvates or salts of solvates.

6. The compound of general formula (I) according to any one of claim 1 to 5, wherein
R² represents the group 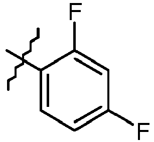
or its salts, solvates or salts of solvates.

7. The compound of general formula (I) according to claim 1, wherein
R¹ represents a group selected from methyl, ethyl, *n*-propyl, *iso*-propyl, *tert-butyl,* cyclopropyl or 2-methoxyethyl;
R² represents a group selected from 2,4-difluorophenyl, 2,3,4-trifluorophenyl or 2,4,5-trifluorophenyl;
R³ represents a fluoro atom, -SF₅ or a trifluoromethyl group; and
R⁴ represents a hydrogen atom;
or its salts, solvates or salts of solvates.

8. The compound according to claim 1, which is selected from
• 4-(2,4-Difluorophenyl)-5-fluoro-N-{3-[(methylsulfonyl)methyl]-5-(trifluoromethyl)phenyl}pyrimidin-2-amine
• 4-(2,4-Difluorophenyl)-5-fluoro-N-{3-[(propan-2-ylsulfonyl)methyl]phenyl}pyrimidin-2-amine
• N-{3-[(tert-Butylsulfonyl)methyl]phenyl}-4-(2,4-difluorophenyl)-5-fluoropyrimidin-2-amine
• 4-(2,4-Difluorophenyl)-5-fluoro-N-(3-{[(2-methoxyethyl)sulfonyl]methyl}phenyl)pyrimidin-2-amine
• 4-(2,4-Difluorophenyl)-5-fluoro-N-{3-[(methylsulfonyl)methyl]phenyl}pyrimidin-2-amine
• 4-(2,4-Difluorophenyl)-5-fluoro-N-{3-[(methylsulfonyl)methyl]-5-(pentafluoro-λ⁶-sulfanyl)phenyl}pyrimidin-2-amine
• 4-(2,4-Difluorophenyl)-N-{3-[(ethylsulfonyl)methyl]phenyl}-5-fluoropyrimidin-2-amine
• 4-(2,4-Difluorophenyl)-5-fluoro-N-{3-[(propylsulfonyl)methyl]phenyl}pyrimidin-2-amine
• N-{3-[(Cyclopropylsulfonyl)methyl]phenyl}-4-(2,4-difluorophenyl)-5-fluoropyrimidin-2-amine
• 4-(2,4-Difluorophenyl)-5-fluoro-N-{3-fluoro-5-[(methylsulfonyl)methyl]phenyl}pyrimidin-2-amine
• 5-Fluoro-N-{3-fluoro-5-[(methylsulfonyl)methyl]phenyl} -4-(2,4,5 -trifluorophenyl)pyrimidin-2-amine
• 5-Fluoro-N-{3-fluoro-5-[(methylsulfonyl)methyl]phenyl}-4-(2,3,4-trifluorophenyl)pyrimidin-2-amine
• 4-(2,4-Difluorophenyl)-5-fluoro-N-{3-fluoro-5-[(propylsulfonyl)methyl]phenyl}pyrimidin-2-
• 5-Fluoro-N-{3-fluoro-5-[(propylsulfonyl)methyl]phenyl}-4-(2,4,5-trifluorophenyl)pyrimidin-2- or its salts, solvates or salts of solvates.

9. A compound of general formula (I) according to any one of claims 1 to 8 for use in the treatment and/or
prophylaxis of hyper-proliferative disorders, virally induced infectious diseases and/or of cardiovascular diseases.

10. A compound of general formula (I) according to any one of claims 1 to 8 for use in the treatment and/or
prophylaxis of lung carcinomas, prostate carcinomas, cervical carcinomas, colorectal carcinomas, melanomas or ovarian carcinomas.

11. A pharmaceutical combination comprising a compound according to any one of claims 1 to 8 in combination with at least one or more further active ingredients.

12. A pharmaceutical composition comprising a compound according to any one of claims 1 to 8 in combination with an inert, nontoxic, pharmaceutically suitable adjuvant.

13. The pharmaceutical combination according to claim 11 for use in the treatment and/or prophylaxis of hyper-proliferative disorders, virally induced infectious diseases and/or of cardiovascular diseases.

14. The pharmaceutical composition according to claim 12 for use in the treatment and/or prophylaxis of hyper-proliferative disorders, virally induced infectious diseases and/or of cardiovascular diseases.

15. A compound of of general formula (5) 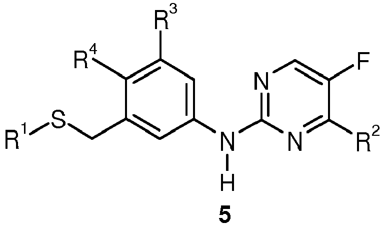 wherein R¹, R², R³ and R⁴ are as defined for the compounds of general formula **(I)** according to any one of claims 1 to 7.

16. A method for the preparation of the compounds of formula (I) according to any one of claims 1 to 8, in which method a compound of formula (5) 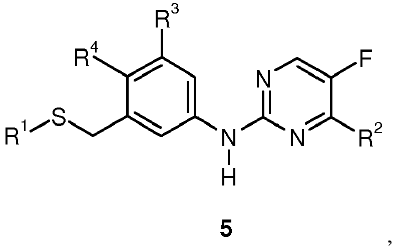
in which R¹, R², R³ and R⁴ are as defined according to any one of claims 1 to 7 for the compounds of general formula (I), is oxidized with an alkali salt of permanganic acid in an aliphatic ketone of the formula C₁-C₂-alkyl-C(=O)-C₁-C₂-alkyl as a solvent, thus providing a compound of general formula (I) according to any one of claims 1 to 8,
and in which method the resulting compound of formula (I) is optionally, if appropriate, reacted with the corresponding (i) solvents and/or (ii) bases or acids to the solvates, salts and/or solvates of the salts of the compounds of formula (I).

17. A method for the preparation of the compounds of formula (5) in which R¹, R², R³ and R⁴ are as defined according to any one of claims 1 to 7 for the compounds of general formula (I),
in which method a compound of formula (3) 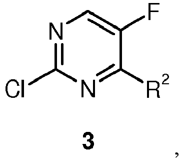 in which R² is as defined for the compounds of general formula (I) in any one of claims 1 to 7, is reacted with a compound of formula (4) 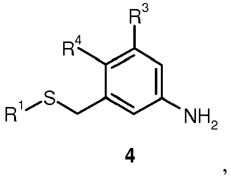 in which R¹, R³ and R⁴ are as defined for the compounds of general formula (I) in any one of claims 1 to 7, in the presence of a a strong inorganic or organic acid and in an aliphatic alcohol, an ether, or *N,N*-dimethylformamide as a solvent, or in a mixture of such solvents, thus providing a compound of general formula (5) 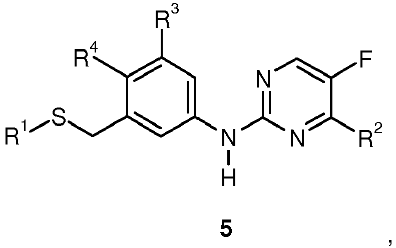 in which R¹, R², R³ and R⁴ are as defined according to any one of claims 1 to 7 for the compounds of general formula **(I).**

18. A method for the preparation of the compounds of formula **(I)** according to any one of claims 1 to 8, in which method a compound of formula **(3)** 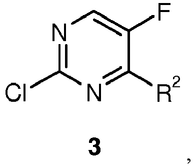 in which R² is as defined for the compounds of general formula **(I)** in any one of claims 1 to 7, is reacted with a compound of formula **(6)** 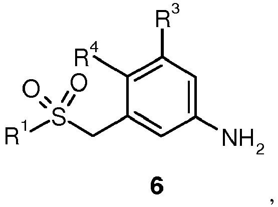
in which R¹, R³ and R⁴ are as defined for the compounds of general formula **(I)** in any one of claims 1 to 7, in the presence of a strong inorganic or organic acid and in an aliphatic alcohol, an ether, or *N,N*-dimethylformamide as a solvent, or in a mixture of such solvents, thus providing a compound of general formula **(I)** according to any one of claims 1 to 8,
and in which method the resulting compound of formula **(I)** is optionally, if appropriate, reacted with the corresponding (i) solvents and/or (ii) bases or acids to the solvates, salts and/or solvates of the salts of the compounds of formula **(I).**

## Patentansprüche

1. Verbindung der allgemeinen Formel (I) 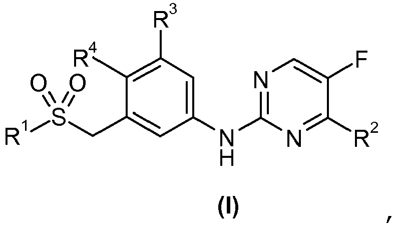 wobei
R¹ für eine aus C₁-C₆-Alkyl-, C₃-C₇-Cycloalkyl-, Heterocyclyl-, Phenyl, Heteroaryl, Phenyl-C₁-C₃ alkyl- und Heteroaryl-C₁-C₃-alkyl- ausgewählte Gruppe steht,
wobei die Gruppe gegebenenfalls durch einen oder zwei oder drei gleiche oder verschiedene Substituenten ausgewählt aus der Gruppe bestehend aus Hydroxy, Cyano, Halogen, Halogen-C₁-C₃-alkyl-, C₁-C₆-Alkoxy-, C₁-C₃-Fluoralkoxy-, Amino, Alkylamino-, Dialkylamino-, Acetylamino-, N-Methyl-N-acetylamino-, cyclischen Aminen, -OP(O)(OH)₂, -C(O)OH und -C(O)NH₂ substituiert ist,
R² für eine Gruppe 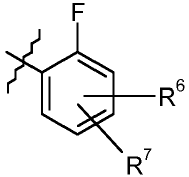
steht,
R³, R⁴ unabhängig voneinander für eine aus einem Wasserstoffatom, Halogenatom, Cyano, SF₅, C₁-C₃-Alkyl-, C₁-C₃ Alkoxy-, Hydroxy, Halogen-C₁-C₃-alkyl-und C₁-C₃-Fluoralkoxy- ausgewählte Gruppe stehen,
R⁶, R⁷ unabhängig voneinander für eine aus einem Wasserstoffatom, Fluoratom, Chloratom, C₁-C₃-Alkyl-, C₁-C₃-Alkoxy-, Halogen-C₁-C₃-alkyl- und C₁-C₃-Fluoroalkoxy-ausgewählte Gruppe stehen,
oder deren Salze, Solvate oder Salze von Solvaten,
mit der Maßgabe, dass es sich bei der Verbindung nicht um 4-(2,4-Difluorphenyl)-5-fluor-N-{3-[(methylsulfonyl)-methyl]phenyl}pyrimidin-2-amin handelt.

2. Verbindung der allgemeinen Formel (I) nach Anspruch 1, wobei
R¹ für eine aus C₁-C₆-Alkyl-, C₃-C₇-Cycloalkyl-, Heterocyclyl-, Phenyl, Heteroaryl, Phenyl-C₁-C₃-alkyl- und Heteroaryl-C₁-C₃-alkyl- ausgewählte Gruppe steht,
wobei die Gruppe gegebenenfalls durch einen oder zwei oder drei gleiche oder verschiedene Substituenten ausgewählt aus der Gruppe bestehend aus Hydroxy, Cyano, Halogen, Halogen-C₁-C₃-alkyl-, C₁-C₆-Alkoxy-, C₁-C₃-Fluoralkoxy-, Amino, Alkylamino-, Dialkylamino-, Acetylamino-, N-Methyl-N-acetylamino-, cyclischen Aminen, -OP(O)(OH)₂, -C(O)OH und -C(O)NH₂ substituiert ist,
R² für eine Gruppe 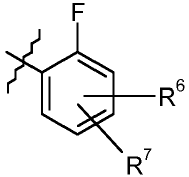
steht,
R³ für eine aus einem Halogenatom, Cyano, -sF₅, C₁-C₃-Alkyl-, C₁-C₃-Alkoxy-, Hydroxy, Halogen-C₁-C₃-alkyl-und C₁-C₃-Fluoralkoxy- ausgewählte Gruppe steht
R⁴ für eine aus einem Wasserstoffatom, Halogenatom, Cyano, -SF₅, C₁-C₃-Alkyl-, C₁-C₃-Alkoxy-, Hydroxy, Halogen-C₁-C₃-alkyl- und C₁-C₃-Fluoralkoxy-ausgewählte Gruppe steht,
R⁶, R⁷ unabhängig voneinander für eine aus einem Wasserstoffatom, Fluoratom, Chloratom, C₁-C₃-Alkyl-, C₁-C₃-Alkoxy-, Halogen-C₁-C₃-alkyl- und C₁-C₃-Fluoroalkoxy- ausgewählte Gruppe stehen,
oder deren Salze, Solvate oder Salze von Solvaten.

3. Verbindung der allgemeinen Formel (I) nach Anspruch 1, 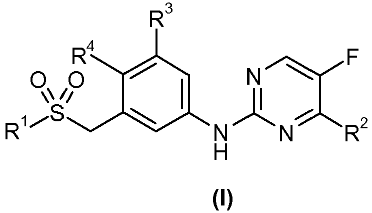 wobei
R¹ für eine aus C₁-C₆-Alkyl-, C₃-C₇-Cycloalkyl-, Heterocyclyl-, Phenyl, Heteroaryl, Phenyl-C₁-C₃-alkyl- und Heteroaryl-C₁-C₃-alkyl- ausgewählte Gruppe steht,
wobei die Gruppe gegebenenfalls durch einen oder zwei oder drei gleiche oder verschiedene Substituenten ausgewählt aus der Gruppe bestehend aus Hydroxy, Cyano, Halogen, Halogen-C₁-C₃-alkyl-, C₁-C₆-Alkoxy-, C₁-C₃-Fluoralkoxy-, Amino, Alkylamino-, Dialkylamino-, Acetylamino-, N-Methyl-N-acetylamino-, cyclischen Aminen, -OP(O)(OH)₂, -C(O)OH und -C(O)NH₂ substituiert ist,
R² für eine Gruppe 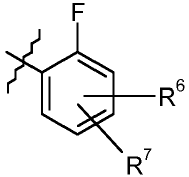
steht,
R³ für eine Trifluormethylgruppe steht,
R⁴ für eine aus einem Wasserstoffatom, Halogenatom, Cyano, SF₅, C₁-C₃-Alkyl-, C₁-C₃-Alkoxy-, Hydroxy, Halogen-C₁-C₃-alkyl- und C₁-C₃-Fluoralkoxy-ausgewählte Gruppe steht,
R⁶, R⁷ unabhängig voneinander für eine aus einem Wasserstoffatom, Fluoratom, Chloratom, C₁-C₃-Alkyl-, C₁-C₃-Alkoxy-, Halogen-C₁-C₃-alkyl- und C₁-C₃-Fluoroalkoxy- ausgewählte Gruppe stehen,
oder deren Salze, Solvate oder Salze von Solvaten.

4. Verbindung der allgemeinen Formel (I) nach Anspruch 1, wobei
R¹ für eine aus C₁-C₆-Alkyl- und C₃-C₅-Cycloalkyl ausgewählte Gruppe steht,
wobei die Gruppe gegebenenfalls durch einen Substituenten ausgewählt aus der Gruppe bestehend aus Amino, C₁-C₃-Alkoxy, Hydroxy und -OP(O)(OH)₂ substituiert ist,
R² für eine Gruppe 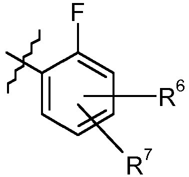
steht,
R³ für eine aus einem Halogenatom, -SF₅ und Halogen-C₁-C₃-alkyl- ausgewählte Gruppe steht,
R⁴ für ein Wasserstoffatom oder ein Fluoratom steht,
R⁶, R⁷ unabhängig voneinander für eine aus einem Wasserstoffatom und einem Fluoratom ausgewählte Gruppe stehen,
oder deren Salze, Solvate oder Salze von Solvaten.

5. Verbindung der allgemeinen Formel (I) nach einem der Ansprüche 1, 2 oder 4, wobei
R³ für -SF₅ steht und
R⁴ für ein Wasserstoffatom steht,
oder deren Salze, Solvate oder Salze von Solvaten.

6. Verbindung der allgemeinen Formel (I) nach einem der Ansprüche 1 bis 5, wobei
R² für die Gruppe 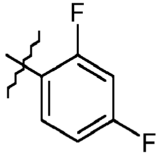
steht, oder deren Salze, Solvate oder Salze von Solvaten.

7. Verbindung der allgemeinen Formel (I) nach Anspruch 1, wobei
R¹ für eine aus Methyl, Ethyl, *n*-Propyl, Isopropyl, *tert*.-Butyl, Cyclopropyl und 2-Methoxyethyl ausgewählte Gruppe steht,
R² für eine aus 2,4-Difluorphenyl, 2,3,4-Trifluorphenyl und 2,4,5-Trifluorphenyl ausgewählte Gruppe steht,
R³ für ein Fluoratom, -SF₅ oder eine Trifluormethylgruppe steht und
R⁴ für ein Wasserstoffatom steht,
oder deren Salze, Solvate und Salze von Solvaten.

8. Verbindung nach Anspruch 1, ausgewählt aus
• 4-(2,4-Difluorphenyl)-5-fluor-N-{3-[(methylsulfonyl)methyl]-5-(trifluormethyl)phenyl}pyrimidin-2-amin
• 4-(2,4-Difluorphenyl)-5-fluor-N-{3-[(propan-2-ylsulfonyl)methyl]phenyl}pyrimidin-2-amin
• N-{3-[(tert.-Butylsulfonyl)methyl]phenyl}-4-(2,4-difluorphenyl)-5-fluorpyrimidin-2-amin
• 4-(2,4-Difluorphenyl)-5-fluor-N-(3-{[(2-methoxyethyl)sulfonyl]methyl}phenyl)pyrimidin-2-amin
• 4-(2,4-Difluorphenyl)-5-fluor-N-{3-[(methylsulfonyl)methyl]phenyl}pyrimidin-2-amin
• 4-(2,4-Difluorphenyl)-5-fluor-N-{3-[(methylsulfonyl)methyl]-5-(pentafluor-λ⁶-sulfanyl)phenyl}pyrimidin-2-amin
• 4-(2,4-Difluorphenyl)-N-{3-[(ethylsulfonyl)methyl]phenyl}-5-fluorpyrimidin-2-amin
• 4-(2,4-Difluorphenyl)-5-fluor-N-{3-[(propylsulfonyl)methyl]phenyl}pyrimidin-2-amin
• N-{3-[(Cyclopropylsulfonyl)methyl]phenyl}-4-(2,4-difluorphenyl)-5-fluorpyrimidin-2-amin
• 4-(2,4-Difluorphenyl)-5-fluor-N-{3-fluor-5-[(methylsulfonyl)methyl]phenyl}pyrimidin-2-amin
• 5-Fluor-N-{3-fluor-5-[(methylsulfonyl)methyl]phenyl}-4-(2,4,5-trifluorphenyl)pyrimidin-2-amin
• 5-Fluor-N-{3-fluor-5-[(methylsulfonyl)methyl]phenyl}-4-(2,3,4-trifluorphenyl)pyrimidin-2-amin
• 4-(2,4-Difluorphenyl)-5-fluor-N-{3-fluor-5-[(propylsulfonyl)methyl]phenyl}pyrimidin-2-amin
• 5-Fluor-N-{3-fluor-5-[(propylsulfonyl)methyl]phenyl}-4-(2,4,5-trifluorphenyl)pyrimidin-2-amin
oder deren Salze, Solvate oder Salze von Solvaten.

9. Verbindung der allgemeinen Formel (I) nach einem der Ansprüche 1 bis 8 zur Behandlung und/oder Prophylaxe von hyperproliferativen Erkrankungen, virusinduzierten Infektionskrankheiten und/oder Herz-Kreislauf-Krankheiten.

10. Verbindung der allgemeinen Formel (I) nach einem der Ansprüche 1 bis 8, zur Behandlung und/oder Prophylaxe von Lungenkarzinomen, Prostatakarzinomen, Gebärmutterhalskarzinomen, Kolorektalkarzinomen, Melanomen oder Eierstockkarzinomen.

11. Pharmazeutische Kombination, umfassend eine Verbindung nach einem der Ansprüche 1 bis 8 in Kombination mit mindestens einem oder mehreren weiteren Wirkstoffen.

12. Pharmazeutische Zusammensetzung, umfassend eine Verbindung nach einem der Ansprüche 1 bis 8 in Kombination mit einem inerten, nichttoxischen, pharmazeutisch geeigneten Adjuvans.

13. Pharmazeutische Kombination nach Anspruch 11 zur Behandlung und/oder Prophylaxe von hyperproliferativen Erkrankungen, virusinduzierten Infektionskrankheiten und/oder Herz-Kreislauf-Krankheiten.

14. Pharmazeutische Zusammensetzung nach Anspruch 12 zur Behandlung und/oder Prophylaxe von hyperproliferativen Erkrankungen, virusinduzierten Infektionskrankheiten und/oder Herz-Kreislauf-Krankheiten.

15. Verbindung der allgemeinen Formel (5) 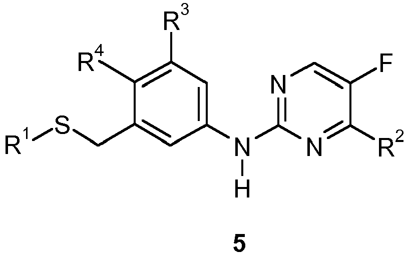 wobei R¹, R², R³ und R⁴ wie für die Verbindungen der allgemeinen Formel (I) nach einem der Ansprüche 1 bis 7 definiert sind.

16. Verfahren zur Herstellung der Verbindungen der Formel (I) nach einem der Ansprüche 1 bis 8, wobei man bei dem Verfahren eine Verbindung der Formel (5) 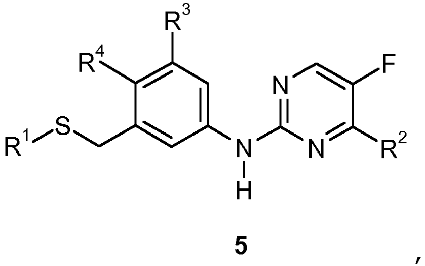 in welcher R¹, R², R³ und R⁴ wie nach einem der Ansprüche 1 bis 7 für Verbindungen der allgemeinen Formel (I) definiert sind, in einem aliphatischen Keton der Formel C₁-C₂-Alkyl-C(=O)-C₁-C₂-alkyl als Lösungsmittel mit einem Alkalisalz von Permangansäure oxidiert, unter Bereitstellung einer Verbindung der allgemeinen Formel (I) nach einem der Ansprüche 1 bis 8, und wobei man bei dem Verfahren die erhaltene Verbindung der Formel (I) gegebenenfalls, falls angebracht, mit den entsprechenden (i) Lösungsmitteln und/oder (ii) Basen oder Säuren zu den Solvaten, Salzen und/oder Solvaten der Salze der Verbindungen der Formel (I) umsetzt.

17. Verfahren zur Herstellung der Verbindungen der allgemeinen Formel (5), in welcher R¹, R², R³ und R⁴ wie nach einem der Ansprüche 1 bis 7 für die Verbindungen der allgemeinen Formel (I) definiert sind,
wobei man bei dem Verfahren eine Verbindung der Formel (3) 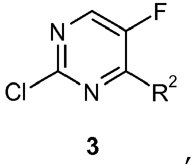 in welcher R² wie für die Verbindungen der allgemeinen Formel (I) in einem der Ansprüche 1 bis 7 definiert ist, in Gegenwart einer starken anorganischen oder organischen Säure und in einem aliphatischen Alkohol, einem Ether oder *N,N*-Dimethylformamid als Lösungsmittel oder in einer Mischung solcher Lösungsmittel mit einer Verbindung der Formel (4) 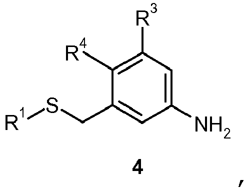 in welcher R¹, R³ und R⁴ wie für die Verbindungen der allgemeinen Formel (I) in einem der Ansprüche 1 bis 7 definiert sind, umsetzt, unter Bereitstellung einer Verbindung der allgemeinen Formel (5) 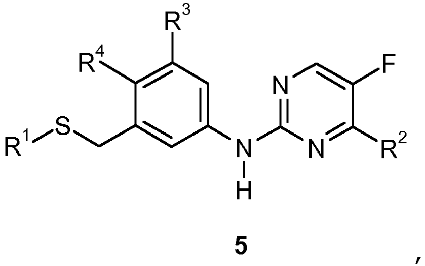 in welcher R¹, R², R³ und R⁴ wie nach einem der Ansprüche 1 bis 7 für die Verbindungen der allgemeinen Formel (1) definiert sind.

18. Verfahren zur Herstellung der Verbindungen der Formel (I) nach einem der Ansprüche 1 bis 8, wobei man bei dem Verfahren eine Verbindung der Formel (3) 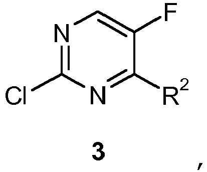 in welcher R² wie für die Verbindungen der allgemeinen Formel (I) in einem der Ansprüche 1 bis 7 definiert ist, in Gegenwart einer starken anorganischen oder organischen Säure und in einem aliphatischen Alkohol, einem Ether oder *N,N*-dimethylformamid als Lösungsmittel oder in einer Mischung solcher Lösungsmittel mit einer Verbindung der Formel (6) 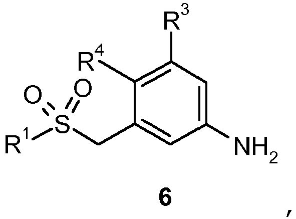 in welcher R¹, R³ und R⁴ wie für die Verbindungen der allgemeinen Formel (I) in einem der Ansprüche 1 bis 7 definiert sind, umsetzt, unter Bereitstellung einer Verbindung der allgemeinen Formel (I) nach einem der Ansprüche 1 bis 8,
und wobei man bei dem Verfahren die erhaltene Verbindung der Formel (I) gegebenenfalls, falls angebracht, mit den entsprechenden (i) Lösungsmitteln und/oder (ii) Basen oder Säuren zu den Solvaten, Salzen und/oder Solvaten der Salze der Verbindungen der Formel (I) umsetzt.

## Revendications

1. Composé de formule générale (I) 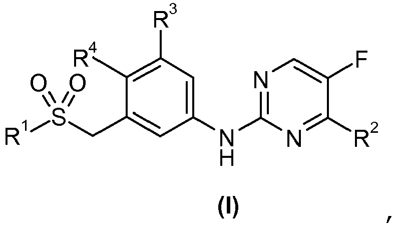 où
R¹ représente un groupement choisi parmi les groupements (alkyle en C₁-C₆,) -, (cycloalkyle en C₃-C₇)-, hétérocyclyl-, phényle, hétéroaryle, phényl-(alkyle en C₁-C₃)- ou hétéroaryl-(alkyle en C₁-C₃)-,
où ledit groupement est éventuellement substitué par un, deux ou trois substituants, identiques ou différents, choisis dans le groupe constitué par les groupements hydroxy, cyano, halogéno, halogéno-(alkyle en C₁-C₃)-, (alkoxy en C₁-C₆)-, (fluoroalkoxy en C₁-C₃)-, amino, alkylamino-, dialkylamino-, acétylamino-, N-méthyl-N-acétylamino-, amines cycliques, -OP(O)(OH)₂, -C(O)OH, - C(O)NH₂;
R² représente un groupement 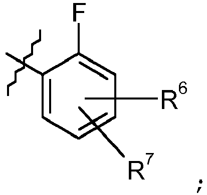
chacun des radicaux R³, R⁴ représente indépendamment de l'autre un groupement choisi parmi les atomes d'hydrogène et d'halogène et les groupements cyano, SF₅, (alkyle en C₁-C₃)-, (alkoxy en C₁-C₃)-, hydroxy, halogéno-(alkyle en C₁-C₃)-, (fluoroalkoxy en C₁-C₃)- ;
chacun des radicaux R⁶, R⁷ représente indépendamment de l'autre un groupement choisi parmi les atomes d'hydrogène, de fluor et de chlore et les groupements (alkyle en C₁-C₃)-, (alkoxy en C₁-C₃)-, halogéno-(alkyle en C₁-C₃)-, (fluoroalkoxy en C₁-C₃)- ;
ou ses sels, solvates ou sels de solvates,
à la condition que le composé ne soit pas la 4-(2,4-Difluorophényl)-5-fluoro-N-{3-[(méthylsulfonyl)méthyl]phényl}pyrimidin-2-amine.

2. Composé de formule générale (I) selon la revendication 1, où
R¹ représente un groupement choisi parmi les groupements (alkyle en C₁-C₆)-, (cycloalkyle en C₃-C₇)-, hétérocyclyl-, phényle, hétéroaryle, phényl-(alkyle en C₁-C₃)- ou hétéroaryl-(alkyle en C₁-C₃)-,
où ledit groupement est éventuellement substitué par un, deux ou trois substituants, identiques ou différents, choisis dans le groupe constitué par les groupements hydroxy, cyano, halogéno, halogéno-(alkyle en C₁-C₃)-, (alkoxy en C₁-C₆)-, (fluoroalkoxy en C₁-C₃)-, amino, alkylamino-, dialkylamino-, acétylamino-, N-méthyl-N-acétylamino-, amines cycliques, -OP(O)(OH)₂, -C(O)OH, - C(O)NH₂ ;
R² représente un groupement 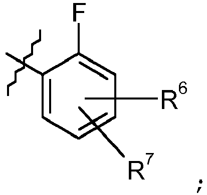 R³ représente un groupement choisi parmi les atomes d'halogène et les groupements cyano, SF₅, (alkyle en C₁-C₃)-, (alkoxy en C₁-C₃)**-,** hydroxy, halogéno-(alkyle en C₁-C₃)-, (fluoroalkoxy en C₁-C₃)- ;
R⁴ représente un groupement choisi parmi les atomes d'hydrogène et d'halogène et les groupements cyano, SF₅, (alkyle en C₁-C₃)-, (alkoxy en C₁-C₃)-, hydroxy, halogéno-(alkyle en C₁-C₃)-, (fluoroalkoxy en C₁-C₃)- ;
chacun des radicaux R⁶, R⁷ représente indépendamment de l'autre un groupement choisi parmi les atomes d'hydrogène, de fluor et de chlore et les groupements (alkyle en C₁-C₃)-, (alkoxy en C₁-C₃)-, halogéno-(alkyle en C₁-C₃)-, (fluoroalkoxy en C₁-C₃)- ;
ou ses sels, solvates ou sels de solvates.

3. Composé de formule générale (I) selon la revendication 1, 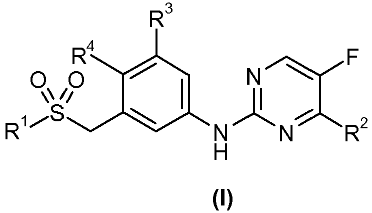 où
R¹ représente un groupement choisi parmi les groupements (alkyle en C₁-C₆,) -, (cycloalkyle en C₃-C₇)-, hétérocyclyl-, phényle, hétéroaryle, phényl-(alkyle en C₁-C₃)- ou hétéroaryl-(alkyle en C₁-C₃)-,
où ledit groupement est éventuellement substitué par un, deux ou trois substituants, identiques ou différents, choisis dans le groupe constitué par les groupements hydroxy, cyano, halogéno, halogéno-(alkyle en C₁-C₃)-, (alkoxy en C₁-C₆)-, (fluoroalkoxy en C₁-C₃)-, amino, alkylamino-, dialkylamino-, acétylamino-, N-méthyl-N-acétylamino-, amines cycliques, -OP(O)(OH)₂, -C(O)OH, - C(O)NH₂ ;
R² représente un groupement 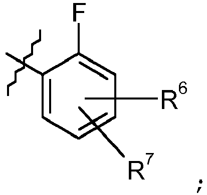
R³ représente un groupement trifluorométhyle ;
R⁴ représente un groupement choisi parmi les atomes d'hydrogène et d'halogène et les groupements cyano, SF₅, (alkyle en C₁-C₃)-, (alkoxy en C₁-C₃)-, hydroxy, halogéno-(alkyle en C₁-C₃)-, (fluoroalkoxy en C₁-C₃)-,
chacun des radicaux R⁶, R⁷ représente indépendamment de l'autre un groupement choisi parmi les atomes d'hydrogène, de fluor et de chlore et les groupements (alkyle en C₁-C₃)-, (alkoxy en C₁-C₃)-, halogéno-(alkyle en C₁-C₃)-, (fluoroalkoxy en C₁-C₃)- ;
ou ses sels, solvates ou sels de solvates.

4. Composé de formule générale (I) selon la revendication 1, où
R¹ représente un groupement choisi parmi les groupements (alkyle en C₁-C₆)- ou cycloalkyle en C₃-C₅ ;
où ledit groupement est éventuellement substitué par un substituant choisi dans le groupe constitué par les groupements amino, alkoxy en C₁-C₃, hydroxy, -OP(O)(OH)₂ ;
R² représente un groupement 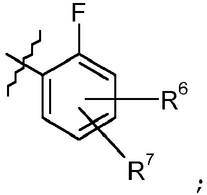 R³ représente un groupement choisi parmi les atomes d'halogène et les groupements -SF₅ ou halogéno-(alkyle en C₁-C₃) - ;
R⁴ représente un atome d'hydrogène ou de fluor ; chacun des radicaux R⁶, R⁷ représente indépendamment de l'autre un groupement choisi parmi un atome d'hydrogène ou de fluor,
ou ses sels, solvates ou sels de solvates.

5. Composé de formule générale (I) selon l'une quelconque des revendications 1, 2 ou 4, où
R³ représente un groupement -SF₅, et
R⁴ représente un atome d'hydrogène ;
ou ses sels, solvates ou sels de solvates.

6. Composé de formule générale (I) selon l'une quelconque des revendications 1 ou 5, où R² représente le groupement 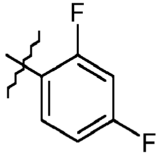 ou ses sels, solvates ou sels de solvates.

7. Composé de formule générale (I) selon la revendication 1, où
R¹ représente un groupement choisi parmi les groupements méthyle, éthyle, n-propyle, iso-propyle, tert-butyle, cyclopropyle ou
2-méthoxyéthyle ;
R² représente un groupement choisi parmi les groupements 2,4-difluorophényle, 2,3,4-trifluorophényle ou 2,4,5-trifluorophényle ;
R³ représente un atome de fluor ou un groupement -SF₅ ou trifluorométhyle ; et
R⁴ représente un atome d'hydrogène ;
ou ses sels, solvates ou sels de solvates.

8. Composé selon la revendication 1, choisi parmi les suivants :
4-(2,4-Difluorophényl)-5-fluoro-N-{3-[(méthylsulfonyl)méthyl]-5-(trifluorométhyl)phényl}pyrimidin-2-amine
4-(2,4-Difluorophényl)-5-fluoro-N-{3-[(propan-2-ylsulfonyl)méthyl]phényl}pyrimidin-2-amine
N-{3-[(tert-Butylsulfonyl)méthyl]phényl}-4-(2,4-difluorophényl)-5-fluoropyrimidin-2-amine
4-(2,4-Difluorophényl)-5-fluoro-N-(3-{[(2-méthoxyéthyl)sulfonyl]méthyl}phényl)pyrimidin-2-amine
4-(2,4-Difluorophényl)-5-fluoro-N-{3-[(méthylsulfonyl)méthyl]phényl}pyrimidin-2-amine
4-(2,4-Difluorophényl)-5-fluoro-N-{3-[(méthylsulfonyl) méthyl] -5- (pentafluoro-λ⁶ sulfanyl)phényl}pyrimidin-2-amine
4-(2,4-Difluorophényl)-N-{3-[(éthylsulfonyl)méthyl]phényl}-5-fluoropyrimidin-2-amine
4-(2,4-Difluorophényl)-5-fluoro-N-{3-[(propylsulfonyl)méthyl]phényl}pyrimidin-2-amine
N-{3-[(Cyclopropylsulfonyl)méthyl]phényl}-4-(2,4-difluorophényl)-5-fluoropyrimidin-2-amine
4-(2,4-Difluorophényl)-5-fluoro-N-{3-fluoro-5-[(méthylsulfonyl)méthyl]phényl}pyrimidin-2-amine
5-Fluoro-N-{3-fluoro-5-[(méthylsulfonyl)méthyl]phényl}-4-(2,4,5-trifluorophényl)pyrimidin-2-amine
5-Fluoro-N-{3-fluoro-5-[(méthylsulfonyl)méthyl]phényl}-4-(2,3,4-trifluorophényl)pyrimidin-2-amine
4-(2,4-Difluorophényl)-5-fluoro-N-{3-fluoro-5-[(propylsulfonyl)méthyl]phényl}pyrimidin-2-amine
5-Fluoro-N-{3-fluoro-5-[(propylsulfonyl)méthyl]phényl}-4-(2,4,5-trifluorophényl)pyrimidin-2-amine
ou ses sels, solvates ou sels de solvates.

9. Composé de formule générale **(I)** selon l'une quelconque des revendications 1 à 8, destiné au traitement prophylactique et/ou thérapeutique de troubles hyperprolifératifs, de maladies infectieuses induites par des virus et/ou de maladies cardiovasculaires.

10. Composé de formule générale **(I)** selon l'une quelconque des revendications 1 à 8, destiné au traitement prophylactique et/ou thérapeutique des carcinomes pulmonaires, des carcinomes prostatiques, des carcinomes du col de l'utérus, des carcinomes colorectaux, des mélanomes ou des carcinomes ovariens.

11. Combinaison pharmaceutique comprenant un composé selon l'une quelconque des revendications 1 à 8 en combinaison avec un ou plusieurs principes actifs supplémentaires.

12. Composition pharmaceutique comprenant un composé selon l'une quelconque des revendications 1 à 8 en combinaison avec un adjuvant inerte non toxique pharmaceutiquement adapté.

13. Combinaison pharmaceutique selon la revendication 11 destinée au traitement prophylactique et/ou thérapeutique de troubles hyperprolifératifs, de maladies infectieuses induites par des virus et/ou de maladies cardio-vasculaires.

14. Composition pharmaceutique selon la revendication 12 destinée au traitement prophylactique et/ou thérapeutique de troubles hyperprolifératifs, de maladies infectieuses induites par des virus et/ou de maladies cardio-vasculaires.

15. Composé de formule générale **(5)** 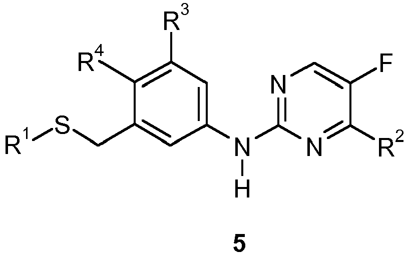 où les radicaux R¹, R², R³ et R⁴ sont tels que définis pour les composés de formule générale **(I)** selon l'une quelconque des revendications 1 à 7.

16. Procédé de synthèse des composés de formule **(I)** selon l'une quelconque des revendications 1 à 8, dans lequel un composé de formule **(5)** 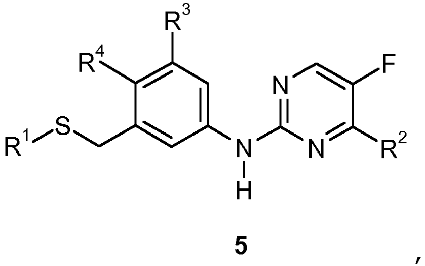
dans laquelle les radicaux R¹, R², R³ et R⁴ sont tels que définis selon l'une quelconque des revendications 1 à 7 pour les composés de formule générale **(I),** est oxydé avec un sel alcalin d'acide permanganique dans une cétone aliphatique de formule (alkyle en C₁-C₂)-C(=O)-(alkyle en C₁-C₂) en tant que solvant, pour obtenir un composé de formule générale **(I)** selon l'une quelconque des revendications 1 à 8,
et dans lequel le composé résultant de formule **(I)** réagit éventuellement, le cas échéant, avec les (i) solvants correspondants et/ou (ii) les bases ou acides correspondants en les solvates, sels et/ou solvates des sels des composés de formule **(I).**

17. Procédé de synthèse des composés de formule **(5)** dans lequel les radicaux R¹, R², R³ et R⁴ sont tels que définis selon l'une quelconque des revendications 1 à 7 pour les composés de formule générale **(I),**
dans lequel un composé de formule **(3)** 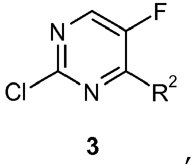 dans laquelle R² est tel que défini pour les composés de formule générale **(I)** dans l'une quelconque des revendications 1 à 7, réagit avec un composé de formule **(4)** 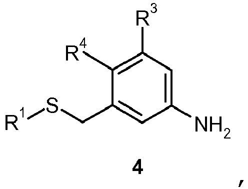 dans laquelle les radicaux R¹, R³ et R⁴ sont tels que définis pour les composés de formule générale **(I)** dans l'une quelconque des revendications 1 à 7, en présence d'un acide inorganique ou organique fort et dans un alcool aliphatique, un éther ou le *N,N-*diméthylformamide en tant que solvant, ou un mélange de tels solvants, pour obtenir un composé de formule générale **(5)** 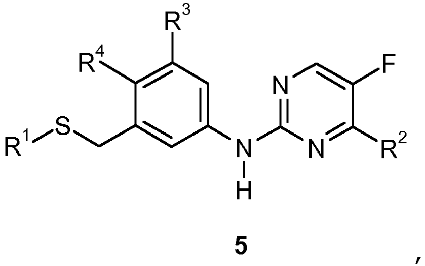 dans laquelle les radicaux R¹, R², R³ et R⁴ sont tels que définis selon l'une quelconque des revendications 1 à 7 pour les composés de formule générale **(I).**

18. Procédé de synthèse des composés de formule **(I)** selon l'une quelconque des revendications 1 à 8, dans lequel un composé de formule **(3)** 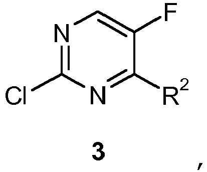 dans laquelle R² est tel que défini pour les composés de formule générale **(I)** dans l'une quelconque des revendications 1 à 7,
réagit avec un composé de formule **(6)** 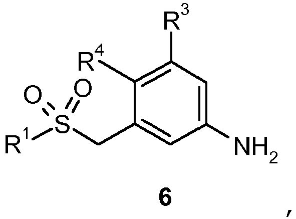
dans laquelle les radicaux R¹, R³ et R⁴ sont tels que définis pour les composés de formule générale **(I)** dans l'une quelconque des revendications 1 à 7, en présence d'un acide inorganique ou organique fort et dans un alcool aliphatique, un éther ou le *N,N-*diméthylformamide en tant que solvant, ou un mélange de tels solvants, pour obtenir un composé de formule générale **(I)** selon l'une quelconque des revendications 1 à 8,
et dans lequel le composé résultant de formule **(I)** réagit éventuellement, le cas échéant, avec les (i) solvants correspondants et/ou (ii) les bases ou acides correspondants en les solvates, sels et/ou solvates des sels des composés de formule **(I).**
